# EUROPEAN PATENT APPLICATION

(11) **EP 4 851 192 A1**
(43) Date of publication of application: **09.12.2026**
(21) Application number: 25747775.2
(22) Date of filing: 24.01.2025
(51) Int. Cl.: C07K 16/28, C12N 15/13, C12N 15/63, A61K 39/395, A61P 35/00

(54) **ANTI-LIV1 ANTIBODY AND USAGE METHOD**

(30) Priority: 31.01.2024 CN 202410137493
(71) Applicant: Shanghai Henlius Biotech, Inc., Shanghai 201210 (CN); Shanghai Henlius Biopharmaceutical Co., Ltd., Shanghai 200233 (CN); Shanghai Henlius Biologics Co., Ltd., Shanghai 201616 (CN)
(72) Inventor: SONG, Ge, Shanghai 201210 (CN); DING, Lan, Shanghai 201210 (CN); CHEN, Yu-Syaun, Shanghai 201210 (CN); SHAN, Yongqiang, Shanghai 201210 (CN)
(74) Representative: Rentsch Partner AG
(86) International application number: PCT/CN2025/074516
(87) International publication number: WO 2025/162207

(57) **Abstract**

The present disclosure relates to an antibody, antibody derivative or antibody conjugate which binds to LIV-1 and methods of using the same.

## Description

### CROSS-REFERENCE TO RELATED APPLICATION(S)

This application claims priority to Chinese patent application CN202410137493.7, filed on January 31, 2024, which is incorporated herein by reference in its entirety.

### FIELD

The present disclosure relates to antibodies and antibody derivatives that bind to LIV-1 and methods of using the same.

### BACKGROUND

Zinc ion plays an important role in DNA replication, transcription, protein synthesis, signal transduction, cell proliferation and apoptosis. Intracellular zinc ion level is strictly regulated by specific zinc transporters, and the disorder of intracellular zinc ion homeostasis is associated with tumorigenesis. Zinc ion level iss usually upregulated in tumor cells, which is beneficial to the survival and metastasis of tumor cells. It is shown that levels of zinc ions in breast and lung cancer tissues are significantly higher than in normal tissues.

There are two zinc transporter families in mammals, SLC39A (SLC39A6, solute carrier family 39 member) and SLC30A (SLC30A3, solute carrier family 30 member), and they can directly participate in the homeostasis of zinc ion in cells. It is shown that SLC39A family proteins can promote extracellular or intracellular transport of zinc ion to the cytoplasm, and several members of the SLC30A family assist in the outflow of zinc ion from the cytoplasm into the extracellular or intracellular organelles.

LIV-1 (SLC39A6), also known as ZIP6, belongs to the SLC39A family of zinc transporters capable of transporting zinc ions and contains eight transmembrane domains. Recently, it is shown that LIV-1 is highly expressed in a variety of tumor cells, such as breast cancer, prostate cancer, pancreatic cancer, cervical cancer and liver cancer, while has limited expression in normal tissues. Among them, breast cancer is the most common malignant tumor in women. Compared with the normal breast tissue, zinc ion is highly enriched in breast tumor cells. LIV-1 is the first zinc ion transporter found in breast cancer cells that can be induced by estrogen, so it is considered to be closely related to the onset and development of breast cancer. In addition to its function as a zinc transporter in cell growth, LIV-1 can also play a role in cell migration by binding to matrix metalloproteinases. In a variety of tumor cells, the concentration of zinc ion is significantly higher than that in normal tissues, and STAT3 was activated, contributing to the enhanced expression of LIV-1. It is also shown that ZIP6/ZIP10 heteropolymer transport zinc into cells, leading to STAT3 phosphorylation and mitosis acceleration, and promoting epithelial mesenchymal cell transformation of tumor cells and tumor metastasis. This suggests that LIV-1 may have an important role in embryonic development as well as in tumor development.

Currently, a LIV-1 targeting antibody-drug conjugate (ADC, SGN-LIV1A) comprising an anti-LIV-1 antibody hLIV22 developed by Seattle Genetics is in clinical phase II stage for treating the recurrent or metastatic breast cancer and other LIV-1-positive indications. In addition, SGN-LIV1A is also being used in combination with other antibody drugs like anti-PD-1 antibody in clinical trials.

There is a need for a novel anti-LIV-1 antibody for use in the anti-tumor immune-therapy with lower toxic side effect and better clinical benefit, providing the patients with more therapeutic options.

### SUMMARY OF THE INVENTION

The present disclosure provides isolated monoclonal antibodies and antibody derivatives that bind specifically to LIV-1 with high affinity, including monospecific anti-LIV-1 antibodies and multispecific antibodies that binds to LIV-1 and one or more additional target. In certain embodiments, an antibody, antibody derivative or antibody conjugate disclosed herein comprises a full-length antibody that binds to LIV-1. In certain embodiments, an antibody, antibody derivative or antibody conjugate disclosed herein comprises a scFv that binds to LIV-1. This disclosure further provides methods of making and using antibodies and antibody derivatives disclosed herein and pharmaceutical compositions comprising the same, e.g., for treating diseases and disorders, e.g., cancer. The invention is based, in part, on the discovery of novel antibodies that bind to LIV-1, which can target a tumor cell.

The present disclosure provides an antibody that binds to LIV-1 comprising: a) a heavy chain variable region comprising: (1) a heavy chain variable region CDR-H1 comprising an amino acid sequence of any one of SEQ ID NOs: 1, 11, 21, 31, and 41, or a variant thereof comprising up to about 3 amino acid substitutions; (2) a heavy chain variable region CDR-H2 comprising an amino acid sequence of any one of SEQ ID NOs: 2, 12, 22, 32, and 42, or a variant thereof comprising up to about 3 amino acid substitutions; and (3) a heavy chain variable region CDR-H3 comprising an amino acid sequence of any one of SEQ ID NOs: 3, 13, 23, 33, and 43, or a variant thereof comprising up to about 3 amino acid substitutions; and b) a light chain variable region comprising: (1) a light chain variable region CDR-L1 comprising an amino acid sequence of any one of SEQ ID NOs: 4, 14, 24, 34, and 44, or a variant thereof comprising up to about 3 amino acid substitutions; (2) a light chain variable region CDR-L2 comprising an amino acid sequence of any one of SEQ ID NOs: 5, 15, 25, 35, and 45, or a variant thereof comprising up to about 3 amino acid substitutions; and (3) a light chain variable region CDR-L3 comprising an amino acid sequence of any one of SEQ ID NOs: 6, 16, 26, 36, and 46, or a variant thereof comprising up to about 3 amino acid substitutions.

In certain embodiments, the antibody binds to LIV-1 with a KD of 1x10⁻⁸ M or less. In certain embodiments, the antibody binds to LIV-1 with a KD of 1x10⁻⁹ M or less. In certain embodiments, the antibody binds to LIV-1 with a KD of 1x10⁻¹⁰ M or less. In certain embodiments, the antibody binds to LIV-1 with a KD of 5x10⁻¹¹ M or less. In certain embodiments, the antibody binds to LIV-1 with a KD of between about 1x10⁻¹¹ M and about 1x10⁻⁹ M. In certain embodiments, the antibody binds to LIV-1 with a KD of between about 1x10⁻¹¹ M and about 1x10⁻¹⁰ M. In certain embodiments, the antibody binds to LIV-1 with a KD of between about 1x10⁻¹¹ M and about 5x10⁻¹¹ M.

In certain embodiments, the antibody cross-competes with a reference anti-LIV-1 antibody comprising: a) a heavy chain variable domain (VH) sequence comprising (1) a CDR-H1 comprising the amino acid sequence set forth in SEQ ID NO: 1, (2) a CDR- H2 comprising the amino acid sequence set forth in SEQ ID NO: 2, and (3) a CDR-H3 comprising the amino acid sequence set forth in SEQ ID NO: 3; and a light chain variable domain (VL) sequence comprising (1) a CDR-L1 comprising the amino acid sequence set forth in SEQ ID NO: 4, (2) a CDR-L2 comprising the amino acid sequence set forth in SEQ ID NO: 5, and (3) a CDR-L3 comprising the amino acid sequence set forth in SEQ ID NO: 6; b) a heavy chain variable domain (VH) sequence comprising (1) a CDR-H1 comprising the amino acid sequence set forth in SEQ ID NO: 11, (2) a CDR- H2 comprising the amino acid sequence set forth in SEQ ID NO: 12, and (3) a CDR-H3 comprising the amino acid sequence set forth in SEQ ID NO: 13; and a light chain variable domain (VL) sequence comprising (1) a CDR-L1 comprising the amino acid sequence set forth in SEQ ID NO: 14, (2) a CDR-L2 comprising the amino acid sequence set forth in SEQ ID NO: 15, and (3) a CDR-L3 comprising the amino acid sequence set forth in SEQ ID NO: 16; c) a heavy chain variable domain (VH) sequence comprising (1) a CDR-H1 comprising the amino acid sequence set forth in SEQ ID NO: 21, (2) a CDR- H2 comprising the amino acid sequence set forth in SEQ ID NO: 22, and (3) a CDR-H3 comprising the amino acid sequence set forth in SEQ ID NO: 23; and a light chain variable domain (VL) sequence comprising (1) a CDR-L1 comprising the amino acid sequence set forth in SEQ ID NO: 24, (2) a CDR-L2 comprising the amino acid sequence set forth in SEQ ID NO: 25, and (3) a CDR-L3 comprising the amino acid sequence set forth in SEQ ID NO: 26; d) a heavy chain variable domain (VH) sequence comprising (1) a CDR-H1 comprising the amino acid sequence set forth in SEQ ID NO: 31, (2) a CDR- H2 comprising the amino acid sequence set forth in SEQ ID NO: 32, and (3) a CDR-H3 comprising the amino acid sequence set forth in SEQ ID NO: 33; and a light chain variable domain (VL) sequence comprising (1) a CDR-L1 comprising the amino acid sequence set forth in SEQ ID NO: 34, (2) a CDR-L2 comprising the amino acid sequence set forth in SEQ ID NO: 35, and (3) a CDR-L3 comprising the amino acid sequence set forth in SEQ ID NO: 36; or e) a heavy chain variable domain (VH) sequence comprising (1) a CDR-H1 comprising the amino acid sequence set forth in SEQ ID NO: 41, (2) a CDR- H2 comprising the amino acid sequence set forth in SEQ ID NO: 42, and (3) a CDR-H3 comprising the amino acid sequence set forth in SEQ ID NO: 43; and a light chain variable domain (VL) sequence comprising (1) a CDR-L1 comprising the amino acid sequence set forth in SEQ ID NO: 44, (2) a CDR-L2 comprising the amino acid sequence set forth in SEQ ID NO: 45, and (3) a CDR-L3 comprising the amino acid sequence set forth in SEQ ID NO: 46.

In certain embodiments, the antibody comprises: a) a heavy chain variable region that comprises a CDR-H1 domain, a CDR-H2 domain and a CDR-H3 domain, wherein the CDR-H1 domain, the CDR-H2 domain and the CDR-H3 domain respectively comprise a CDR-H1 domain, a CDR-H2 domain and a CDR-H3 domain comprised in a reference heavy chain variable region comprising the amino acid sequence selected from the group consisting of SEQ ID NOs: 7, 17, 27, 37, and 47; and b) a light chain variable region that comprises a CDR-L1 domain, a CDR-L2 domain and a CDR-L3 domain, wherein the CDR-L1 domain, the CDR-L2 domain and the CDR-L3 domain respectively comprise a CDR-L1 domain, a CDR-L2 domain and a CDR-L3 domain comprised in a reference light chain variable region comprising the amino acid sequence selected from the group consisting of SEQ ID NOs: 8, 18, 28, 38, and 48.

In certain embodiments, the antibody comprises a heavy chain variable domain (VH) sequence comprising (1) a CDR-H1 comprising the amino acid sequence set forth in SEQ ID NO: 1, (2) a CDR- H2 comprising the amino acid sequence set forth in SEQ ID NO: 2, and (3) a CDR-H3 comprising the amino acid sequence set forth in SEQ ID NO: 3; and a light chain variable domain (VL) sequence comprising (1) a CDR-L1 comprising the amino acid sequence set forth in SEQ ID NO: 4, (2) a CDR-L2 comprising the amino acid sequence set forth in SEQ ID NO: 5, and (3) a CDR-L3 comprising the amino acid sequence set forth in SEQ ID NO: 6. In certain embodiments, the antibody comprises a heavy chain variable domain (VH) sequence comprising (1) a CDR-H1 comprising the amino acid sequence set forth in SEQ ID NO: 11, (2) a CDR- H2 comprising the amino acid sequence set forth in SEQ ID NO: 12, and (3) a CDR-H3 comprising the amino acid sequence set forth in SEQ ID NO: 13; and a light chain variable domain (VL) sequence comprising (1) a CDR-L1 comprising the amino acid sequence set forth in SEQ ID NO: 14, (2) a CDR-L2 comprising the amino acid sequence set forth in SEQ ID NO: 15, and (3) a CDR-L3 comprising the amino acid sequence set forth in SEQ ID NO: 16. In certain embodiments, the antibody comprises a heavy chain variable domain (VH) sequence comprising (1) a CDR-H1 comprising the amino acid sequence set forth in SEQ ID NO: 21, (2) a CDR- H2 comprising the amino acid sequence set forth in SEQ ID NO: 22, and (3) a CDR-H3 comprising the amino acid sequence set forth in SEQ ID NO: 23; and a light chain variable domain (VL) sequence comprising (1) a CDR-L1 comprising the amino acid sequence set forth in SEQ ID NO: 24, (2) a CDR-L2 comprising the amino acid sequence set forth in SEQ ID NO: 25, and (3) a CDR-L3 comprising the amino acid sequence set forth in SEQ ID NO: 26. In certain embodiments, the antibody comprises a heavy chain variable domain (VH) sequence comprising (1) a CDR-H1 comprising the amino acid sequence set forth in SEQ ID NO: 31, (2) a CDR- H2 comprising the amino acid sequence set forth in SEQ ID NO: 32, and (3) a CDR-H3 comprising the amino acid sequence set forth in SEQ ID NO: 33; and a light chain variable domain (VL) sequence comprising (1) a CDR-L1 comprising the amino acid sequence set forth in SEQ ID NO: 34, (2) a CDR-L2 comprising the amino acid sequence set forth in SEQ ID NO: 35, and (3) a CDR-L3 comprising the amino acid sequence set forth in SEQ ID NO: 36. In certain embodiments, the antibody comprises a heavy chain variable domain (VH) sequence comprising (1) a CDR-H1 comprising the amino acid sequence set forth in SEQ ID NO: 41, (2) a CDR- H2 comprising the amino acid sequence set forth in SEQ ID NO: 42, and (3) a CDR-H3 comprising the amino acid sequence set forth in SEQ ID NO: 43; and a light chain variable domain (VL) sequence comprising (1) a CDR-L1 comprising the amino acid sequence set forth in SEQ ID NO: 44, (2) a CDR-L2 comprising the amino acid sequence set forth in SEQ ID NO: 45, and (3) a CDR-L3 comprising the amino acid sequence set forth in SEQ ID NO: 46.

In certain embodiments, the antibody comprises a heavy chain variable region comprising the amino acid sequence set forth in SEQ ID NO: 7, and a light chain variable region comprising the amino acid sequence set forth in SEQ ID NO: 8. In certain embodiments, the antibody comprises a heavy chain variable region comprising the amino acid sequence set forth in SEQ ID NO: 17, and a light chain variable region comprising the amino acid sequence set forth in SEQ ID NO: 18. In certain embodiments, the antibody comprises a heavy chain variable region comprising the amino acid sequence set forth in SEQ ID NO: 27, and a light chain variable region comprising the amino acid sequence set forth in SEQ ID NO: 28. In certain embodiments, the antibody comprises a heavy chain variable region comprising the amino acid sequence set forth in SEQ ID NO: 37, and a light chain variable region comprising the amino acid sequence set forth in SEQ ID NO: 38. In certain embodiments, the antibody comprises a heavy chain variable region comprising the amino acid sequence set forth in SEQ ID NO: 47, and a light chain variable region comprising the amino acid sequence set forth in SEQ ID NO: 48.

In certain embodiments, the antibody comprises a human framework. In certain embodiments, the antibody is a humanized antibody. In certain embodiments, the antibody comprises a full-length immunoglobulin, a single-chain Fv (scFv) fragment, a Fab fragment, a Fab' fragment, a F(ab')2, an Fv fragment, a disulfide stabilized Fv fragment (dsFv), a (dsFv)2, a Fv-Fc fusion, a scFv-Fc fusion, a scFv-Fv fusion, a diabody, a tribody, a tetrabody or any combination thereof.

In certain embodiments, the antibody comprises a Fc region. In certain embodiments, the Fc region comprises a human Fc region. In certain embodiments, the Fc region comprises a Fc region selected from the group consisting of the Fc regions of IgG, IgA, IgD, IgE and IgM.

In certain embodiments, the Fc region comprises a Fc region selected from the group consisting of the Fc regions of IgG1, IgG2, IgG3 and IgG4. In certain embodiments, the Fc region comprises an IgG1 Fc region. In certain embodiments, the Fc region comprises an IgG4 Fc region. In certain embodiments, the IgG4 Fc region comprises a mutation of S228P. In certain embodiments, the Fc region comprises a C-terminal lysine. In certain embodiments, the Fc region comprises a deletion of a C-terminal lysine.

In certain embodiments, the antibody is comprised in a multispecific antibody, e.g., a bispecific antibody, wherein the multispecific antibody comprises a second antibody moiety that specifically binds to a second antigen. In certain embodiments, the second antigen is a tumor associated antigen. In certain embodiments, the tumor associated antigen is selected from the group consisting of Her-2, EGFR, PDL1, MSLN, c-Met, B Cell Maturation Antigen (BCMA), carbonic anhydrase IX (CA1X), carcinoembryonic antigen (CEA), CD5, CD7, CD10, CD19, CD20, CD22, CD30, CD33, CD34, CD38, CD41, CD44, CD49f, CD56, CD74, CD123, CD133, CD138, CD276 (B7H3), epithelial glycoprotein (EGP2), trophoblast cell-surface antigen 2 (TROP-2), epithelial glycoprotein- 40 (EGP-40), epithelial cell adhesion molecule (EpCAM), receptor tyrosine-protein kinases erb-B2,3,4, folate-binding protein (FBP), fetal acetylcholine receptor (AChR), folate receptor-a, Ganglioside G2 (GD2), Ganglioside G3 (GD3), human telomerase reverse transcriptase (hTERT), kinase insert domain receptor (KDR), Lewis A (CA 1.9.9), Lewis Y (LeY), Glypican-3 (GPC3), L1 cell adhesion molecule (L1CAM), Mucin 16 (Muc-16), Mucin 1 (Muc-1), NG2D ligands, oncofetal antigen (h5T4), prostate stem cell antigen (PSCA), prostate-specific membrane antigen (PSMA), tumor- associated glycoprotein 72 (TAG-72), Claudin18.2 (CLDN18.2), vascular endothelial growth factor R2 (VEGF- R2), Wilms tumor protein (WT-1), type 1 tyrosine-protein kinase transmembrane receptor (ROR1), PVR, PVRL2, and any combination thereof. In certain embodiments, the second antigen is an immune checkpoint regulator. In certain embodiments, the immune checkpoint regulator is selected from the group consisting of TIGIT, PD1, CTLA4, LAG-3, 2B4, BTLA and any combination thereof. In certain embodiments, the second antigen is an immune costimulatory molecule or a subunit of a T cell receptor/CD3 complex. In certain embodiments, the immune costimulatory molecule is selected from the group consisting of CD28, ICOS, CD27, 4-1BB, OX40 and CD40 and any combination thereof. In certain embodiments, the subunit of the T cell receptor/CD3 complex is selected from the group consisting of CD3γ, CD3δ, CD3ε and any combination thereof.

The present disclosure provides an immunoconjugate comprising any antibody disclosed herein linked to a therapeutic agent or a label. In certain embodiments, the therapeutic agent is a cytotoxin or a radioactive isotope. In certain embodiments, the label is selected from the group consisting of a radioisotope, a fluorescent dye and an enzyme.

The present disclosure provides a chimeric antigen receptor (CAR) comprising an extracellular antigen-binding domain that comprises an antibody disclosed herein. In certain embodiments, the antibody is a scFv.

The present disclosure provides an immunoresponsive cell comprising a CAR disclosed herein. In certain embodiments, the immunoresponsive cell is selected from the group consisting of a T cell, a Natural Killer (NK) cell, a cytotoxic T lymphocyte (CTL), a regulatory T cell, a Natural Killer T (NKT) cell and a myeloid cell. In certain embodiments, the immunoresponsive cell is a T cell.

The present disclosure further provides pharmaceutical compositions. In certain embodiments, the pharmaceutical composition comprises a) an antibody, an immunoconjugate or an immunoresponsive cell disclosed herein, and b) a pharmaceutically acceptable carrier.

The present disclosure further provides nucleic acids encoding any antibodies disclosed herein, vectors comprising any nucleic acid disclosed herein, and host cells comprising a nucleic acid or a vector disclosed herein.

The present disclosure provides methods for preparing an antibody disclosed herein. In certain embodiments, the method comprises expressing an antibody in a host cell disclosed herein and isolating the antibody from the host cell.

The present disclosure further provides methods of reducing tumor burden in a subject. In certain embodiments, the method comprises administering to the subject an effective amount of an antibody, an immunoconjugate, or a pharmaceutical composition disclosed herein.

In certain embodiments, the method reduces the number of tumor cells. In certain embodiments, the method reduces tumor size. In certain embodiments, the method eradicates the tumor in the subject. In certain embodiments, the tumor is selected from the group consisting of mesothelioma, lung cancer, pancreatic cancer, ovarian cancer, breast cancer (e.g., three-negative breast cancer (TNBC)), colon cancer, pleural tumor, glioblastoma, esophageal cancer, gastric cancer, synovial sarcoma, thymic carcinoma, endometrial carcinoma, stomach cancer, cholangiocarcinoma, head and neck cancer, blood cancer, prostate cancer, cervical cancer, melanoma, squamous cell carcinoma, liver cancer and a combination thereof.

The present disclosure further provides methods of treating and/or preventing a neoplasm in a subject. In certain embodiments, the method comprises administering to the subject an effective amount of an antibody, an immunoconjugate, or a pharmaceutical composition disclosed herein.

The present disclosure further provides methods of lengthening survival of a subject having a neoplasm. In certain embodiments, the method comprises administering to the subject an effective amount of an antibody, an immunoconjugate, or a pharmaceutical composition disclosed herein.

In certain embodiments, the neoplasm is selected from the group consisting of mesothelioma, lung cancer, pancreatic cancer, ovarian cancer, breast cancer (e.g., three-negative breast cancer (TNBC)), colon cancer, pleural tumor, glioblastoma, esophageal cancer, gastric cancer, synovial sarcoma, thymic carcinoma, endometrial carcinoma, stomach cancer, cholangiocarcinoma, head and neck cancer, blood cancer, prostate cancer, cervical cancer, melanoma, squamous cell carcinoma, liver cancer and a combination thereof.

The present disclosure provides any antibody, immunoconjugate or immunoresponsive cell disclosed herein for use as a medicament. The present disclosure further provides any antibody, immunoconjugate or immunoresponsive cell disclosed herein for use in treating cancer. The present disclosure further provides pharmaceutical compositions disclosed herein for use as a medicament. The present disclosure further provides pharmaceutical compositions disclosed herein for use in treating cancer. In certain embodiments, the cancer is selected from the group consisting of mesothelioma, lung cancer, pancreatic cancer, ovarian cancer, breast cancer (e.g., three-negative breast cancer (TNBC)), colon cancer, pleural tumor, glioblastoma, esophageal cancer, gastric cancer, synovial sarcoma, thymic carcinoma, endometrial carcinoma, stomach cancer, cholangiocarcinoma, head and neck cancer, blood cancer, prostate cancer, cervical cancer, melanoma, squamous cell carcinoma, liver cancer and a combination thereof.

The present disclosure provides kits comprising an antibody, an immunoconjugate, a pharmaceutical composition, a nucleic acid, a vector or an immunoresponsive cell disclosed herein. In certain embodiments, the kit comprise a written instruction for treating and/or preventing a neoplasm.

The present disclosure further provides a method of treating cancer in a subject comprising administering to the subject an effective amount of the antibody, immunoconjugate, or pharmaceutical composition disclosed herein and an anti-PD1 antibody. In certain embodiments, the cancer is selected from the group consisting of mesothelioma, lung cancer, pancreatic cancer, ovarian cancer, breast cancer (e.g., three-negative breast cancer (TNBC)), colon cancer, pleural tumor, glioblastoma, esophageal cancer, gastric cancer, synovial sarcoma, thymic carcinoma, endometrial carcinoma, stomach cancer, cholangiocarcinoma, head and neck cancer, blood cancer, prostate cancer, cervical cancer, melanoma, squamous cell carcinoma, liver cancer and a combination thereof. In certain embodiments, the antibody, immunoconjugate, or pharmaceutical composition disclosed herein and the anti-PD1 antibody are administered concurrently or sequentially.

### BRIEF DESCRIPTION OF THE FIGURES

Figure 1 depicts the binding ability of anti-LIV-1 antibodies to human LIV-1-ECD as determined by ELISA. Anti-LIV-1 ref. Ab (an analog of hLIV22 as disclosed in US9228026B2, hereinafter the same), was used as a positive control. IgG1 isotype control was used as a negative control.
Figure 2 depicts the binding ability of anti-LIV-1 antibodies to cyno-LIV-1-ECD as determined by ELISA. Anti-LIV-1 ref. Ab, a hLIV22 analog, was used as a positive control. IgG1 isotype control was used as a negative control.
Figure 3 depicts the binding ability of anti-LIV-1 antibodies to murine LIV-1-ECD as determined by ELISA. Anti-LIV-1 ref. Ab, a hLIV22 analog, was used as a positive control. IgG1 isotype control was used as a negative control.
Figure 4 depicts the binding ability of anti-LIV-1 antibodies to rat LIV-1-ECD as determined by ELISA. Anti-LIV-1 ref. Ab, a hLIV22 analog, was used as a positive control. IgG1 isotype control was used as a negative control.
Figure 5 depicts the binding ability of anti-LIV-1 antibodies to LIV-1 on the surface of CHO-S-hLIV-1 cells as determined by flow cytometry. Anti-LIV-1 ref. Ab, a hLIV22 analog, was used as a positive control. IgG1 isotype control was used as a negative control.
Figure 6 depicts the binding ability of anti-LIV-1 antibodies to LIV-1 on the surface of MCF-7 breast cancer cells as determined by flow cytometry. Anti-LIV-1 ref. Ab, a hLIV22 analog, was used as a positive control. IgG1 isotype control was used as a negative control.
Figure 7 depicts the binding ability of anti-LIV-1 antibodies to LIV-1 on the surface of CHO-S-cyno-LIV-1 cells as determined by flow cytometry. Anti-LIV-1 ref. Ab, a hLIV22 analog, was used as a positive control. IgG1 isotype control was used as a negative control.
Figures 8A to 8D depict the kinetic curves of anti-LIV-1 chimeric antibodies with respect to LIV-1 as measured by Octet. Anti-LIV-1 ref. Ab, a hLIV22 analog, was used as a positive control.
Figures 9A and 9B depict the epitope analysis of anti-LIV-1 chimeric antibodies as measured by Octet. Anti-LIV-1 ref. Ab, a hLIV22 analog, was used as a positive control. IgG1 isotype control was used as a negative control.
Figures 10A and 10B depict anti-LIV-1 chimeric antibodies' ADCC effects on NFAT-CD16a Jurkat in an effector-to-target ratio of 10:1 or 5:1 as described in Example 6.
Figure 11 depicts the internalization of anti-LIV-1 chimeric antibodies into CHOK1-hLIV1 cells as described in Example 7.
Figure 12 depicts the pharmacokinetics (PK) assay of the humanized anti-LIV-1 antibodies in rat as described in Example 9. Anti-LIV-1 ref. Ab, a hLIV22 analog, was used as a positive control.
Figures 13A to 13C depict in vitro cell killing of the humanized anti-LIV-1 antibody conjugates as described in Example 11. hLIV22-vc MMAE (a product resulting from the conjugation of a hLIV22 analog to MC-VC-PABC-MMAE, hereinafter the same) was used as a control.
Figure 14 depicts the anti-tumor activity of the humanized anti-LIV-1 antibody conjugates in MCF7-hLIV-1 inoculated mice. hLIV22-vc MMAE was used as a control. All data points in the tumor growth curves are the means ± SEM.

### DETAILED DESCRIPTION

The present disclosure provides isolated monoclonal antibodies and antibody derivatives that bind specifically to LIV-1 with high affinity, including monospecific anti-LIV-1 antibodies and multispecific antibodies that binds to LIV-1 and one or more additional target. In certain embodiments, an antibody, antibody derivative or antibody conjugate disclosed herein comprises a full-length antibody that binds to LIV-1. In certain embodiments, an antibody, antibody derivative or antibody conjugate disclosed herein comprises a scFv that binds to LIV-1. This disclosure further provides methods of making and using antibodies and antibody derivatives disclosed herein and pharmaceutical compositions comprising the same, e.g., for treating diseases and disorders, e.g., cancer. The invention is based, in part, on the discovery of novel antibodies that bind to LIV-1, which can target a tumor cell.

For clarity and not by way of limitation the detailed description of the presently disclosed subject matter is divided into the following subsections:
1. Definitions;
2. Antibodies and antibody derivatives;
3. Methods of use;
4. Pharmaceutical formulations; and
5. Articles of manufacture.

### 1. DEFINITIONS

The term "antibody" as referred to herein includes full-length antibodies and any antigen-binding fragment thereof (i.e., antibody fragment). An "antibody" can be a standalone molecule or a portion of an antibody derivative. Exemplary antibody derivatives include, but are not limited to, a multifunctional antibody, e.g., a multispecific antibody (e.g., a bispecific antibody), an antigen-recognizing receptor (e.g., a chimeric antigen receptor), an antibody conjugate comprising an additional proteinaceous or non-proteinaceous moiety (e.g., an antibody-drug conjugate or a polymer-coated antibody), and other multifuctional molecules comprising an antibody.

A "full-length antibody", "intact antibody" and "whole antibody" refers to an antibody similar to a native antibody structure or having heavy chains that contain an Fc region as defined herein. In certain embodiments, a full-length antibody comprises two heavy chains and two light chains. In certain embodiments, the variable regions of the light and heavy chains are responsible for antigen binding. The variable regions of a heavy chain and a light chain may be referred to as "VH" and "VL", respectively. The variable regions in both chains generally contain three highly variable loops called the complementarity determining regions (CDRs) (light chain (LC) CDRs including LC-CDR1, LC-CDR2, and LC-CDR3, heavy chain (HC) CDRs including HC-CDR1, HC-CDR2, and HC-CDR3). CDR boundaries for the antibodies and antigen-binding fragments disclosed herein may be defined or identified by well-known conventions, e.g., the conventions of Kabat, Chothia, MacCallum, IMGT and AHo as described below. The three CDRs of the heavy or light chains are interposed between flanking stretches known as framework regions (FRs), which are more conserved than the CDRs and form a scaffold to support the hypervariable loops. The constant regions of the heavy and light chains are not involved in antigen binding but exhibit various effector functions. Antibodies are assigned to classes based on the amino acid sequence of the constant region of their heavy chain. The five major classes or isotypes of antibodies are IgA, IgD, IgE, IgG, and IgM, which are characterized by the presence of α, δ, ε, γ, and µ heavy chains, respectively. Several of the major antibody classes are divided into subclasses such as IgG1 (γ1 heavy chain), IgG2 (γ2 heavy chain), IgG3 (γ3 heavy chain), IgG4 (γ4 heavy chain), IgA1 (α1 heavy chain), or IgA2 (α2 heavy chain). In certain embodiments, a full-length antibody is glycosylated. In certain embodiments, a full-length antibody comprises a glycan linked to its Fc region. In certain embodiments, a full-length antibody comprises a branched glycan.

The term "antigen-binding portion", "antibody fragment" and "antibody portion" of an antibody, as used herein, refers to one or more fragments of an antibody that retain the ability to specifically bind to an antigen. It has been shown that the antigen-binding function of an antibody can be performed by fragments of a full-length antibody. Examples of antibody fragments include but are not limited to Fv, Fab, Fab', Fab'-SH, F(ab')2, diabodies, linear antibodies, single-chain antibody molecules (e.g., scFv and scFv-Fc), a single domain antibody, a VHH, a VHH-Fc, a nanobody, a domain antibody, a bivalent domain antibody, or any other fragment or combination thereof of an antibody that binds to an antigen. A "VHH" refers to a single domain antibody isolated from a camelid animal. In certain embodiments, a VHH comprises a variable region of a heavy chain of a camelid heavy chain antibody.

An "antibody that cross-competes for binding" with a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is described in Antibodies, Harlow and Lane (Cold Spring Harbor Press, Cold Spring Harbor, NY).

"Fv" is a minimum antibody fragment which contains a complete antigen-recognition and -binding site. This fragment consists of a dimer of one heavy- and one light-chain variable region in tight, non-covalent association. From the folding of these two domains emanate six hypervariable loops (3 loops in each of the heavy and light chains) that contribute the amino acid residues to antigen binding and confer antigen binding specificity to the antibody. However, even a single variable domain (or half of an Fv comprising only three CDRs specific for an antigen) can recognize and bind to an antigen, although sometimes at a lower affinity than the entire binding site.

"Single-chain Fv," also abbreviated as "sFv" or "scFv," are antibody fragments that comprise the V_{H} and V_{L} antibody domains connected into a single polypeptide chain. In some embodiments, the scFv polypeptide further comprises a polypeptide linker between the V_{H} and V_{L} domains which enables the scFv to form the desired structure for antigen binding. For a review of scFv, see Plückthun in The Pharmacology of Monoclonal Antibodies, vol. 113, Rosenburg and Moore eds., Springer-Verlag, New York, pp. 269-315 (1994).

An "acceptor human framework" or "human framework" for the purposes herein is a framework comprising the amino acid sequence of a light chain variable domain (VL) framework or a heavy chain variable domain (VH) framework derived from a human immunoglobulin framework or a human consensus framework. An acceptor human framework "derived from" a human immunoglobulin framework or a human consensus framework may comprise the same amino acid sequence thereof, or it may contain amino acid sequence changes. In certain embodiments, the number of amino acid changes are 10 or less, 9 or less, 8 or less, 7 or less, 6 or less, 5 or less, 4 or less, 3 or less, or 2 or less. In certain embodiments, the VL acceptor human framework is identical in sequence to the VL human immunoglobulin framework sequence or human consensus framework sequence.

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (KD). Affinity can be measured by common methods known in the art, including those described herein. Specific illustrative and exemplary embodiments for measuring binding affinity are described in the following.

An "affinity matured" antibody refers to an antibody with one or more alterations in one or more CDRs or hypervariable regions (HVRs), compared to a parent antibody which does not possess such alterations, which alterations provide improved affinity of the antibody for antigen.

"LIV-1", "LIV-1 protein" or "LIV-1 polypeptide" as used herein, refers to any LIV-1 polypeptide from any vertebrate source, including mammals such as primates (e.g., humans and cynomolgus monkeys), or any fragment thereof, and may optionally comprise up to one, up to two, up to three, up to four, up to five, up to six, up to seven, up to eight, up to nine or up to ten amino acid substitutions, additions and/or deletions. The term encompasses full-length, unprocessed LIV-1 as well as any form of LIV-1 that results from processing in the cell. The term also encompasses naturally occurring variants of LIV-1, e.g., splice variants or allelic variants. In certain embodiments, a LIV-1 polypeptide comprises or has an amino acid sequence that is at least about 80%, at least about 85%, at least about 90%, at least about 95%, at least about 96%, at least about 97%, at least about 98%, at least about 99% or at least about 100% homologous or identical to the sequence of SEQ ID NO: 72, 74, 75 or 76 (homology herein may be determined using standard software such as BLAST or FASTA). In certain embodiments, the LIV-1 polypeptide comprises or has an amino acid sequence that is the entirety or a consecutive portion of SEQ ID NO: 72, 74, 75 or 76.

The term "ECD of LIV-1" refers to an extracellular domain of LIV-1. In certain embodiments, the extracellular domain of LIV-1 is a N-terminal extracellular domain of LIV-1. In certain embodiments, the N-terminal ECD of an exemplary LIV-1 polypeptide can comprise the amino acid sequence set forth in SEQ ID NO: 73.

The terms "anti-LIV-1 antibody" and "an antibody that binds to LIV-1" refer to an antibody that is capable of binding to LIV-1 with sufficient affinity such that the antibody is useful as a diagnostic and/or therapeutic agent for targeting LIV-1. In one embodiment, the extent of binding of an anti-LIV-1 antibody to an unrelated, non-LIV-1 protein is less than about 10% of the binding of the antibody to LIV-1 as measured, e.g., by a BIACORE^{®} surface plasmon resonance assay. In certain embodiments, an antibody that binds to LIV-1 has a dissociation constant (KD) of < about 1 µM, < about 100 nM, < about 10 nM, < about 1 nM, < about 0.1 nM, < about 0.01 nM, or < about 0.001 nM (e.g., 10⁻⁹ M or less, e.g., from 10⁻⁹ M to 10⁻¹¹ M, e.g., from 10⁻¹⁰ M to 10⁻¹¹ M). In certain embodiments, an anti-LIV-1 antibody binds to an epitope of LIV-1 that is conserved among LIV-1 from different species. In certain embodiments, an anti-LIV-1 antibody binds to an epitope on LIV-1 that is in the ECD of the protein.

The term "chimeric" antibody refers to an antibody in which a portion of the heavy and/or light chain is derived from a particular source or species, while the remainder of the heavy and/or light chain is derived from a different source or species.

As used herein, the term "CDR" or "complementarity determining region" is intended to mean the non-contiguous antigen combining sites within the variable region of a heavy chain and/or a light chain. These particular regions have been described by Kabat et al., J. Biol. Chem. 252:6609-6616 (1977); Kabat et al., U.S. Dept. of Health and Human Services, "Sequences of proteins of immunological interest" (1991); Chothia et al., J. Mol. Biol. 196:901-917 (1987); Al-Lazikani B. et al., J. Mol. Biol., 273: 927-948 (1997); MacCallum et al., J. Mol. Biol. 262:732-745 (1996); Abhinandan and Martin, Mol. Immunol., 45: 3832-3839 (2008); Lefranc M.P. et al., Dev. Comp. Immunol., 27: 55-77 (2003); and Honegger and Plückthun, J. Mol. Biol., 309:657-670 (2001), where the definitions include overlapping or subsets of amino acid residues when compared against each other. Nevertheless, application of any one of the definitions to refer to a CDR of an antibody or grafted antibodies or variants thereof is intended to be within the scope of the term as defined and used herein. The amino acid residues which encompass the CDRs as defined by each of the above cited references are set forth below in Table 1 as a comparison. CDR prediction algorithms and interfaces are known in the art, including, for example, Abhinandan and Martin, Mol. Immunol., 45: 3832-3839 (2008); Ehrenmann F. et al., Nucleic Acids Res., 38: D301-D307 (2010); and Adolf-Bryfogle J. et al., Nucleic Acids Res., 43: D432-D438 (2015). The contents of the references cited in this paragraph are incorporated herein by reference in their entireties for use in the present application and for possible inclusion in one or more claims herein.

**Table 1: CDR definitions**

| | **Kabat¹** | **Chothia²** | **MacCallum³** | **IMGT⁴** | **AHo⁵** |
|---|---|---|---|---|---|
| V_{H} CDR1 | 31-35 | 26-32 | 30-35 | 27-38 | 25-40 |
| V_{H} CDR2 | 50-65 | 53-55 | 47-58 | 56-65 | 58-77 |
| V_{H} CDR3 | 95-102 | 96-101 | 93-101 | 105-117 | 109-137 |
| V_{L} CDR1 | 24-34 | 26-32 | 30-36 | 27-38 | 25-40 |
| V_{L} CDR2 | 50-56 | 50-52 | 46-55 | 56-65 | 58-77 |
| V_{L} CDR3 | 89-97 | 91-96 | 89-96 | 105-117 | 109-137 |

| | | | | | |
|---|---|---|---|---|---|
| ¹Residue numbering follows the nomenclature of Kabat et al., supra. ²Residue numbering follows the nomenclature of Chothia et al., supra. ³Residue numbering follows the nomenclature of MacCallum et al., supra. ⁴Residue numbering follows the nomenclature of Lefranc et al., supra. ⁵Residue numbering follows the nomenclature of Honegger and Plückthun, supra. | | | | | |

The expression "variable-domain residue-numbering as in Kabat" or "amino-acid-position numbering as in Kabat," and variations thereof, refers to the numbering system used for heavy-chain variable domains or light-chain variable domains of the compilation of antibodies in Kabat et al., supra. Using this numbering system, the actual linear amino acid sequence may contain fewer or additional amino acids corresponding to a shortening of, or insertion into, a FR or CDR of the variable domain. For example, a heavy-chain variable domain may include a single amino acid insert (residue 52a according to Kabat) after residue 52 of H2 and inserted residues (e.g. residues 82a, 82b, and 82c, etc. according to Kabat) after heavy-chain FR residue 82. The Kabat numbering of residues may be determined for a given antibody by alignment at regions of homology of the sequence of the antibody with a "standard" Kabat numbered sequence.

In certain embodiments, the amino acid residues which encompass the CDRs of a single domain antibody is defined according to the IMGT nomenclature in Lefranc et al., supra. In certain embodiments, the amino acid residues which encompass the CDRs of a full-length antibody or a scFv is defined according to the Kabat nomenclature in Kabat et al., supra. In certain embodiments, the numbering of the residues in an immunoglobulin heavy chain, e.g., in an Fc region, is that of the EU index as in Kabat et al., supra. The "EU index as in Kabat" refers to the residue numbering of the human IgG1 EU antibody.

"Framework" or "FR" refers to residues that are those variable-domain residues other than the CDR residues as herein defined.

A "humanized" antibody refers to a chimeric antibody comprising amino acid residues from non-human CDRs/HVRs and amino acid residues from human FRs. In certain embodiments, a humanized antibody will comprise substantially all of at least one, and typically two, variable domains, in which all or substantially all of the HVRs/CDRs correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

A "human antibody" is an antibody that possesses an amino-acid sequence corresponding to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art, including phage-display libraries. Hoogenboom and Winter, J. Mol. Biol., 227:381 (1991); Marks et al., J. Mol. Biol., 222:581 (1991). Also available for the preparation of human monoclonal antibodies are methods described in Cole et al., Monoclonal Antibodies and Cancer Therapy, Alan R. Liss, p. 77 (1985); Boerner et al., J. Immunol., 147(1):86-95 (1991). See also van Dijk and van de Winkel, Curr. Opin. Pharmacol., 5: 368-74 (2001). Human antibodies can be prepared by administering the antigen to a transgenic animal that has been modified to produce such antibodies in response to antigenic challenge, but whose endogenous loci have been disabled, e.g., immunized xenomice (see, e.g., U.S. Pat. Nos. 6,075,181 and 6,150,584 regarding XENOMOUSE^{™} technology). See also, for example, Li et al., Proc. Natl. Acad. Sci. USA, 103:3557-3562 (2006) regarding human antibodies generated via a human B-cell hybridoma technology.

"Percent (%) amino acid sequence identity" or "homology" with respect to the polypeptide and antibody sequences identified herein is defined as the percentage of amino acid residues in a candidate sequence that are identical with the amino acid residues in the polypeptide being compared, after aligning the sequences considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, Megalign (DNASTAR), or MUSCLE software. Those skilled in the art can determine appropriate parameters for measuring alignment, including any algorithms needed to achieve maximal alignment over the full-length of the sequences being compared. For purposes herein, however, % amino acid sequence identity values are generated using the sequence comparison computer program MUSCLE (Edgar, R.C., Nucleic Acids Research 32(5):1792-1797, 2004; Edgar, R.C., BMC Bioinformatics 5(1):113, 2004).

"Homologous" refers to the sequence similarity or sequence identity between two polypeptides or between two nucleic acid molecules. When a position in both of the two compared sequences is occupied by the same base or amino acid monomer subunit, e.g., if a position in each of two DNA molecules is occupied by adenine, then the molecules are homologous at that position. The percent of homology between two sequences is a function of the number of matching or homologous positions shared by the two sequences divided by the number of positions compared times 100. For example, if 6 of 10 of the positions in two sequences are matched or homologous then the two sequences are 60% homologous. By way of example, the DNA sequences ATTGCC and TATGGC share 50% homology. Generally, a comparison is made when two sequences are aligned to give maximum homology.

The term "constant domain" refers to the portion of an immunoglobulin molecule having a more conserved amino acid sequence relative to the other portion of the immunoglobulin, the variable domain, which contains the antigen-binding site. The constant domain contains the C_{H}1, C_{H}2 and C_{H}3 domains (collectively, C_{H}) of the heavy chain and the C_{L} domain of the light chain.

The "light chains" of antibodies (e.g., immunoglobulins) from any mammalian species can be assigned to one of two clearly distinct types, called kappa ("κ") and lambda ("λ"), based on the amino acid sequences of their constant domains.

The "CH1 domain" (also referred to as "C1" of "H1" domain) usually extends from about amino acid 118 to about amino acid 215 (EU numbering system).

"Hinge region" is generally defined as a region in IgG corresponding to Glu216 to Pro230 of human IgG1 (Burton, Molec. Immunol.22:161-206 (1985)). Hinge regions of other IgG isotypes may be aligned with the IgG1 sequence by placing the first and last cysteine residues forming inter-heavy chain S-S bonds in the same positions.

The "CH2 domain" of a human IgG Fc region (also referred to as "C2" domain) usually extends from about amino acid 231 to about amino acid 340. The CH2 domain is unique in that it is not closely paired with another domain. Rather, two N-linked branched carbohydrate chains are interposed between the two CH2 domains of an intact native IgG molecule. It has been speculated that the carbohydrate may provide a substitute for the domain-domain pairing and help stabilize the CH2 domain. Burton, Molec Immunol. 22:161-206 (1985).

The "CH3 domain" (also referred to as "C2" domain) comprises the residues between a CH2 domain and the C-terminal of an Fc region (i.e. from about amino acid residue 341 to the C-terminal end of an antibody sequence, typically at amino acid residue 446 or 447 of an IgG).

The term "Fc region" or "fragment crystallizable region" herein is used to define a C-terminal region of an immunoglobulin heavy chain, including native-sequence Fc regions and variant Fc regions. Although the boundaries of the Fc region of an immunoglobulin heavy chain might vary, the human IgG heavy-chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue. Suitable native-sequence Fc regions for use in the antibodies described herein include human IgG1, IgG2 (IgG2A, IgG2B), IgG3 and IgG4.

"Fc receptor" or "FcR" describes a receptor that binds the Fc region of an antibody. The preferred FcR is a native human FcR. Moreover, a preferred FcR is one which binds an IgG antibody (a gamma receptor) and includes receptors of the FcγRI, FcγRII, and FcγRIII subclasses, including allelic variants and alternatively spliced forms of these receptors, FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibitory receptor FcγRIIB contains an immunoreceptor tyrosine-based inhibition motif (ITIM) in its cytoplasmic domain. (See M. Daëron, Annu. Rev. Immunol. 15:203-234 (1997). FcRs are reviewed in Ravetch and Kinet, Annu. Rev. Immunol. 9: 457-92 (1991); Capel et al., Immunomethods 4: 25-34 (1994); and de Haas et al., J. Lab. Clin. Med. 126: 330-41 (1995). Other FcRs, including those to be identified in the future, are encompassed by the term "FcR" herein.

The term "epitope" as used herein refers to the specific group of atoms or amino acids on an antigen to which an antibody, antibody derivative or antibody conjugate binds. Two antibodies or antigen-binding moieties may bind the same epitope within an antigen if they exhibit competitive binding for the antigen.

As use herein, the terms "specifically binds," "specifically recognizing," and "is specific for" refer to measurable and reproducible interactions, such as binding between a target and an antibody or antibody moiety, which is determinative of the presence of the target in the presence of a heterogeneous population of molecules, including biological molecules. For example, an antibody or antibody moiety that specifically recognizes a target (which can be an epitope) is an antibody or antibody moiety that binds this target with greater affinity, greater avidity, greater readiness, and/or greater duration than its bindings to other targets. In some embodiments, the extent of binding of an antibody to an unrelated target is less than about 10% of the binding of the antibody to the target as measured, e.g., by a radioimmunoassay (RIA). In some embodiments, an antibody that specifically binds a target has a dissociation constant (K_{D}) of ≤10⁻⁵ M, ≤10⁻⁶ M, ≤10⁻⁷ M, ≤10⁻⁸ M, ≤10⁻⁹ M, ≤10⁻¹⁰ M, ≤10⁻¹¹ M, or ≤10⁻¹² M. In some embodiments, an antibody specifically binds an epitope on a protein that is conserved among the protein from different species. In some embodiments, specific binding can include, but does not require exclusive binding. Binding specificity of the antibody or antigen-binding domain can be determined experimentally by methods known in the art. Such methods comprise, but are not limited to Western blots, ELISA-, RIA-, ECL-, IRMA-, EIA-, BIACORE^{TM} -tests and peptide scans.

An "isolated" antibody (or construct) is one that has been identified, separated and/or recovered from a component of its production environment (e.g., natural or recombinant). In certain embodiments, the isolated polypeptide is free or substantially free from association with all other components from its production environment.

An "isolated" nucleic acid molecule encoding a construct, antibody, or antigen-binding fragment thereof described herein is a nucleic acid molecule that is identified and separated from at least one contaminant nucleic acid molecule with which it is ordinarily associated in the environment in which it was produced. In certain embodiments, the isolated nucleic acid is free or substantially free from association with all components associated with the production environment. The isolated nucleic acid molecules encoding the polypeptides and antibodies described herein is in a form other than in the form or setting in which it is found in nature. Isolated nucleic acid molecules therefore are distinguished from nucleic acid encoding the polypeptides and antibodies described herein existing naturally in cells. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

Nucleic acid is "operably linked" or "operatively linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading frame. However, enhancers do not have to be contiguous. Linking is accomplished by ligation at convenient restriction sites. If such sites do not exist, the synthetic oligonucleotide adaptors or linkers are used in accordance with conventional practice.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The term "transfected" or "transformed" or "transduced" as used herein refers to a process by which exogenous nucleic acid is transferred or introduced into the host cell. A "transfected" or "transformed" or "transduced" cell is one which has been transfected, transformed or transduced with exogenous nucleic acid, which cell includes the primary subject cell and its progeny.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell and may contain mutations. Mutant progeny that has the same function or biological activity as screened or selected for in the originally transformed cell are included herein.

The terms "subject," "individual," and "patient" are used interchangeably herein to refer to a mammal, including, but not limited to, human, bovine, horse, feline, canine, rodent, or primate. In some embodiments, the subject is a human.

An "effective amount" of an agent refers to an amount effective, at dosages and for periods of time necessary, to achieve the desired therapeutic or prophylactic result. The specific dose may vary depending on one or more of the particular agent chosen, the dosing regimen to be followed, whether it is administered in combination with other compounds, timing of administration, the tissue to be imaged, and the physical delivery system in which it is carried.

A "therapeutically effective amount" of a substance/molecule of the application, agonist or antagonist may vary according to factors such as the disease state, age, sex, and weight of the individual, and the ability of the substance/molecule, agonist or antagonist to elicit a desired response in the individual. A therapeutically effective amount is also one in which any toxic or detrimental effects of the substance/molecule, agonist or antagonist are outweighed by the therapeutically beneficial effects. A therapeutically effective amount may be delivered in one or more administrations.

As used herein, "treatment" or "treating" is an approach for obtaining beneficial or desired results, including clinical results. For purposes of this application, beneficial or desired clinical results include, but are not limited to, one or more of the following: alleviating one or more symptoms resulting from the disease, diminishing the extent of the disease, stabilizing the disease (e.g., preventing or delaying the worsening of the disease), preventing or delaying the spread (e.g., metastasis) of the disease, preventing or delaying the recurrence of the disease, delaying or slowing the progression of the disease, ameliorating the disease state, providing a remission (partial or total) of the disease, decreasing the dose of one or more other medications required to treat the disease, delaying the progression of the disease, increasing or improving the quality of life, increasing weight gain, and/or prolonging survival. Also encompassed by "treatment" is a reduction of pathological consequence of cancer (such as, for example, tumor volume). The methods of the application contemplate any one or more of these aspects of treatment. "Treatment" does not necessarily mean that the condition being treated will be cured.

"Effector functions" refer to those biological activities attributable to the Fc region of an antibody, which vary with the antibody isotype. Examples of antibody effector functions include: C1q binding and complement dependent cytotoxicity (CDC), Fc receptor binding, antibody dependent cell-mediated cytotoxicity (ADCC), phagocytosis, down regulation of cell surface receptors (e.g., B cell receptor), and B cell activation.

An "immunoconjugate" refers to an antibody conjugated to one or more heterologous molecule(s), including but not limited to a cytotoxic agent.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier," as used herein, refers to an ingredient in a pharmaceutical formulation, other than an active ingredient, which is nontoxic to a subject. A pharmaceutically acceptable carrier includes, but is not limited to, a buffer, excipient, stabilizer, or preservative.

The term "variable region" or "variable domain" refers to the domain of an antibody heavy or light chain that is involved in binding the antibody to antigen. In certain embodiments, the variable domains of the heavy chain and light chain (VH and VL, respectively) of a native antibody generally have similar structures, with each domain comprising four conserved framework regions (FRs) and three CDRs. (See, e.g., Kindt et al. Kuby Immunology, 61ed., W.H. Freeman and Co., page 91 (2007).) A single VH or VL domain may be sufficient to confer antigen-binding specificity. Furthermore, antibodies that bind to a particular antigen may be isolated using a VH or VL domain from an antibody that binds to the antigen to screen a library of complementary VL or VH domains, respectively. See, e.g., Portolano et al., J. Immunol. 150:880-887 (1993); Clarkson et al., Nature 352:624-628 (1991).

The term "antigen-recognizing receptor" as used herein refers to a receptor that is capable of activating an immunoresponsive cell (e.g., a T-cell) in response to its binding to an antigen. Non-limiting examples of antigen-recognizing receptors include native and modified T cell receptors ("TCRs") and chimeric antigen receptors ("CARs").

The term "chimeric antigen receptor" or "CAR" as used herein refers to a molecule comprising an extracellular antigen-binding domain that is fused to an intracellular signaling domain that is capable of activating or stimulating an immunoresponsive cell, and a transmembrane domain. In certain embodiments, the extracellular antigen-binding domain of a CAR comprises an antibody or an antibody fragment, e.g., a VHH or a scFv. In certain embodiments, the antibody (e.g., VHH or scFv) is fused to the transmembrane domain, which is fused to the intracellular signaling domain. In certain embodiments, the CAR is selected to have high binding affinity or avidity for the antigen.

By "immunoresponsive cell" is meant a cell that functions in an immune response or a progenitor or progeny thereof.

It is understood that embodiments of the application described herein include "consisting" and/or "consisting essentially of" embodiments.

As used herein, the term "about" or "approximately" means within an acceptable error range for the particular value as determined by one of ordinary skill in the art, which will depend in part on how the value is measured or determined, i.e., the limitations of the measurement system. In certain embodiments, "about" can mean within 3 or more than 3 standard deviations, per the practice in the art. In certain embodiments, "about" can mean a range of up to 20%, e.g., up to 10%, up to 5%, or up to 1% of a given value. In certain embodiments, particularly with respect to biological systems or processes, the term can mean within an order of magnitude, e.g., within 5-fold or within 2-fold, of a value.

As used herein, the term "modulate" means positively or negatively alter. Exemplary modulations include a about 1%, about 2%, about 5%, about 10%, about 25%, about 50%, about 75%, or about 100% change.

As used herein, the term "increase" means alter positively by at least about 5%. An alteration may be by about 5%, about 10%, about 25%, about 30%, about 50%, about 75%, about 100% or more.

As used herein, the term "reduce" means alter negatively by at least about 5%. An alteration may be by about 5%, about 10%, about 25%, about 30%, about 50%, about 75%, or even by about 100%.

The term "about X-Y" used herein has the same meaning as "about X to about Y."

As used herein and in the appended claims, the singular forms "a," "or," and "the" include plural referents unless the context clearly dictates otherwise.

### 2. ANTIBODIES AND ANTIBODY DERIVATIVES

The present disclosure provides antibodies, antibody derivatives and immunoconjugates thereof. In certain embodiments, the disclosure is based, in part, on the discovery of monoclonal antibodies that bind to LIV-1 and immunoconjugates thereof, which can be used in antitumor therapeutics selectively targeting a tumor cell. In certain embodiments, the anti-LIV-1 antibodies exhibit low binding and/or toxicity to normal cells. In certain embodiments, the anti-LIV-1 antibodies exhibit superior binding ability and pharmacokinetic pattern compared with a reference antibody, e.g., a hLIV22 analog.

In certain embodiments, an antibody of the present disclosure can be or comprise a monoclonal antibody, including a chimeric, humanized or human antibody. In certain embodiments, the antibody disclosed herein comprises a humanized antibody. In certain embodiments, the antibody comprises an acceptor human framework, e.g., a human immunoglobulin framework or a human consensus framework. In certain embodiments, the antibody disclosed herein comprises a human antibody.

In certain embodiments, an antibody of the present disclosure can be an antibody fragment, e.g., a Fv, Fab, Fab', scFv, diabody, or F(ab')2 fragment. In certain embodiments, the antibody is a full-length antibody, e.g., an intact IgG1 antibody, or other antibody class or isotype as defined herein. In certain embodiments, an antibody, antibody derivative or antibody conjugate of the present disclosure can incorporate any of the features, singly or in combination, as described in this application, e.g., Sections 2.1-2.9 detailed herein.

Antibodies and antibody derivatives of the present disclosure are useful, e.g., for the diagnosis or treatment of a neoplasm or a cancer. In certain embodiments, the neoplasia and cancers whose growth may be inhibited using the antibodies of this disclosure include neoplasia and cancers typically responsive to immunotherapy. In certain embodiments, the neoplasia and cancers include breast cancer (e.g., breast cell carcinoma, three-negative breast cancer (TNBC)), ovarian cancer (e.g., ovarian cell carcinoma) and renal cell carcinoma (RCC). Examples of other cancers that may be treated using the methods of this disclosure include melanoma (e.g., metastatic malignant melanoma), prostate cancer, colon cancer, lung cancer, bone cancer, pancreatic cancer, skin cancer, brain tumors, chronic or acute leukemias including acute myeloid leukemia, chronic myeloid leukemia, acute lymphoblastic leukemia, chronic lymphocytic leukemia, lymphomas (e.g., Hodgkin's and non-Hodgkin's lymphoma, lymphocytic lymphoma, primary CNS lymphoma, T-cell lymphoma) nasopharangeal carcinomas, cancer of the head or neck, cutaneous or intraocular malignant melanoma, uterine cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, cancer of the esophagus, cancer of the small intestine, cancer of the endocrine system, cancer of the thyroid gland, cancer of the parathyroid gland, cancer of the breast gland, sarcoma of soft tissue, cancer of the urethra, cancer of the penis, solid tumors of childhood, cancer of the bladder, cancer of the kidney or ureter, carcinoma of the breast pelvis, neoplasm of the central nervous system (CNS), tumor angiogenesis, spinal axis tumor, brain stem glioma, pituitary adenoma, Kaposi's sarcoma, epidermoid cancer, squamous cell cancer, environmentally induced cancers including those induced by asbestos, e.g., mesothelioma and combinations of said cancers.

### 2.1 Exemplary Anti-LIV-1 Antibodies

The present disclosure provides isolated antibodies that bind to a LIV-1 protein. In certain embodiments, an anti-LIV-1 antibody of the present disclosure binds to an ECD of LIV-1. In certain embodiments, the anti-LIV-1 antibody binds to the N-terminal ECD of LIV-1 that comprises the amino acid sequence set forth in SEQ ID NO: 73. In certain embodiments, the anti-LIV-1 antibody binds to the same epitope with an anti-LIV-1 antibody described herein, e.g., Clones 34C7D11, 43E5C6 and 70B8F3D9 and their humanized variants, e.g., h34C and h70B.

In certain embodiments, the anti-LIV-1 antibody disclosed herein can function as an antagonist of LIV-1. In certain embodiments, the anti-LIV-1 antibody exhibits superior binding ability, antitumor efficacy and pharmacokinetics (PK) profile (e.g., T1/2 and/or systematic exposure) compared to a reference anti-LIV-1 antibody, e.g., a hLIV22 analog. HLIV22 is an anti-LIV-1 antibody as disclosed in WO2012078688A2 or US9228026B2.

In certain embodiments, the antibody binds to LIV-1 with a KD of about 1x10⁻⁸ M or less. In certain embodiments, the antibody binds to LIV-1 with a KD of about 1x10⁻⁹ M or less. In certain embodiments, the antibody binds to LIV-1 with a KD of about 1x10⁻¹⁰ M or less. In certain embodiments, the antibody binds to LIV-1 with a KD of 5x10⁻¹¹ M or less. In certain embodiments, the antibody binds to LIV-1 with a KD of between about 1x10⁻¹¹ M and about 1x10⁻⁹ M. In certain embodiments, the antibody binds to LIV-1 with a KD of between about 1x10⁻¹¹ M and about 1x10⁻¹⁰ M. In certain embodiments, the antibody binds to LIV-1 with a KD of between about 1x10⁻¹¹ M and about 5x10⁻¹¹ M.

In certain embodiments, the anti-LIV-1 antibody comprises: a) a heavy chain variable region comprising: (1) a heavy chain variable region CDR-H1 comprising an amino acid sequence of any one of SEQ ID NOs: 1, 11, 21, 31, and 41, or a variant thereof comprising up to about 3 amino acid substitutions; (2) a heavy chain variable region CDR-H2 comprising an amino acid sequence of any one of SEQ ID NOs: 2, 12, 22, 32, and 42, or a variant thereof comprising up to about 3 amino acid substitutions; and (3) a heavy chain variable region CDR-H3 comprising an amino acid sequence of any one of SEQ ID NOs: 3, 13, 23, 33, and 43, or a variant thereof comprising up to about 3 amino acid substitutions; and b) a light chain variable region comprising: (1) a light chain variable region CDR-L1 comprising an amino acid sequence of any one of SEQ ID NOs: 4, 14, 24, 34, and 44, or a variant thereof comprising up to about 3 amino acid substitutions; (2) a light chain variable region CDR-L2 comprising an amino acid sequence of any one of SEQ ID NOs: 5, 15, 25, 35, and 45, or a variant thereof comprising up to about 3 amino acid substitutions; and (3) a light chain variable region CDR-L3 comprising an amino acid sequence of any one of SEQ ID NOs: 6, 16, 26, 36, and 46, or a variant thereof comprising up to about 3 amino acid substitutions.

In certain embodiments, the anti-LIV-1 antibody cross-competes with a reference anti-LIV-1 antibody that comprises: a) a heavy chain variable domain (VH) sequence comprising (1) a CDR-H1 comprising the amino acid sequence set forth in SEQ ID NO: 1, (2) a CDR- H2 comprising the amino acid sequence set forth in SEQ ID NO: 2, and (3) a CDR-H3 comprising the amino acid sequence set forth in SEQ ID NO: 3; and a light chain variable domain (VL) sequence comprising (1) a CDR-L1 comprising the amino acid sequence set forth in SEQ ID NO: 4, (2) a CDR-L2 comprising the amino acid sequence set forth in SEQ ID NO: 5, and (3) a CDR-L3 comprising the amino acid sequence set forth in SEQ ID NO: 6; b) a heavy chain variable domain (VH) sequence comprising (1) a CDR-H1 comprising the amino acid sequence set forth in SEQ ID NO: 11, (2) a CDR- H2 comprising the amino acid sequence set forth in SEQ ID NO: 12, and (3) a CDR-H3 comprising the amino acid sequence set forth in SEQ ID NO: 13; and a light chain variable domain (VL) sequence comprising (1) a CDR-L1 comprising the amino acid sequence set forth in SEQ ID NO: 14, (2) a CDR-L2 comprising the amino acid sequence set forth in SEQ ID NO: 15, and (3) a CDR-L3 comprising the amino acid sequence set forth in SEQ ID NO: 16; c) a heavy chain variable domain (VH) sequence comprising (1) a CDR-H1 comprising the amino acid sequence set forth in SEQ ID NO: 21, (2) a CDR- H2 comprising the amino acid sequence set forth in SEQ ID NO: 22, and (3) a CDR-H3 comprising the amino acid sequence set forth in SEQ ID NO: 23; and a light chain variable domain (VL) sequence comprising (1) a CDR-L1 comprising the amino acid sequence set forth in SEQ ID NO: 24, (2) a CDR-L2 comprising the amino acid sequence set forth in SEQ ID NO: 25, and (3) a CDR-L3 comprising the amino acid sequence set forth in SEQ ID NO: 26; d) a heavy chain variable domain (VH) sequence comprising (1) a CDR-H1 comprising the amino acid sequence set forth in SEQ ID NO: 31, (2) a CDR- H2 comprising the amino acid sequence set forth in SEQ ID NO: 32, and (3) a CDR-H3 comprising the amino acid sequence set forth in SEQ ID NO: 33; and a light chain variable domain (VL) sequence comprising (1) a CDR-L1 comprising the amino acid sequence set forth in SEQ ID NO: 34, (2) a CDR-L2 comprising the amino acid sequence set forth in SEQ ID NO: 35, and (3) a CDR-L3 comprising the amino acid sequence set forth in SEQ ID NO: 36; or e) a heavy chain variable domain (VH) sequence comprising (1) a CDR-H1 comprising the amino acid sequence set forth in SEQ ID NO: 41, (2) a CDR- H2 comprising the amino acid sequence set forth in SEQ ID NO: 42, and (3) a CDR-H3 comprising the amino acid sequence set forth in SEQ ID NO: 43; and a light chain variable domain (VL) sequence comprising (1) a CDR-L1 comprising the amino acid sequence set forth in SEQ ID NO: 44, (2) a CDR-L2 comprising the amino acid sequence set forth in SEQ ID NO: 45, and (3) a CDR-L3 comprising the amino acid sequence set forth in SEQ ID NO: 46.

In certain embodiments, the anti-LIV-1 antibody comprises a heavy chain variable region that comprises a CDR-H1 domain, a CDR-H2 domain and a CDR-H3 domain, and a light chain variable region that comprises a CDR-L1 domain, a CDR-L2 domain and a CDR-L3 domain, wherein the CDR-H1 domain, the CDR-H2 domain and the CDR-H3 domain respectively comprise a CDR-H1 domain, a CDR-H2 domain and a CDR-H3 domain comprised in a reference heavy chain variable region comprising the amino acid sequence selected from the group consisting of SEQ ID NOs: 7, 17, 27, 37, and 47, and the CDR-L1 domain, the CDR-L2 domain and the CDR-L3 domain respectively comprise a CDR-L1 domain, a CDR-L2 domain and a CDR-L3 domain comprised in a reference light chain variable region comprising the amino acid sequence selected from the group consisting of SEQ ID NOs: 8, 18, 28, 38, and 48.

In certain embodiments, the anti-LIV-1 antibody comprises a heavy chain variable region that comprises a CDR-H1 domain, a CDR-H2 domain and a CDR-H3 domain, and a light chain variable region that comprises a CDR-L1 domain, a CDR-L2 domain and a CDR-L3 domain, wherein the CDR-H1 domain, the CDR-H2 domain and the CDR-H3 domain respectively comprise a CDR-H1 domain, a CDR-H2 domain and a CDR-H3 domain comprised in a reference heavy chain variable region comprising the amino acid sequence set forth in SEQ ID NO: 7, and the CDR-L1 domain, the CDR-L2 domain and the CDR-L3 domain respectively comprise a CDR-L1 domain, a CDR-L2 domain and a CDR-L3 domain comprised in a reference light chain variable region comprising the amino acid sequence forth in SEQ ID NO: 8.

In certain embodiments, the anti-LIV-1 antibody comprises a heavy chain variable region that comprises a CDR-H1 domain, a CDR-H2 domain and a CDR-H3 domain, and a light chain variable region that comprises a CDR-L1 domain, a CDR-L2 domain and a CDR-L3 domain, wherein the CDR-H1 domain, the CDR-H2 domain and the CDR-H3 domain respectively comprise a CDR-H1 domain, a CDR-H2 domain and a CDR-H3 domain comprised in a reference heavy chain variable region comprising the amino acid sequence set forth in SEQ ID NO: 17, and the CDR-L1 domain, the CDR-L2 domain and the CDR-L3 domain respectively comprise a CDR-L1 domain, a CDR-L2 domain and a CDR-L3 domain comprised in a reference light chain variable region comprising the amino acid sequence forth in SEQ ID NO: 18.

In certain embodiments, the anti-LIV-1 antibody comprises a heavy chain variable region that comprises a CDR-H1 domain, a CDR-H2 domain and a CDR-H3 domain, and a light chain variable region that comprises a CDR-L1 domain, a CDR-L2 domain and a CDR-L3 domain, wherein the CDR-H1 domain, the CDR-H2 domain and the CDR-H3 domain respectively comprise a CDR-H1 domain, a CDR-H2 domain and a CDR-H3 domain comprised in a reference heavy chain variable region comprising the amino acid sequence set forth in SEQ ID NO: 27, and the CDR-L1 domain, the CDR-L2 domain and the CDR-L3 domain respectively comprise a CDR-L1 domain, a CDR-L2 domain and a CDR-L3 domain comprised in a reference light chain variable region comprising the amino acid sequence forth in SEQ ID NO: 28.

In certain embodiments, the anti-LIV-1 antibody comprises a heavy chain variable region that comprises a CDR-H1 domain, a CDR-H2 domain and a CDR-H3 domain, and a light chain variable region that comprises a CDR-L1 domain, a CDR-L2 domain and a CDR-L3 domain, wherein the CDR-H1 domain, the CDR-H2 domain and the CDR-H3 domain respectively comprise a CDR-H1 domain, a CDR-H2 domain and a CDR-H3 domain comprised in a reference heavy chain variable region comprising the amino acid sequence set forth in SEQ ID NO: 37, and the CDR-L1 domain, the CDR-L2 domain and the CDR-L3 domain respectively comprise a CDR-L1 domain, a CDR-L2 domain and a CDR-L3 domain comprised in a reference light chain variable region comprising the amino acid sequence forth in SEQ ID NO: 38.

In certain embodiments, the anti-LIV-1 antibody comprises a heavy chain variable region that comprises a CDR-H1 domain, a CDR-H2 domain and a CDR-H3 domain, and a light chain variable region that comprises a CDR-L1 domain, a CDR-L2 domain and a CDR-L3 domain, wherein the CDR-H1 domain, the CDR-H2 domain and the CDR-H3 domain respectively comprise a CDR-H1 domain, a CDR-H2 domain and a CDR-H3 domain comprised in a reference heavy chain variable region comprising the amino acid sequence set forth in SEQ ID NO: 47, and the CDR-L1 domain, the CDR-L2 domain and the CDR-L3 domain respectively comprise a CDR-L1 domain, a CDR-L2 domain and a CDR-L3 domain comprised in a reference light chain variable region comprising the amino acid sequence forth in SEQ ID NO: 48.

In certain embodiments, the anti-LIV-1 antibody comprises a heavy chain variable domain (VH) sequence comprising (1) a CDR-H1 comprising the amino acid sequence set forth in SEQ ID NO: 1, (2) a CDR- H2 comprising the amino acid sequence set forth in SEQ ID NO: 2, and (3) a CDR-H3 comprising the amino acid sequence set forth in SEQ ID NO: 3; and a light chain variable domain (VL) sequence comprising (1) a CDR-L1 comprising the amino acid sequence set forth in SEQ ID NO: 4, (2) a CDR-L2 comprising the amino acid sequence set forth in SEQ ID NO: 5, and (3) a CDR-L3 comprising the amino acid sequence set forth in SEQ ID NO: 6. In certain embodiments, the anti-LIV-1 antibody comprises a heavy chain variable domain (VH) sequence comprising (1) a CDR-H1 comprising the amino acid sequence set forth in SEQ ID NO: 11, (2) a CDR- H2 comprising the amino acid sequence set forth in SEQ ID NO: 12, and (3) a CDR-H3 comprising the amino acid sequence set forth in SEQ ID NO: 13; and a light chain variable domain (VL) sequence comprising (1) a CDR-L1 comprising the amino acid sequence set forth in SEQ ID NO: 14, (2) a CDR-L2 comprising the amino acid sequence set forth in SEQ ID NO: 15, and (3) a CDR-L3 comprising the amino acid sequence set forth in SEQ ID NO: 16. In certain embodiments, the anti-LIV-1 antibody comprises a heavy chain variable domain (VH) sequence comprising (1) a CDR-H1 comprising the amino acid sequence set forth in SEQ ID NO: 21, (2) a CDR- H2 comprising the amino acid sequence set forth in SEQ ID NO: 22, and (3) a CDR-H3 comprising the amino acid sequence set forth in SEQ ID NO: 23; and a light chain variable domain (VL) sequence comprising (1) a CDR-L1 comprising the amino acid sequence set forth in SEQ ID NO: 24, (2) a CDR-L2 comprising the amino acid sequence set forth in SEQ ID NO: 25, and (3) a CDR-L3 comprising the amino acid sequence set forth in SEQ ID NO: 26. In certain embodiments, the anti-LIV-1 antibody comprises a heavy chain variable domain (VH) sequence comprising (1) a CDR-H1 comprising the amino acid sequence set forth in SEQ ID NO: 31, (2) a CDR- H2 comprising the amino acid sequence set forth in SEQ ID NO: 32, and (3) a CDR-H3 comprising the amino acid sequence set forth in SEQ ID NO: 33; and a light chain variable domain (VL) sequence comprising (1) a CDR-L1 comprising the amino acid sequence set forth in SEQ ID NO: 34, (2) a CDR-L2 comprising the amino acid sequence set forth in SEQ ID NO: 35, and (3) a CDR-L3 comprising the amino acid sequence set forth in SEQ ID NO: 36. In certain embodiments, the anti-LIV-1 antibody comprises a heavy chain variable domain (VH) sequence comprising (1) a CDR-H1 comprising the amino acid sequence set forth in SEQ ID NO: 41, (2) a CDR-H2 comprising the amino acid sequence set forth in SEQ ID NO: 42, and (3) a CDR-H3 comprising the amino acid sequence set forth in SEQ ID NO: 43; and a light chain variable domain (VL) sequence comprising (1) a CDR-L1 comprising the amino acid sequence set forth in SEQ ID NO: 44, (2) a CDR-L2 comprising the amino acid sequence set forth in SEQ ID NO: 45, and (3) a CDR-L3 comprising the amino acid sequence set forth in SEQ ID NO: 46.

In certain embodiments, the anti-LIV-1 antibody comprises a heavy chain variable region comprising an amino acid sequence having at least about 80%, 85%, 90%, 91%, 92%, 93%, 94%, 95%, 96%, 97%, 98%, 99%, or 100% sequence identity to the amino acid sequence selected from the group consisting of SEQ ID NOs: 7, 17, 27, 37, and 47, and a light chain variable region comprising an amino acid sequence having at least about 80%, 85%, 90%, 91%, 92%, 93%, 94%, 95%, 96%, 97%, 98%, 99%, or 100% sequence identity to the amino acid sequence selected from the group consisting of SEQ ID NOs: 8, 18, 28, 38, and 48. In certain embodiments, the anti-LIV-1 antibody comprises a heavy chain variable region comprising an amino acid sequence selected from the group consisting of SEQ ID NOs: 7, 17, 27, 37, and 47, and a light chain variable region comprising an amino acid sequence selected from the group consisting of SEQ ID NOs: 8, 18, 28, 38, and 48.

In certain embodiments, the anti-LIV-1 antibody comprises a heavy chain variable region comprising the amino acid sequence set forth in SEQ ID NO: 7, and a light chain variable region comprising the amino acid sequence set forth in SEQ ID NO: 8. In certain embodiments, the anti-LIV-1 antibody comprises a heavy chain variable region comprising the amino acid sequence set forth in SEQ ID NO: 17, and a light chain variable region comprising the amino acid sequence set forth in SEQ ID NO: 18. In certain embodiments, the anti-LIV-1 antibody comprises a heavy chain variable region comprising the amino acid sequence set forth in SEQ ID NO: 27, and a light chain variable region comprising the amino acid sequence set forth in SEQ ID NO: 28. In certain embodiments, the anti-LIV-1 antibody comprises a heavy chain variable region comprising the amino acid sequence set forth in SEQ ID NO: 37, and a light chain variable region comprising the amino acid sequence set forth in SEQ ID NO: 38. In certain embodiments, the anti-LIV-1 antibody comprises a heavy chain variable region comprising the amino acid sequence set forth in SEQ ID NO: 47, and a light chain variable region comprising the amino acid sequence set forth in SEQ ID NO: 48.

In certain embodiments, any one of the amino acid sequences comprised in the heavy chain variable region can comprise up to about 1, about 2, about 3, about 4, about 5, about 6, about 7, about 8, about 9 or about 10 amino acid substitutions, deletions and/or additions. In certain embodiments, the amino acid substitution is a conservative substitution.

In certain embodiments, the antibody comprises a human framework. In certain embodiments, the antibody is a humanized antibody.

In certain embodiments, the anti-LIV-1 antibody does not comprise a Fc region. In certain embodiments, the anti-LIV-1 antibody further comprises a Fc region. In certain embodiments, the Fc region comprises a human Fc region. In certain embodiments, the Fc region comprises a Fc region selected from the group consisting of the Fc regions of IgG, IgA, IgD, IgE and IgM. In certain embodiments, the Fc region comprises a Fc region selected from the group consisting of the Fc regions of IgG1, IgG2, IgG3 and IgG4. In certain embodiments, the Fc region comprises an IgG1 Fc region. In certain embodiments, the IgG1 Fc region comprising one or more mutation that modifies an antibody-dependent cell-mediated cytotoxicity (ADCC). In certain embodiments, the IgG1 Fc region comprising one or more mutation that reduces an antibody-dependent cell-mediated cytotoxicity (ADCC). In certain embodiments, the IgG1 Fc region comprising one or more mutation that enhances an antibody-dependent cell-mediated cytotoxicity (ADCC). In certain embodiments, the Fc region comprises an IgG4 Fc region. In certain embodiments, the IgG4 Fc region comprises a mutation of S228P. In certain embodiments, the Fc region comprises a C-terminal lysine. In certain embodiments, the Fc region comprises a deletion of a C-terminal lysine.

In certain embodiments, the anti-LIV-1 antibody comprises a full-length immunoglobulin, a single-chain Fv (scFv) fragment, a Fab fragment, a Fab' fragment, a F(ab')2, an Fv fragment, a disulfide stabilized Fv fragment (dsFv), a (dsFv)2, a VHH, a Fv-Fc fusion, a scFv-Fc fusion, a VHH-Fv fusion, a diabody, a tribody, a tetrabody or any combination thereof.

In certain embodiments, the antibody is comprised in a larger molecule that is an antibody derivative. In certain embodiments, the antibody derivative is a multispecific antibody, e.g., a bispecific antibody, wherein the multispecific antibody comprises a second antibody moiety that specifically binds to a second antigen. In certain embodiments, the second antigen is a tumor associated antigen. In certain embodiments, the tumor associated antigen is selected from the group consisting of Her-2, B7H3, EGFR, PD-L1, MSLN, c-Met, B Cell Maturation Antigen (BCMA), carbonic anhydrase IX (CA1X), carcinoembryonic antigen (CEA), CD5, CD7, CD10, CD19, CD20, CD22, CD30, CD33, CD34, CD38, CD41, CD44, CD49f, CD56, CD74, CD123, CD133, CD138, CD276 (B7H3), epithelial glycoprotein (EGP2), trophoblast cell-surface antigen 2 (TROP-2), epithelial glycoprotein- 40 (EGP-40), epithelial cell adhesion molecule (EpCAM), receptor tyrosine-protein kinases erb-B2,3,4, folate-binding protein (FBP), fetal acetylcholine receptor (AChR), folate receptor-a, Ganglioside G2 (GD2), Ganglioside G3 (GD3), human telomerase reverse transcriptase (hTERT), kinase insert domain receptor (KDR), Lewis A (CA 1.9.9), Lewis Y (LeY), Glypican-3 (GPC3), L1 cell adhesion molecule (L1CAM), Mucin 16 (Muc-16), Mucin 1 (Muc-1), NG2D ligands, oncofetal antigen (h5T4), prostate stem cell antigen (PSCA), prostate-specific membrane antigen (PSMA), tumor- associated glycoprotein 72 (TAG-72), Claudin18.2 (CLDN18.2), vascular endothelial growth factor R2 (VEGF- R2), Wilms tumor protein (WT-1), type 1 tyrosine-protein kinase transmembrane receptor (ROR1), PVR, PVRL2 and any combination thereof. In certain embodiments, the second antigen is an immune checkpoint regulator. In certain embodiments, the immune checkpoint regulator is selected from the group consisting of TIGIT, PD1, CTLA4, LAG-3, 2B4, BTLA and any combination thereof. In certain embodiments, binding of the antibody derivative or multispecific antibody to the second antigen inhibits the immune checkpoint regulator. In certain embodiments, the second antigen is an immune costimulatory molecule or a subunit of a T cell receptor/CD3 complex. In certain embodiments, the immune costimulatory molecule is selected from the group consisting of CD28, ICOS, CD27, 4-1BB, OX40 and CD40 and any combination thereof. In certain embodiments, binding of the antibody derivative or multispecific antibody to the second antigen activates the immune costimulatory molecule. In certain embodiments, the subunit of the T cell receptor/CD3 complex is selected from the group consisting of CD3γ, CD3δ, CD3ε and any combination thereof. In certain embodiments, binding of the antibody derivative or multispecific antibody to the second antigen activates the T cell receptor/CD3 complex.

In certain embodiments, the anti-LIV-1 antibody is linked to the second antigen binding moiety via a linker. In certain embodiments, the linker is a peptide linker. In certain embodiments, the peptide linker comprises about four to about thirty amino acids. In certain embodiments, the peptide linker comprises about four to about fifteen amino acids.

In certain embodiments, the anti-LIV-1 antibody is conjugated to a therapeutic agent or a label. In certain embodiments, the label is selected from the group consisting of a radioisotope, a fluorescent dye and an enzyme. In certain embodiments, the therapeutic agent is a cytotoxin or a radioactive isotope.

### 2.2 Antibody Affinity

In certain embodiments, an antibody or antibody derivative disclosed herein has a high binding affinity to its target antigen. In certain embodiments, the antibody or antibody derivative binds to the target with a KD of about 1x10⁻⁸ M or less. In certain embodiments, the antibody or antibody derivative binds to the target with a KD of about 1x10⁻⁹ M or less. In certain embodiments, the antibody or antibody derivative binds to the target with a KD of about 1x10⁻¹⁰ M or less. In certain embodiments, the antibody or antibody derivative binds to the target with a KD of about 5x10⁻¹¹ M or less.

In certain embodiments, the antibody or antibody derivative binds to the target with a KD of between about 1x10⁻¹¹ M and about 1x10⁻⁹ M. In certain embodiments, the antibody or antibody derivative binds to the target with a KD of between about 1x10⁻¹¹ M and about 1x10⁻¹⁰ M. In certain embodiments, the antibody or antibody derivative binds to the target with a KD of between about 1x10⁻¹¹ M and about 5x10⁻¹¹ M.

The KD of the antibody or antibody derivative can be determined by methods known in the art. Such methods comprise, but are not limited to Western blots, ELISA-, RIA-, ECL-, IRMA-, EIA-, Octet- BIACORE^{®}-tests and peptide scans.

In certain embodiments, KD can be measured using a BIACORE^{®} surface plasmon resonance assay. For example, and not by way of limitation, an assay using a BIACORE^{®}-2000 or a BIACORE^{®} 3000 (Biacore, Inc., Piscataway, NJ) is performed at 25°C with immobilized antigen CMS chips at about 10 response units (RU). In certain embodiments, carboxymethylated dextran biosensor chips (CMS, Biacore, Inc.) are activated with N-ethyl-N'-(3- dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 µg/ml (about 0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20TM) surfactant (PBST) at 25°C at a flow rate of approximately 25 µl/min. Association rates (kₒₙ) and dissociation rates (k_{off}) are calculated using a simple one-to-one Langmuir binding model (BIACORE^{®} Evaluation Software version 3.2) by simultaneously fitting the association and dissociation sensorgrams. The equilibrium dissociation constant (KD) can be calculated as the ratio koff/kon. See, e.g., Chen et al., J. Mol. Biol. 293:865-881

(1999). If the on-rate exceeds 10⁶ M^{-l} s⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO^{™} spectrophotometer (ThermoSpectronic) with a stirred cuvette.

### 2.3 Antibody Fragments

In certain embodiments, an antibody of the present disclosure comprises an antigen-binding fragment or antibody fragment. Antibody fragments include, but are not limited to, Fab, Fab', Fab'-SH, F(ab')2, VHH, Fv, and scFv fragments, and other fragments described herein. For a review of certain antibody fragments, see Hudson et al. Nat. Med. 9: 129-134 (2003). For a review of scFv fragments, see e.g., Pluckthtin, in The Pharmacology of Monoclonal Antibodies, vol. 113, Rosenburg and Moore eds., (Springer Verlag, New York), pp. 269-31 5 (1994); see also WO 93/16185; and U.S. Patent Nos. 5,571,894 and 5,587,458. For discussion of Fab and F(ab)₂ fragments comprising salvage receptor binding epitope residues and having increased in vivo half-life, see U.S. Patent No. 5,869,046.

In certain embodiments, an antibody of the present disclosure can be a diabody. Diabodies are antibody fragments with two antigen-binding sites that may be bivalent or bispecific. See, for example, EP 404,097; WO 1993/01 161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9: 129-134 (2003).

In certain embodiments, an antibody of the present disclosure can comprise a single domain antibody. Single domain antibodies are antibody fragments that comprise all or a portion of the heavy chain variable domain or all or a portion of the light chain variable domain of an antibody. In certain embodiments, the single domain antibody is a human single-domain antibody (Domantis, Inc., Waltham, MA; see, e.g., U.S. Patent No. 6,248,516 B1). In certain embodiments, the single domain antibody is camelid single-domain antibody. In certain embodiments, the single domain antibody is a VHH. In certain embodiments, the single domain antibody is a chimeric antibody. In certain embodiments, the single domain antibody is a humanized antibody.

Antibody fragments can be made by various techniques including, but not limited to, proteolytic digestion of an intact antibody as well as production by recombinant host cells (e.g., E. coli or phage), as described herein.

### 2.4 Chimeric and Humanized Antibodies

In certain embodiments, an antibody of the present disclosure is a chimeric antibody. Certain chimeric antibodies are described, e.g., in U.S. Patent No. 4,816,567; and Morrison et al., Proc. Natl. Acad. Sci. USA, 81:6851-6855 (1984)). In certain embodiments, a chimeric antibody comprises a non-human variable region (e.g., a variable region derived from mouse) and a human constant region. In certain embodiments, a chimeric antibody is a "class switched" antibody in which the class or subclass has been changed from that of the parent antibody. Chimeric antibodies include antigen-binding fragments thereof.

In certain embodiments, an antibody of the present disclosure can be a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and one or more framework (FR) (or any portion thereof) are derived from human antibody sequences. A humanized antibody optionally can also comprise at least a portion of a human constant region. In certain embodiments, certain FR residues in a humanized antibody are substituted with corresponding residues from a non-human antibody (e.g., the antibody from which the HVR residues are derived), e.g., to restore or improve antibody specificity or affinity.

Humanized antibodies and methods of making them are described, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing SDR (a-CDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); Framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

### 2.5 Antibody Derivatives

In certain embodiments, an antibody described herein may be further modified to be an antibody derivative comprising additional proteinaceous or nonproteinaceous moieties that are known in the art and readily available. Nonproteinaceous moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymers are attached, they can be the same or different molecules. In general, the number and/or type of polymers used for derivatization can be determined based on considerations including, but not limited to, the particular properties or functions of the antibody to be improved, whether the antibody derivative will be used in diagnosis under defined conditions, etc.

In certain embodiments, an antibody may be further modified to be an antibody derivative comprising one or more biologically active protein, polypeptides or fragments thereof. "Bioactive" or "biologically active", as used herein interchangeably, means showing biological activity in the body to carry out a specific function. For example, it may mean the combination with a particular biomolecule such as protein, DNA, etc., and then promotion or inhibition of the activity of such biomolecule. In certain embodiments, the bioactive protein or fragments thereof include proteins and polypeptides that are administered to patients as the active drug substance for prevention of or treatment of a disease or condition, as well as proteins and polypeptides that are used for diagnostic purposes, such as enzymes used in diagnostic tests or in vitro assays, as well as proteins and polypeptides that are administered to a patient to prevent a disease such as a vaccine.

### 2.6 Methods of Production

The antibodies and antibody derivatives disclosed herein can be produced using any available or known technique in the art. For example, but not by way of limitation, antibodies and antibody derivatives can be produced using recombinant methods and compositions, e.g., as described in U.S. Patent No. 4,816,567. Detailed procedures to generate antibodies and antibody derivatives are described in the Examples below.

The presently disclosed subject matter further provides isolated nucleic acids encoding an antibody or antibody derivative disclosed herein. For example, the isolated nucleic acid can encode an amino acid sequence comprising the VL and/or an amino acid sequence comprising the VH of the antibody, e.g., the light and/or heavy chains of the antibody.

In certain embodiments, the nucleic acid can be present in one or more vectors, e.g., expression vectors. As used herein, the term "vector" refers to a nucleic acid molecule capable of transporting another nucleic acid to which it has been linked. One type of vector is a "plasmid," which refers to a circular double stranded DNA loop into which additional DNA segments can be ligated. Another type of vector is a viral vector, where additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors, expression vectors, are capable of directing the expression of genes to which they are operably linked. In general, expression vectors of utility in recombinant DNA techniques are often in the form of plasmids (vectors). However, the disclosed subject matter is intended to include such other forms of expression vectors, such as viral vectors (e.g., replication defective retroviruses, adenoviruses and adeno associated viruses) that serve equivalent functions.

Different parts of an antibody or antibody derivative disclosed herein can be constructed in a single, multicistronic expression cassette, in multiple expression cassettes of a single vector, or in multiple vectors. Examples of elements that create polycistronic expression cassette include, but are not limited to, various viral and non-viral Internal Ribosome Entry Sites (IRES, e.g., FGF-l IRES, FGF-2 IRES, VEGF IRES, IGF-II IRES, NF-kB IRES, RUNX1 IRES, p53 IRES, hepatitis A IRES, hepatitis C IRES, pestivirus IRES, aphthovirus IRES, picornavirus IRES, poliovirus IRES and encephalomyocarditis virus IRES) and cleavable linkers (e.g., 2A peptides , e.g., P2A, T2A, E2A and F2A peptides). Combinations of retroviral vector and an appropriate packaging line are also suitable, where the capsid proteins will be functional for infecting human cells. Various amphotropic virus- producing cell lines are known, including, but not limited to, PA12 (Miller, et al. (1985) Mol. Cell. Biol. 5:431-437); PA317 (Miller, et al. (1986) Mol. Cell. Biol. 6:2895-2902); and CRIP (Danos, et al. (1988) Proc. Natl. Acad. Sci. USA 85:6460-6464). Non- amphotropic particles are suitable too, e.g., particles pseudotyped with VSVG, RD114 or GALV envelope and any other known in the art.

In certain embodiments, the nucleic acid encoding an antibody or antibody derivative of the present disclosure and/or the one or more vectors including the nucleic acid can be introduced into a host cell. In certain embodiments, the introduction of a nucleic acid into a cell can be carried out by any method known in the art including, but not limited to, transfection, electroporation, microinjection, infection with a viral or bacteriophage vector containing the nucleic acid sequences, cell fusion, chromosome-mediated gene transfer, microcell-mediated gene transfer, spheroplast fusion, etc. In certain embodiments, a host cell can include, e.g., has been transformed with: a vector comprising a nucleic acid that encodes an amino acid sequence comprising a single domain antibody and/or the VH of a single domain antibody. In certain embodiments, a host cell can include, e.g., has been transformed with: (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In certain embodiments, the host cell is eukaryotic, e.g., a Chinese Hamster Ovary (CHO) cell or lymphoid cell (e.g., YO, NSO, Sp20 cell).

In certain embodiments, the methods of making an antibody or antibody derivative disclosed herein can include culturing a host cell, in which a nucleic acid encoding the antibody or antibody derivative has been introduced, under conditions suitable for expression of the antibody or antibody derivative, and optionally recovering the antibody or antibody derivative from the host cell and/or host cell culture medium. In certain embodiments, the antibody or antibody derivative is recovered from the host cell through chromatography techniques.

For recombinant production of an antibody or antibody derivative of the present disclosure, a nucleic acid encoding an antibody or antibody derivative, e.g., as described above, can be isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody or antibody derivative). Suitable host cells for cloning or expression of antibody-encoding vectors include prokaryotic or eukaryotic cells described herein. For example, an antibody or antibody derivative can be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in E. coli.) After expression, the antibody or antibody derivative may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody or antibody derivative with a partially or fully human glycosylation pattern. See Gemgross, Nat. Biotech. 22:1409-1414 (2004), and Li et al., Nat. Biotech. 24:21 0-215 (2006). Suitable host cells for the expression of glycosylated antibody can also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells. In certain embodiments, plant cell cultures can be utilized as host cells. See, e.g., US Patent Nos. 5,959,177, 6,040,498, 6,420,548, 7,125,978, and 6,417,429 (describing PLANTIBODIES^{™} technology for producing antibodies in transgenic plants).

In certain embodiments, vertebrate cells can also be used as hosts. For example, and not by way of limitation, mammalian cell lines that are adapted to grow in suspension can be useful. Non-limiting examples of useful mammalian host cell lines are monkey kidney CV1 line transformed by SY40 (COS-7); human embryonic kidney line (293 or 293 cells as described, e.g., in Graham et al., J Gen Viral. 36:59 (1977)); baby hamster kidney cells (BHK); mouse sertoli cells (TM4 cells as described, e.g., in Mather, Biol. Reprod. 23:243-251 (1980)); monkey kidney cells (CV 1); African green monkey kidney cells (VERO-76); human cervical carcinoma cells (HELA); canine kidney cells (MDCK; buffalo rat liver cells (BRL 3A); human lung cells (W138); human liver cells (Hep 02); mouse mammary tumor (MMT 060562); TRI cells, as described, e.g., in Mather et al., Annals N. Y. Acad. Sci. 383:44-68 (1982); MRC 5 cells; and FS4 cells. Other useful mammalian host cell lines include Chinese hamster ovary (CHO) cells, including DHFK CHO cells (Urlaub et al., Proc. Natl. Acad. Sci. USA 77:42 I6 (1980)); and myeloma cell lines such as YO, NSO and Sp2/0. For a review of certain mammalian host cell lines suitable for antibody or antibody derivative production, see, e.g., Yazaki and Wu, Methods in Molecular Biology, Vol. 248 (B.K.C. Lo, ed., Humana Press, Totowa, NJ ), pp. 255-268 (2003).

In certain embodiments, an animal system can be used to produce an antibody or antibody derivative of the present disclosure. One animal system for preparing hybridomas is the murine system.

Hybridoma production in the mouse is a very well-established procedure. Immunization protocols and techniques for isolation of immunized splenocytes for fusion are known in the art. Fusion partners (e.g., murine myeloma cells) and fusion procedures are also known (see, e.g., Harlow and Lane (1988), Antibodies, A Laboratory Manual, Cold Spring Harbor Laboratory Press, Cold Spring Harbor New York).

### 2.7 Assays

The antibodies and antibody derivatives of the present disclosure provided herein can be identified, screened for, or characterized for their physical/chemical properties and/or biological activities by various assays known in the art and provided herein.

In certain embodiments, an antibody or antibody derivative of the present disclosure can be tested for its antigen binding activity by known methods, such enzyme-linked immunosorbent assay (ELISA), a radioimmunoassay (RIA), or a Western Blot Assay. Each of these assays generally detects the presence of protein-antibody complexes of particular interest by employing a labeled reagent (e.g., an antibody) specific for the complex of interest. For example, the antibody or antibody derivative can be detected using, e.g., an enzyme-linked antibody or antibody fragment which recognizes and specifically binds to the antibody or antibody derivative. Alternatively, the antibody or antibody derivative can be detected using any of a variety of other immunoassays. For example, the antibody or antibody derivative can be radioactively labeled and used in a radioimmunoassay (RIA) (see, for example, Weintraub, B., Principles of Radioimmunoassays, Seventh Training Course on Radioligand Assay Techniques, The Endocrine Society, March 1986, which is incorporated by reference herein). The radioactive isotope can be detected by such means as the use of a Geiger counter or a scintillation counter or by autoradiography.

In certain embodiments, competition assays can be used to identify an antibody or antibody derivative that competes with an antibody of the present disclosure for binding to LIV-1. In certain embodiments, such a competing antibody binds to the same epitope (e.g., a linear or a conformational epitope) that is bound by an antibody disclosed herein. Detailed exemplary methods for mapping an epitope to which an antibody binds are provided in Morris (1996) "Epitope Mapping Protocols," in Methods in Molecular Biology vol. 66 (Humana Press, Totowa, NJ).

In a non-limiting example of a competition assay, immobilized LIV-1 can be incubated in a solution comprising a first labeled antibody or antibody derivative that binds to LIV-1 and a second unlabeled antibody that is being tested for its ability to compete with the first antibody for binding to LIV-1. The second antibody may be present in a hybridoma supernatant. As a control, immobilized LIV-1 is incubated in a solution comprising the first labeled antibody but not the second unlabeled antibody. After incubation under conditions permissive for binding of the first antibody to LIV-1, excess unbound antibody is removed, and the amount of label associated with immobilized LIV-1 is measured. If the amount of label associated with immobilized LIV-1 is substantially reduced in the test sample relative to the control sample, then that indicates that the second antibody is competing with the first antibody for binding to LIV-1. See Harlow and Lane (1988) Antibodies: A Laboratory Manual ch.14 (Cold Spring Harbor Laboratory, Cold Spring Harbor, NY).

The present disclosure provides assays for identifying anti-LIV-1 antibodies or antibody derivatives thereof having biological activity. Biological activity may include, e.g., activating an immune cell or an immune activation reporter, e.g., a NFAT reporter or a NF-κB reporter. Antibodies having such biological activity in vivo and/or in vitro are also provided.

### 2.8 Immunoconjugates

The presently disclosed subject matter further provides immunoconjugates comprising an antibody or antibody derivative, disclosed herein, conjugated to one or more detection probe and/or cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes. For example, an antibody or antigen-binding portion of the disclosed subject matter can be functionally linked (e.g., by chemical coupling, genetic fusion, noncovalent association or otherwise) to one or more other binding molecules, such as another antibody, antibody fragment, peptide or binding mimetic.

In certain embodiments, an immunoconjugate is an antibody drug conjugate (ADC) in which an antibody is conjugated to one or more drugs, including but not limited to a maytansinoid (see U.S. Patent Nos. 5,208,020, 5,416,064 and European Patent EP 0 425 235); an auristatin such as monomethylauristatin drug moieties DE and DF (MMAE and MMAF) (see U.S. Patent Nos. 5,635,483 and 5,780,588, and 7,498,298); a dolastatin; a calicheamicin or derivative thereof (see U.S. Patent Nos. 5,712,374, 5,714,586, 5,739,1 16, 5,767,285, 5,770,701 , 5,770,710, 5,773,001, and 5,877,296; Hinman et al., Cancer Res. 53:3336-3342 (1993); and Lode et al., Cancer Res. 58:2925-2928 (1998)); an anthracycline such as daunomycin or doxorubicin (see Kratz et al., Current Med Chem. 13:477-523 (2006); Jeffrey et al., Bioorganic & Med. Chem. Letters 16:358- 362 (2006); Torgov et al., Bioconj. Chem. 16:717-721 (2005); Nagy et al., Proc. Natl. Acad. Sci. USA 97:829-834 (2000); Dubowchik et al., Bioorg. & Med. Chem. Letters 12:1529-1532 (2002); King et al., J Med. Chem. 45:4336-4343 (2002); and U.S. Patent No. 6,630,579); methotrexate; vindesine; a taxane such as docetaxel, paclitaxel, larotaxel, tesetaxel, and ortataxel; a trichothecene; and CC1065.

In certain embodiments, an immunoconjugate comprises an antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes.

In certain embodiments, an immunoconjugate comprises an antibody as described herein conjugated to a cytotoxin, including but not limited to amanitin, auristatin, calicheamicin, camptothecin, cryptophycin, daunomycin, dolastatin, doxorubicin, duocarmycin, epothilone, esperamicin, geldanamycin, maytansinoid, methotrexate, monomethyl auristatin E ("MMAE"), monomethyl auristatin F ("MMAF"), pyrrolobenzodiazepine, SN-38, rhizoxin, SG2285, tubulysin, vindesine, toxoid, or any combination thereof.

In certain embodiments, an immunoconjugate comprises an antibody as described herein conjugated to a radioactive atom to form a radioconjugate. A variety of radioactive isotopes are available for the production of radioconjugates. Non-limiting examples include At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu. When the radioconjugate is used for detection, it can include a radioactive atom for scintigraphic studies, for example tc99m or 1123, or a spin label for nuclear magnetic resonance (NMR) imaging (also known as magnetic resonance imaging, MRI), such as iodine-123, iodine-131, indium-11, fluorine-19, carbon- 13, nitrogen-15, oxygen-17, gadolinium, manganese or iron.

Conjugates of an antibody and cytotoxic agent can be made using a variety of bi functional protein coupling agents such as N-succinimid yl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine ), bis-diazonium derivatives (such as bis-(p-diazoniumbenzoyl) ethylenediamine ), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238: 1098 (1987). Carbon- 4-labeled l isothiocyanatobenzyl-3-methyldiethylene triaminepentaacetic acid (MX-DTPA) is an exemplary chelating agent for conjugation of radionucleotide to the antibody. See WO94/11026. The linker can be a "cleavable linker" facilitating release of a cytotoxic drug in the cell. For example, an acid-labile linker, peptidase-sensitive linker, photolabile linker, dimethyl linker or disulfide containing linker (Chari et al., Cancer Res. 52:127-1 31 (1992); U.S. Patent No. 5,208,020) can be used.

The immunuoconjugates or ADCs herein expressly contemplate, but are not limited to, such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available ( e.g., from Pierce Biotechnology, Inc., Rockford, IL., U.S.A).

In certain embodiments, an antibody is linked to a cytotoxin via a linker. Linkers are any group or moiety that links, connects, or bonds the antibody or antigen-binding proteins described herein with a therapeutic moiety, e.g., cytotoxic agent. Suitable linkers may be found, for example, in Antibody-Drug Conjugates and Immunotoxins; Phillips, G. L., Ed.; Springer Verlag: New York, 2013; Antibody- Drug Conjugates; Ducry, L., Ed.; Humana Press, 2013; Antibody-Drug Conjugates; Wang, J., Shen, W.-C., and Zaro, J. L., Eds.; Springer International Publishing, 2015, the contents of each incorporated herein in their entirety by reference. Generally, suitable binding agent linkers for the antibody conjugates described herein are those that are sufficiently stable to exploit the circulating half-life of the antibody and, at the same time, capable of releasing its payload upon antigen binding and/or antigen-mediated internalization of the conjugate. Linkers can be cleavable or non-cleavable. Cleavable linkers include linkers that are cleaved by intracellular metabolism following internalization, e.g., cleavage via hydrolysis, reduction, or enzymatic reaction. Non-cleavable linkers include linkers that release an attached payload via lysosomal degradation of the antibody following internalization. Suitable linkers include, but are not limited to, acid-labile linkers, hydrolysis-labile linkers, enzymatically cleavable linkers, reduction labile linkers, self-immolative linkers, and non-cleavable linkers. Suitable linkers also include, but are not limited to, those that are or comprise peptides, glucuronides, succinimide-thioethers, polyethylene glycol (PEG) units, hydrazones, mal-caproyl units, dipeptide units, valine-citrulline units, and para-aminobenzyl (PAB) units. In some aspects, the linker comprises one or more PEG groups.

Any linker molecule or linker technology known in the art can be used to create or construct an ADC of the present disclosure. In certain embodiments, the linker is a cleavable linker, e.g., a cathepsin B cleavable linker. According to other embodiments, the linker is a non-cleavable linker. Exemplary linkers that can be used in the context of the present disclosure include, linkers that comprise or consist of e.g., MC (6-maleimidocaproyl), MP (maleimidopropanoyl), val-cit (valine-citrulline), val-ala (valine-alanine), dipeptide site in protease-cleavable linker, ala-phe (alanine-phenylalanine), dipeptide site in protease-cleavable linker, PAB (p-aminobenzyloxycarbonyl), SPP (N-Succinimidyl 4-(2-pyridylthio) pentanoate), SMCC (N-Succinimidyl 4-(N-maleimidomethyl)cyclohexane-1 carboxylate), SIAB (N-Succinimidyl (4-iodo-acetyl)aminobenzoate), and variants and combinations thereof. Additional examples of linkers that can be used in the context of the present disclosure are provided, e.g., in US 7,754,681 and in Ducry, Bioconjugate Chem., 2010, 21:5-13, and the references cited therein, the contents of which are incorporated by reference herein in their entireties.

In certain embodiments, the linkers are stable in physiological conditions. In certain embodiments, the linkers are cleavable, for instance, able to release at least the payload portion in the presence of an enzyme or at a particular pH range or value. In some embodiments, a linker comprises an enzyme-cleavable moiety. Illustrative enzyme-cleavable moieties include, but are not limited to, peptide bonds, ester linkages, hydrazones, and disulfide linkages. In some embodiments, the linker comprises a cathepsin-cleavable linker.

In some embodiments, the linker comprises a non-cleavable moiety.

Suitable linkers also include, but are not limited to, those that are chemically bonded to two cysteine residues of a single binding agent, e.g., antibody. Such linkers can serve to mimic the antibody's disulfide bonds that are disrupted as a result of the conjugation process.

In some embodiments, the linker comprises one or more amino acids. In some embodiments, the linker comprises two amino acids. In some embodiments, the linker comprises three amino acids. In some embodiments, the linker comprises four amino acids. Suitable amino acids include natural, non-natural, standard, non-standard, proteinogenic, non-proteinogenic, and L- or D-α-amino acids. In some embodiments, the linker comprises alanine, valine, glycine, leucine, isoleucine, methionine, tryptophan, phenylalanine, proline, serine, threonine, cysteine, tyrosine, asparagine, glutamine, aspartic acid, glutamic acid, lysine, arginine, histidine, or citrulline, a derivative thereof, or combination thereof. In certain embodiments, one or more side chains of the amino acids is linked to a side chain group, described below. In some embodiments, the linker comprises valine and citrulline. In some embodiments, the linker comprises lysine, valine, and citrulline. In some embodiments, the linker comprises lysine, valine, and alanine. In some embodiments, the linker comprises valine and alanine. In some embodiments, the linker comprises a dipeptide, tripeptide, or tetrapeptide. In some embodiments, the linker comprises a peptide, wherein the peptide is valine-citrulline (val- cit or VC), glutamic acid-valine-citrulline (EVC), glycine-glycine-phenylalanine (GGF), or glycine- glycine-phenylalanine-glycine (GGFG).

In some embodiments, the linker comprises a self-immolative group. The self-immolative group can be any such group known to those of skill. In particular embodiments, the self-immolative group is p-aminobenzyl (PAB), or a derivative thereof. Useful derivatives include p- aminobenzyloxycarbonyl (PABC).

In some embodiments, the linker comprises a maleimidocaproyl group, one or more natural or unnatural amino acids, and a self-immolative group. For example, the linker comprises MC-VC-PABC as described in Example 10.

### 2.9 Antigen-Recognizing Receptor

The presently disclosed subject matter further provides antigen-recognizing receptors comprising an antibody or antibody fragment disclosed herein. An antigen-recognizing receptor is a receptor that is capable of activating, stimulating or inhibiting an immunoresponsive cell (e.g., a T-cell) in response to its binding to an antigen. Non-limiting examples of antigen-recognizing receptors include native and recombinant T cell receptors ("TCRs"), a chimeric co-stimulating receptor (CCR), a chimeric antigen receptor ("CAR") or an inhibitory CAR (iCAR). Antigen-recognizing receptor designs and methods of use are well known in the art, and is described in the literature, e.g., International Publications WO 2018/027155, WO 2019/099483, WO 2019/157454, WO 2019/133969, WO 2019/099993, WO 2015/142314, WO 2018/027197 and WO 2014055668.

In certain embodiments, the presently disclosed subject matter provides chimeric antigen receptors (CARs) comprising an antibody or antibody fragment disclosed herein. CARs are engineered receptors, which can graft or confer a specificity of interest onto an immune effector cell. In certain embodiments, a CAR can be used to graft the specificity of a monoclonal antibody onto a T cell; with transfer of its coding sequence facilitated by a vector. In certain embodiments, the CAR is a "First generation" CAR, which is typically composed of an extracellular antigen-binding domain (e.g., a scFv or a VHH) fused to a transmembrane domain, which is fused to cytoplasmic/intracellular signaling domain. "First generation" CARs can provide de novo antigen recognition and cause activation of an immunoresponsive cell, e.g., CD4+ and CD8+ T cells, through their CD3z chain signaling domain in a single fusion molecule, independent of HLA-mediated antigen presentation. In certain embodiments, the CAR is a "Second generation" CAR, which further comprises an intracellular signaling domain from various co-stimulatory molecules (e.g., CD28, 4-1BB, ICOS, 0X40, CD27, CD40/My88 and NKGD2) to the cytoplasmic tail of the CAR to provide additional signals to the immunoresponsive cell, whereby the "Second generation" CAR comprise those that provide both co-stimulation (e.g., CD28 or 4- 1BB) and activation (CD3z). In certain embodiments, the CAR is a "Third generation" CAR, which comprises multiple co-stimulation domains (e.g., CD28 and 4-1BB) and activation (CD3z). In certain embodiments, the CAR is a second-generation CAR. In certain embodiments, the CAR comprises an extracellular antigen-binding domain that binds to an antigen, a transmembrane domain, and an intracellular signaling domain, wherein the intracellular signaling domain comprises a co-stimulatory signaling domain. In certain embodiments, the CAR further comprises a hinger/spacer region between the extracellular antigen-binding domain and the transmembrane domain. In certain embodiments, the extracellular antigen-binding domain comprises an antibody or antibody fragment disclosed herein. In certain embodiments, the antibody or antibody fragment comprises a VHH or a scFv.

The presently disclosed subject matter provides immunoresponsive cells comprising (a) an antigen-recognizing receptor (e.g., CAR or TCR) disclosed herein. In certain embodiments, the antigen-recognizing receptor is capable of activating the immunoresponsive cell. The immunoresponsive cells of the presently disclosed subject matter can be cells of the lymphoid lineage. The lymphoid lineage, comprising B, T and natural killer (NK) cells, provides for the production of antibodies, regulation of the cellular immune system, detection of foreign agents in the blood, detection of cells foreign to the host, and the like. Non-limiting examples of immunoresponsive cells of the lymphoid lineage include T cells, Natural Killer (NK) cells, embryonic stem cells, and pluripotent stem cells (e.g., those from which lymphoid cells may be differentiated). T cells can be lymphocytes that mature in the thymus and are chiefly responsible for cell-mediated immunity. T cells are involved in the adaptive immune system. The T cells of the presently disclosed subject matter can be any type of T cells, including, but not limited to, helper T cells, cytotoxic T cells, memory T cells (including central memory T cells, stem-cell-like memory T cells (or stem-like memory T cells), and two types of effector memory T cells: e.g., TEM cells and TEMRA cells, Regulatory T cells (also known as suppressor T cells), Natural killer T cells, Mucosal associated invariant T cells, and gd T cells. Cytotoxic T cells (CTL or killer T cells) are a subset of T lymphocytes capable of inducing the death of infected somatic or tumor cells. A patient's own T cells may be genetically modified to target specific antigens through the introduction of an antigen recognizing receptor, e.g., a CAR or a TCR. In certain embodiments, the immunoresponsive cell is a T cell. The T cell can be a CD4+ T cell or a CD8+ T cell. In certain embodiments, the T cell is a CD4+ T cell. In certain embodiments, the T cell is a CD8+ T cell. Natural killer (NK) cells can be lymphocytes that are part of cell-mediated immunity and act during the innate immune response. NK cells do not require prior activation in order to perform their cytotoxic effect on target cells. Types of human lymphocytes of the presently disclosed subject matter include, without limitation, peripheral donor lymphocytes, e.g., those disclosed in Sadelain, M., et al. 2003 Nat Rev Cancer 3:35-45 (disclosing peripheral donor lymphocytes genetically modified to express CARs), in Morgan, R.A., et al. 2006 Science 314: 126-129 (disclosing peripheral donor lymphocytes genetically modified to express a full-length tumor antigen-recognizing T cell receptor complex comprising the a and b heterodimer), in Panelli, M.C., et al. 2000 J Immunol 164:495-504; Panelli, M.C., et al. 2000 J Immunol 164:4382-4392 (disclosing lymphocyte cultures derived from tumor infiltrating lymphocytes (TILs) in tumor biopsies), and in Dupont, J., et al. 2005 Cancer Res 65:5417-5427; Papanicolaou, G.A., et al. 2003 Blood 102:2498-2505 (disclosing selectively in vitro-ex panded antigen-specific peripheral blood leukocytes employing artificial antigen-presenting cells (AAPCs) or pulsed dendritic cells). In certain embodiments, the immunoresponsive cells (e.g., T cells) can be autologous, non-autologous (e.g., allogeneic), or derived in vitro from engineered progenitor or stem cells.

### 3. METHODS OF USE

The presently disclosed subject matter further provides methods for using the disclosed antibodies and antibody derivatives. In certain embodiments, the methods are directed to therapeutic uses of a presently disclosed antibody or antibody derivative. In certain embodiments, the methods are directed to diagnostic use of a presently disclosed antibody or antibody derivative.

### 3.1 Treatment Methods

The present disclosure provides methods and use of an antibody or antibody derivative disclosed herein for treatment of diseases and disorders. In certain embodiments, the antibody, antibody derivative or pharmaceutical compositions comprising the same disclosed herein can be administered to subjects (e.g., mammals such as humans) to treat diseases and disorders. In certain embodiments, the diseases and disorders involve abnormal LIV-1 activity. In certain embodiments, the diseases and disorders that can be treated by an antibody or antibody derivative disclosed herein include, but are not limited to, neoplasia, e.g., cancer.

In certain embodiments, the present disclosure provides an antibody or antibody derivative described herein (or fragments thereof) for use in the manufacture of a medicament. In certain embodiments, the present disclosure provides antibody or antibody derivative described herein (or fragments thereof) for use in the manufacture of a medicament for treating of cancer. In certain embodiments, the present disclosure provides an antibody or antibody derivative described herein (or fragments thereof) for use in treating cancer in a subject. In certain embodiments, the present disclosure provides pharmaceutical compositions comprising an antibody or antibody derivative provided herein (or fragments thereof) for use in treating cancer in a subject. In certain embodiments, the cancer can be blood cancers (e.g. leukemias, lymphomas, and myelomas), ovarian cancer, breast cancer (e.g., three-negative breast cancer (TNBC)), bladder cancer, brain cancer, cervical cancer, colon cancer, intestinal cancer, liver cancer, lung cancer, pancreatic cancer, prostate cancer, skin cancer, squamous cell carcinoma, stomach cancer, glioblastoma, throat cancer, melanoma, neuroblastoma, adenocarcinoma, glioma, soft tissue sarcoma, and various carcinomas (including prostate and small cell lung cancer). Suitable carcinomas further include any known carcinoma in the field of oncology, including, but not limited to, astrocytoma, fibrosarcoma, myxosarcoma, liposarcoma, oligodendroglioma, ependymoma, medulloblastoma, primitive neural ectodermal tumor (PNET), chondrosarcoma, osteogenic sarcoma, pancreatic ductal adenocarcinoma, small and large cell lung adenocarcinomas, chordoma, angiosarcoma, endotheliosarcoma, squamous cell carcinoma, bronchoalveolarcarcinoma, epithelial adenocarcinoma, and liver metastases thereof, lymphangiosarcoma, lymphangioendotheliosarcoma, hepatoma, cholangiocarcinoma, synovioma, mesothelioma, Ewing's tumor, rhabdomyosarcoma, colon carcinoma, basal cell carcinoma, sweat gland carcinoma, papillary carcinoma, sebaceous gland carcinoma, papillary adenocarcinoma, cystadenocarcinoma, medullary carcinoma, bronchogenic carcinoma, renal cell carcinoma, bile duct carcinoma, choriocarcinoma, seminoma, embryonal carcinoma, Wilms' tumor, testicular tumor, medulloblastoma, craniopharyngioma, ependymoma, pinealoma, hemangioblastoma, acoustic neuroma, oligodendroglioma, meningioma, neuroblastoma, retinoblastoma, leukemia, multiple myeloma, Waldenstrom's macroglobulinemia, breast tumors such as ductal and lobular adenocarcinoma, squamous and adenocarcinomas of the uterine cervix, uterine and ovarian epithelial carcinomas, prostatic adenocarcinomas, transitional squamous cell carcinoma of the bladder, B and T cell lymphomas (nodular and diffuse) plasmacytoma, acute and chronic leukemias, malignant melanoma, soft tissue sarcomas and leiomyosarcomas.

In certain embodiments, the cancer can be melanoma, NSCLC, head and neck cancer, urothelial cancer, breast cancer (e.g., triple-negative breast cancer, TNBC), gastric cancer, cholangiocarcinoma, classical Hodgkin's lymphoma (cHL), Non-Hodgkin lymphoma primary mediastinal B-Cell lymphoma (NHL PMBCL), mesothelioma, ovarian cancer, lung cancer (e.g., small-cell lung cancer), esophageal cancer, nasopharyngeal carcinoma (NPC), biliary tract cancer, colorectal cancer, cervical cancer or thyroid cancer.

In certain embodiments, the subject to be treated is a mammal (e.g., human, non-human primate, rat, mouse, cow, horse, pig, sheep, goat, dog, cat, etc.). In certain embodiments, the subject is a human. In certain embodiments, the subject is suspected of having or at risk of having a cancer or be diagnosed with a cancer or any other disease having abnormal LIV-1 expression or activity.

Many diagnostic methods for cancer or any other disease exhibiting abnormal LIV-1 activity and the clinical delineation of those diseases are known in the art. Such methods include, but are not limited to, e.g., immunohistochemistry, PCR, fluorescent in situ hybridization (FISH). Additional details regarding diagnostic methods for abnormal LIV-1 activity or expression are described in, e.g., Gupta et al. (2009) Mod Pathol. 22(1): 128-133; Lopez-Rios et al. (2013) J Clin Pathol. 66(5): 381-385; Ellison et al. (2013) J Clin Pathol 66(2): 79-89; and Guha et al. (2013) PLoS ONE 8(6): e67782.

Administration can be by any suitable route including, e.g., intravenous, intramuscular, or subcutaneous. In some embodiments, the antibody or antibody derivative (or fragments thereof) and/or compositions provided herein are administered in combination with a second, third, or fourth agent (including, e.g., an antineoplastic agent, a growth inhibitory agent, a cytotoxic agent, or a chemotherapeutic agent) to treat the diseases or disorders involving abnormal LIV-1 activity. Such agents include, e.g., docetaxel, gefitinib, FOLFIRI (irinotecan, 5-fluorouracil, and leucovorin), irinotecan, cisplatin, carboplatin, paclitaxel, bevacizumab (anti-VEGF antibody), FOLFOX-4, infusional fluorouracil, leucovorin, and oxaliplatin, afatinib, gemcitabine, capecitabine, pemetrexed, tivantinib, everolimus, CpG-ODN, rapamycin, lenalidomide, vemurafenib, endostatin, lapatinib, PX-866, Imprime PGG, and irlotinibm. In some embodiments, the antibody or antibody derivative (or fragments thereof) is conjugated to the additional agent.

In certain embodiments, the antibody or antibody derivative (or fragments thereof) and/or compositions provided herein are administered in combination with one or more additional therapies, such as radiation therapy, surgery, chemotherapy, and/or targeted therapy. In certain embodiments, the antibody, antibody derivative (or fragments thereof), antibody conjugate and/or composition provided herein are administered in combination with radiation therapy. In certain embodiments, the combination of an antibody, antibody derivative (or fragment thereof), antibody conjugate and/or composition provided herein and radiation therapy is used for treating a neoplasm or cancer disclosed herein.

Depending on the indication to be treated and factors relevant to the dosing that a physician of skill in the field would be familiar with, the antibody, antibody derivative or antibody conjugate provided herein will be administered at a dosage that is efficacious for the treatment of that indication while minimizing toxicity and side effects. For the treatment of a cancer, a typical dose can be, for example, in the rage of 0.001 to 1000 µg; however, doses below or above this exemplary range are within the scope of the invention. The daily dose can be about 0.1 µg /kg to about 100 mg/kg of total body weight, about 0.1 µg /kg to about 100 µg/kg of total body weight or about 1 µg /kg to about 100 µg/kg of total body weight. As noted above, therapeutic or prophylactic efficacy can be monitored by periodic assessment of treated patients. For repeated administrations over several days or longer, depending on the condition, the treatment is repeated until a desired suppression of disease symptoms occurs. However, other dosage regimens may be useful and are within the scope of the invention. The desired dosage can be delivered by a single bolus administration of the composition, by multiple bolus administrations of the composition, or by continuous infusion administration of the composition.

A pharmaceutical composition comprising an antibody or antibody derivative disclosed herein can be administered one, two, three, or four times daily. The compositions can also be administered less frequently than daily, for example, six times a week, five times a week, four times a week, three times a week, twice a week, once a week, once every two weeks, once every three weeks, once a month, once every two months, once every three months, or once every six months. The compositions may also be administered in a sustained release formulation, such as in an implant which gradually releases the composition for use over a period of time, and which allows for the composition to be administered less frequently, such as once a month, once every 2-6 months, once every year, or even a single administration. The sustained release devices (such as pellets, nanoparticles, microparticles, nanospheres, microspheres, and the like) may be administered by injection or surgically implanted in various locations.

Cancer treatments can be evaluated by, e.g., but not limited to, tumor regression, tumor weight or size shrinkage, time to progression, duration of survival, progression free survival, overall response rate, duration of response, quality of life, protein expression and/or activity. Approaches to determining efficacy of the therapy can be employed, including for example, measurement of response through radiological imaging.

In certain embodiments, the efficacy of treatment is measured by the percentage tumor growth inhibition (% TGI), calculated using the equation 100-(T/C x 100), where T is the mean relative tumor volume of the treated tumor, and C is the mean relative tumor volume of a non-treated tumor. In certain embodiments, the %TGI is about 10%, about 20%, about 30%, about 40%, about 50%, about 60%, about 70%, about 80%, about 90%, about 91%, about 92%, about 93%), about 94%), about 95%, or more than 95%.

### 3.2 Methods of Diagnosis and Imaging

Labeled antibody or antibody derivative can be used for diagnostic purposes to detect, diagnose, or monitor diseases and/or disorders associated with the expression, aberrant expression and/or activity of LIV-1. For example, the antibodies and antibody derivatives provided herein can be used in in situ, in vivo, ex vivo, and in vitro diagnostic assays or imaging assays. Methods for detecting expression of a LIV-1 polypeptide, comprising (a) assaying the expression of the polypeptide in cells (e.g., tissue) or body fluid of an individual using one or more antibody or antibody derivative and (b) comparing the level of gene expression with a standard gene expression level, whereby an increase or decrease in the assayed gene expression level compared to the standard expression level is indicative of aberrant expression.

Additional embodiments provided herein include methods of diagnosing a disease or disorder associated with expression or aberrant expression of LIV-1 in an animal (e.g., a mammal such as a human). The methods comprise detecting LIV-1 molecules in the mammal. In certain embodiments, diagnosis comprises: (a) administering an effective amount of a labeled antibody or antibody derivative to a mammal (b) waiting for a time interval following the administering for permitting the labeled antibody or antibody derivative to preferentially concentrate at sites in the subject where the LIV-1 molecule is expressed (and for unbound labeled molecule to be cleared to background level); (c) determining background level; and (d) detecting the labeled molecule in the subject, such that detection of labeled molecule above the background level indicates that the subject has a particular disease or disorder associated with expression or aberrant expression of LIV-1. Background level can be determined by various methods including, comparing the amount of labeled molecule detected to a standard value previously determined for a particular system.

Antibodies and antibody derivatives provided herein can be used to assay protein levels in a biological sample using classical immunohistological methods known to those of skill in the art (e.g., see Jalkanen, et al., J. Cell. Biol. 101:976-985 (1985); Jalkanen, et al., J. Cell. Biol. 105:3087-3096 (1987)). Other antibody-based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase; radioisotopes, such as iodine (¹³¹I, ¹²⁵I, ¹²³I, ¹²¹I), carbon (¹⁴C), sulfur (³⁵S), tritium (³H), indium (^{115m}In, ^{113m}In, ¹¹²In, ¹¹¹In), and technetium (⁹⁹Tc, ^{99m}Tc), thallium (²⁰¹Ti), gallium (⁶⁸Ga, ⁶⁷Ga), palladium (¹⁰³Pd), molybdenum (⁹⁹Mo), xenon (¹³³Xe), fluorine (¹⁸F), ¹⁵³Sm, ¹⁷⁷Lu, ¹⁵⁹Gd, ¹⁴⁹Pm, ¹⁴⁰La, ¹⁷⁵Yb , ¹⁶⁶Ho, ⁹⁰Y, ⁴⁷Sc, ¹⁸⁶Re, ¹⁸⁸Re, ¹⁴²Pr, ¹⁰⁵Rh, ⁹⁷Ru; luminol; and fluorescent labels, such as fluorescein and rhodamine, and biotin.

Techniques known in the art may be applied to labeled antibodies (or fragments thereof) provided herein. Such techniques include, but are not limited to, the use of bifunctional conjugating agents (see e.g., U.S. Pat. Nos. 5,756,065; 5,714,631; 5,696,239; 5,652,361; 5,505,931; 5,489,425; 5,435,990; 5,428,139; 5,342,604; 5,274,119; 4,994,560; and 5,808,003).

Alternatively, or additionally, one can measure levels of a LIV-1 polypeptide-encoding nucleic acid or mRNA in the cell, e.g., via fluorescent in situ hybridization using a nucleic acid based probe corresponding to an LIV-1-encoding nucleic acid or the complement thereof; (FISH; see WO98/45479 published October, 1998), Southern blotting, Northern blotting, or polymerase chain reaction (PCR) techniques, such as real time quantitative PCR (RT-PCR). One can also study LIV-1 overexpression by measuring shed antigen in a biological fluid such as serum, e.g., using antibody-based assays (see also, e.g., U.S. Patent No. 4,933,294 issued June 12, 1990; WO91/05264 published April18, 1991; U.S. Patent 5,401,638 issued March 28, 1995; and Sias et al., J. Immunol. Methods 132:73-80 (1990)). Aside from the above assays, various in vivo and ex vivo assays are available to the skilled practitioner. For example, one can expose cells within the body of the mammal to an antibody which is optionally labeled with a detectable label, e.g., a radioactive isotope, and binding of the antibody to the body cells can be evaluated, e.g., by external scanning for radioactivity or by analyzing a sample (e.g., a biopsy or other biological sample) taken from a mammal previously exposed to the antibody.

### 4. PHARMACEUTICAL FORMULATIONS

The presently disclosed subject matter further provides pharmaceutical formulations containing an antibody, antibody derivative or antibody conjugate disclosed herein, with a pharmaceutically acceptable carrier. In certain embodiments, the pharmaceutical compositions can include a combination of multiple (e.g., two or more) antibodies and/or antibody derivatives of the presently disclosed subject matter.

In certain embodiments, the disclosed pharmaceutical formulations can be prepared by combining an antibody, antibody derivative or antibody conjugate having the desired degree of purity with one or more optional pharmaceutically acceptable carriers (Remington's Pharmaceutical Sciences 16th edition, Osol, A. Ed. (1980)), in the form of lyophilized formulations or aqueous solutions. For example, but not by way of limitation, lyophilized antibody formulations are described in US Patent No. 6,267,958. In certain embodiments, aqueous antibody formulations can include those described in US Patent No. 6,171 ,586 and WO2006/044908, the latter formulations including a histidine-acetate buffer. In certain embodiments, the antibody, antibody derivative or antibody conjugate can be of a purity greater than about 80%, greater than about 90%, greater than about 91%, greater than about 92%, greater than about 93%, greater than about 94%, greater than about 95%, greater than about 96%, greater than about 97%, greater than about 98%, greater than about 99%, greater than about 99.1%, greater than about 99.2%, greater than about 99.3%, greater than about 99.4%, greater than about 99.5%, greater than about 99.6%, greater than about 99.7%, greater than about 99.8% or greater than about 99.9%.

Pharmaceutically acceptable carriers are generally nontoxic to recipients at the dosages and concentrations employed, and include, but are not limited to: buffers such as phosphate, citrate, and other organic acids, antioxidants including ascorbic acid and methionine, preservatives (such as octadecyldimethylbenzyl ammonium chloride, hexamethonium chloride, benzalkonium chloride, benzethonium chloride, phenol, butyl or benzyl alcohol, alkyl parabens such as methyl or propyl paraben, catechol, resorcinol, cyclohexanol, 3-pentanol, and m-cresol), low molecular weight (less than about 10 residues) polypeptides, proteins, such as serum albumin, gelatin, or immunoglobulins, hydrophilic polymers such as polyvinylpyrrolidone, amino acids such as glycine, glutamine, asparagine, histidine, arginine, or lysine, monosaccharides, disaccharides, and other carbohydrates including glucose, mannose, or dextrins, chelating agents such as EDTA, sugars such as sucrose, mannitol, trehalose or sorbitol, salt-forming counter-ions such as sodium, metal complexes (e.g., Zn-protein complexes), and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rHuPH20 (HYLENEX^{®}, Baxter International, Inc.). Certain exemplary sHASEGPs and methods of use, including rHuPH20, are described in US Patent Publication Nos. 2005/0260186 and 2006/0104968. In certain embodiments, a sHASEGP is combined with one or more additional glycosaminoglycanases such as chondroitinases.

The carrier can be suitable for intravenous, intramuscular, subcutaneous, parenteral, spinal or epidermal administration (e.g., by injection or infusion). Depending on the route of administration, the active compound, e.g., an anti-LIV-1 antibody, can be coated in a material to protect the compound from the action of acids and other natural conditions that may inactivate the compound.

Pharmaceutical compositions of the present disclosure also can be administered in combination therapy, i.e., combined with other agents. In certain embodiments, pharmaceutical compositions disclosed herein can also contain more than one active ingredient as necessary for the particular indication being treated, for example, those with complementary activities that do not adversely affect each other. In certain embodiments, the pharmaceutical formulation can include a second active ingredient for treating the same disease treated by the first therapeutic. Such active ingredients are suitably present in combination in amounts that are effective for the purpose intended. For example, and not by way of limitation, the formulation of the present disclosure can also contain more than one active ingredient as necessary for the particular indication being treated, preferably those with complementary activities that do not adversely affect each other. For example, it may be desirable to further provide a second therapeutic useful for treatment of the same disease. Such active ingredients are suitably present in combination in amounts that are effective for the purpose intended.

A composition of the present disclosure can be administered by a variety of methods known in the art. The route and/or mode of administration vary depending upon the desired results. The active compounds can be prepared with carriers that protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are described by e.g., Sustained and Controlled Release Drug Delivery Systems, J.R. Robinson, ed., Marcel Dekker, Inc., New York, 1978. In certain embodiments, the pharmaceutical compositions are manufactured under Good Manufacturing Practice (GMP) conditions of the U.S. Food and Drug Administration.

Sustained-release preparations containing an antibody, antibody derivative or antibody conjugate disclosed herein can also be prepared. Suitable examples of sustained-release preparations include semipermeable matrices of solid hydrophobic polymers containing the antibody, antibody derivative or antibody conjugate, which matrices are in the form of shaped articles, e.g. films, or microcapsules. In certain embodiments, active ingredients can be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

To administer an antibody, antibody derivative or antibody conjugate of the present disclosure by certain routes of administration, it may be necessary to coat the compound with, or co-administer the compound with, a material to prevent its inactivation. For example, the compound may be administered to a subject in an appropriate carrier, for example, liposomes, or a diluent. Pharmaceutically acceptable diluents include saline and aqueous buffer solutions. Liposomes include water-in-oil-in-water CGF emulsions as well as conventional liposomes (Strejan et al. (1984) J Neuroimmunol. 7:27).

Pharmaceutically acceptable carriers include sterile aqueous solutions or dispersions and sterile powders for the extemporaneous preparation of sterile injectable solutions or dispersion. The use of such media and agents for pharmaceutically active substances is known in the art.

Except insofar as any conventional media or agent is incompatible with the active compound, use thereof in the pharmaceutical compositions of the present disclosure is contemplated. Supplementary active compounds can also be incorporated into the compositions.

Therapeutic compositions typically must be sterile, substantially isotonic, and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it is preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin.

Sterile injectable solutions can be prepared by incorporating one or more antibody, antibody derivative or antibody conjugate disclosed herein in the required amount in an appropriate solvent with one or a combination of ingredients enumerated above, as required, followed by sterilization microfiltration, e.g., by filtration through sterile filtration membranes. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

Therapeutic compositions can also be administered with medical devices known in the art. For example, a therapeutic composition of the present disclosure can be administered with a needleless hypodermic injection device, such as the devices disclosed in, e.g., U.S. Patent Nos. 5,399,163, 5,383,851, 5,312,335, 5,064,413, 4,941,880, 4,790,824 or 4,596,556. Examples of implants and modules useful in the present disclosure include: U.S. Patent No. 4,487,603, which discloses an implantable micro-infusion pump for dispensing medication at a controlled rate; U.S. Patent No. 4,486,194, which discloses a therapeutic device for administering medicants through the skin; U.S. Patent No. 4,447,233, which discloses a medication infusion pump for delivering medication at a precise infusion rate; U.S. Patent No. 4,447,224, which discloses a variable flow implantable infusion apparatus for continuous drug delivery; U.S. Patent No. 4,439,196, which discloses an osmotic drug delivery system having multi-chamber compartments; and U.S. Patent No. 4,475,196, which discloses an osmotic drug delivery system. Many other such implants, delivery systems, and modules are known.

For the therapeutic compositions, formulations of the present disclosure include those suitable for oral, nasal, topical (including buccal and sublingual), rectal, vaginal and/or parenteral administration. The formulations can conveniently be presented in unit dosage form and may be prepared by any methods known in the art of pharmacy. The amount of antibody, antibody derivative or antibody conjugate, which can be combined with a carrier material to produce a single dosage form, vary depending upon the subject being treated, and the particular mode of administration. The amount of the antibody, antibody derivative or antibody conjugate which can be combined with a carrier material to produce a single dosage form generally be that amount of the composition which produces a therapeutic effect. Generally, out of one hundred percent, this amount range from about 0.01 percent to about ninety-nine percent of active ingredient, from about 0.1 percent to about 70 percent, or from about 1 percent to about 30 per cent.

Dosage forms for the topical or transdermal administration of compositions of the present disclosure include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

The phrases "parenteral administration" and "administered parenterally" mean modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

These pharmaceutical compositions can also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form can be brought about by the inclusion of agents which delay absorption such as aluminum monostearate and gelatin.

In certain embodiments, when an antibody, antibody derivative or antibody conjugate of the present disclosure are administered as pharmaceuticals, to humans and animals, they can be given alone or as a pharmaceutical composition containing, for example, from about 0.01% to about 99.5% (or about 0.1% to about 90%) of the antibody, antibody derivative or antibody conjugate in combination with a pharmaceutically acceptable carrier.

### 5. ARTICLES OF MANUFACTURE

The presently disclosed subject matter further provides articles of manufacture, e.g., kits, containing materials useful for the treatment, prevention and/or diagnosis of the disorders described above.

In certain embodiments, the article of manufacture/kit includes a container and a label or package insert on or associated with the container. Non limiting examples of suitable containers include bottles, vials, syringes, IV solution bags, etc. The containers can be formed from a variety of materials such as glass or plastic. The container can hold a composition which is by itself or combined with another composition effective for treating, preventing and/or diagnosing the condition and may have a sterile access port (for example, the container may be an intravenous solution bag or a vial having a stopper pierceable by a hypodermic injection needle).

In certain embodiments, at least one active agent in the composition is an antibody, antibody derivative or antibody conjugate of the present disclosure. The label or package insert can indicate that the composition is used for treating the condition of choice.

In certain embodiments, the article of manufacture/kit can comprise (a) a first container with a composition contained therein, wherein the composition comprises an antibody, antibody derivative or antibody conjugate of the present disclosure; and (b) a second container with a composition contained therein, wherein the composition comprises a further cytotoxic or otherwise therapeutic agent. In certain embodiments, the article of manufacture/kit can further comprise a package insert indicating that the compositions can be used to treat a particular condition.

Alternatively, or additionally, the article of manufacture/kit can further an additional container, e.g., a second or third container, including a pharmaceutically acceptable buffer, such as, but not limited to, bacteriostatic water for injection (BWFI), phosphate-buffered saline, Ringer's solution and dextrose solution. The article of manufacture/kit can include other materials desirable from a commercial and user standpoint, including other buffers, diluents, filters, needles, and syringes.

In addition, the present disclosure also relates to the following embodiments:
1. An antibody that binds to LIV-1 comprising:
   a) a heavy chain variable region comprising:
      (1) a heavy chain variable region CDR-H1 comprising an amino acid sequence of any one of SEQ ID NOs: 1, 11, 21, 31, and 41, or a variant thereof comprising up to about 3 amino acid substitutions;
      (2) a heavy chain variable region CDR-H2 comprising an amino acid sequence of any one of SEQ ID NOs: 2, 12, 22, 32, and 42, or a variant thereof comprising up to about 3 amino acid substitutions; and
      (3) a heavy chain variable region CDR-H3 comprising an amino acid sequence of any one of SEQ ID NOs: 3, 13, 23, 33, and 43, or a variant thereof comprising up to about 3 amino acid substitutions; and
   b) a light chain variable region comprising:
      (1) a light chain variable region CDR-L1 comprising an amino acid sequence of any one of SEQ ID NOs: 4, 14, 24, 34, and 44, or a variant thereof comprising up to about 3 amino acid substitutions;
      (2) a light chain variable region CDR-L2 comprising an amino acid sequence of any one of SEQ ID NOs: 5, 15, 25, 35, and 45, or a variant thereof comprising up to about 3 amino acid substitutions; and
      (3) a light chain variable region CDR-L3 comprising an amino acid sequence of any one of SEQ ID NOs: 6, 16, 26, 36, and 46, or a variant thereof comprising up to about 3 amino acid substitutions.
2. The antibody of embodiment 1, wherein the antibody binds to LIV-1 with a KD of about 1x10⁻⁹ M or less.
3. The antibody of embodiment 1 or 2, wherein the antibody binds to LIV-1 with a KD of about 5x10⁻¹⁰ M or less.
4. The antibody of any one of embodiments 1-3, wherein antibody binds to LIV-1 with a KD of between about 1x10⁻¹¹ M and about 1x10⁻¹⁰ M.
5. The antibody of any one of embodiments 1-4, wherein the antibody binds to LIV-1 with a KD of between about 1x10⁻¹¹ M and about 5x10⁻¹¹ M.
6. The antibody of any one of embodiments 1-5, wherein the antibody cross-competes with a reference anti-LIV-1 antibody comprising:
   a) a heavy chain variable domain (VH) sequence comprising (1) a CDR-H1 comprising the amino acid sequence set forth in SEQ ID NO: 1, (2) a CDR- H2 comprising the amino acid sequence set forth in SEQ ID NO: 2, and (3) a CDR-H3 comprising the amino acid sequence set forth in SEQ ID NO: 3; and a light chain variable domain (VL) sequence comprising (1) a CDR-L1 comprising the amino acid sequence set forth in SEQ ID NO: 4, (2) a CDR-L2 comprising the amino acid sequence set forth in SEQ ID NO: 5, and (3) a CDR-L3 comprising the amino acid sequence set forth in SEQ ID NO: 6;
   b) a heavy chain variable domain (VH) sequence comprising (1) a CDR-H1 comprising the amino acid sequence set forth in SEQ ID NO: 11, (2) a CDR- H2 comprising the amino acid sequence set forth in SEQ ID NO: 12, and (3) a CDR-H3 comprising the amino acid sequence set forth in SEQ ID NO: 13; and a light chain variable domain (VL) sequence comprising (1) a CDR-L1 comprising the amino acid sequence set forth in SEQ ID NO: 14, (2) a CDR-L2 comprising the amino acid sequence set forth in SEQ ID NO: 15, and (3) a CDR-L3 comprising the amino acid sequence set forth in SEQ ID NO: 16;
   c) a heavy chain variable domain (VH) sequence comprising (1) a CDR-H1 comprising the amino acid sequence set forth in SEQ ID NO: 21, (2) a CDR- H2 comprising the amino acid sequence set forth in SEQ ID NO: 22, and (3) a CDR-H3 comprising the amino acid sequence set forth in SEQ ID NO: 23; and a light chain variable domain (VL) sequence comprising (1) a CDR-L1 comprising the amino acid sequence set forth in SEQ ID NO: 24, (2) a CDR-L2 comprising the amino acid sequence set forth in SEQ ID NO: 25, and (3) a CDR-L3 comprising the amino acid sequence set forth in SEQ ID NO: 26;
   d) a heavy chain variable domain (VH) sequence comprising (1) a CDR-H1 comprising the amino acid sequence set forth in SEQ ID NO: 31, (2) a CDR- H2 comprising the amino acid sequence set forth in SEQ ID NO: 32, and (3) a CDR-H3 comprising the amino acid sequence set forth in SEQ ID NO: 33; and a light chain variable domain (VL) sequence comprising (1) a CDR-L1 comprising the amino acid sequence set forth in SEQ ID NO: 34, (2) a CDR-L2 comprising the amino acid sequence set forth in SEQ ID NO: 35, and (3) a CDR-L3 comprising the amino acid sequence set forth in SEQ ID NO: 36; or
   e) a heavy chain variable domain (VH) sequence comprising (1) a CDR-H1 comprising the amino acid sequence set forth in SEQ ID NO: 41, (2) a CDR- H2 comprising the amino acid sequence set forth in SEQ ID NO: 42, and (3) a CDR-H3 comprising the amino acid sequence set forth in SEQ ID NO: 43; and a light chain variable domain (VL) sequence comprising (1) a CDR-L1 comprising the amino acid sequence set forth in SEQ ID NO: 44, (2) a CDR-L2 comprising the amino acid sequence set forth in SEQ ID NO: 45, and (3) a CDR-L3 comprising the amino acid sequence set forth in SEQ ID NO: 46.
7. The antibody of any one of embodiments 1-6, wherein the antibody comprises:
   a) a heavy chain variable region that comprises a CDR-H1 domain, a CDR-H2 domain and a CDR-H3 domain, wherein the CDR-H1 domain, the CDR-H2 domain and the CDR-H3 domain respectively comprise a CDR-H1 domain, a CDR-H2 domain and a CDR-H3 domain comprised in a reference heavy chain variable region comprising the amino acid sequence selected from the group consisting of SEQ ID NOs: 7, 17, 27, 37, and 47; and
   b) a light chain variable region that comprises a CDR-L1 domain, a CDR-L2 domain and a CDR-L3 domain, wherein the CDR-L1 domain, the CDR-L2 domain and the CDR-L3 domain respectively comprise a CDR-L1 domain, a CDR-L2 domain and a CDR-L3 domain comprised in a reference light chain variable region comprising the amino acid sequence selected from the group consisting of SEQ ID NOs: 8, 18, 28, 38, and 48.
8. The antibody of any one of embodiments 1-7, wherein the antibody comprises a heavy chain variable domain (VH) sequence comprising (1) a CDR-H1 comprising the amino acid sequence set forth in SEQ ID NO: 1, (2) a CDR- H2 comprising the amino acid sequence set forth in SEQ ID NO: 2, and (3) a CDR-H3 comprising the amino acid sequence set forth in SEQ ID NO: 3; and a light chain variable domain (VL) sequence comprising (1) a CDR-L1 comprising the amino acid sequence set forth in SEQ ID NO: 4, (2) a CDR-L2 comprising the amino acid sequence set forth in SEQ ID NO: 5, and (3) a CDR-L3 comprising the amino acid sequence set forth in SEQ ID NO: 6.
9. The antibody of any one of embodiments 1-7, wherein the antibody comprises a heavy chain variable domain (VH) sequence comprising (1) a CDR-H1 comprising the amino acid sequence set forth in SEQ ID NO: 11, (2) a CDR- H2 comprising the amino acid sequence set forth in SEQ ID NO: 12, and (3) a CDR-H3 comprising the amino acid sequence set forth in SEQ ID NO: 13; and a light chain variable domain (VL) sequence comprising (1) a CDR-L1 comprising the amino acid sequence set forth in SEQ ID NO: 14, (2) a CDR-L2 comprising the amino acid sequence set forth in SEQ ID NO: 15, and (3) a CDR-L3 comprising the amino acid sequence set forth in SEQ ID NO: 16.
10. The antibody of any one of embodiments 1-7, wherein the antibody comprises a heavy chain variable domain (VH) sequence comprising (1) a CDR-H1 comprising the amino acid sequence set forth in SEQ ID NO: 21, (2) a CDR- H2 comprising the amino acid sequence set forth in SEQ ID NO: 22, and (3) a CDR-H3 comprising the amino acid sequence set forth in SEQ ID NO: 23; and a light chain variable domain (VL) sequence comprising (1) a CDR-L1 comprising the amino acid sequence set forth in SEQ ID NO: 24, (2) a CDR-L2 comprising the amino acid sequence set forth in SEQ ID NO: 25, and (3) a CDR-L3 comprising the amino acid sequence set forth in SEQ ID NO: 26.
11. The antibody of any one of embodiments 1-7, wherein the antibody comprises a heavy chain variable domain (VH) sequence comprising (1) a CDR-H1 comprising the amino acid sequence set forth in SEQ ID NO: 31, (2) a CDR- H2 comprising the amino acid sequence set forth in SEQ ID NO: 32, and (3) a CDR-H3 comprising the amino acid sequence set forth in SEQ ID NO: 33; and a light chain variable domain (VL) sequence comprising (1) a CDR-L1 comprising the amino acid sequence set forth in SEQ ID NO: 34, (2) a CDR-L2 comprising the amino acid sequence set forth in SEQ ID NO: 35, and (3) a CDR-L3 comprising the amino acid sequence set forth in SEQ ID NO: 36.
12. The antibody of any one of embodiments 1-7, wherein the antibody comprises a heavy chain variable domain (VH) sequence comprising (1) a CDR-H1 comprising the amino acid sequence set forth in SEQ ID NO: 41, (2) a CDR- H2 comprising the amino acid sequence set forth in SEQ ID NO: 42, and (3) a CDR-H3 comprising the amino acid sequence set forth in SEQ ID NO: 43; and a light chain variable domain (VL) sequence comprising (1) a CDR-L1 comprising the amino acid sequence set forth in SEQ ID NO: 44, (2) a CDR-L2 comprising the amino acid sequence set forth in SEQ ID NO: 45, and (3) a CDR-L3 comprising the amino acid sequence set forth in SEQ ID NO: 46.
13. The antibody of any one of embodiments 1-9, wherein the antibody comprises a heavy chain variable region comprising the amino acid sequence set forth in SEQ ID NO: 7, and a light chain variable region comprising the amino acid sequence set forth in SEQ ID NO: 8.
14. The antibody of any one of embodiments 1-9, wherein the antibody comprises a heavy chain variable region comprising the amino acid sequence set forth in SEQ ID NO: 17, and a light chain variable region comprising the amino acid sequence set forth in SEQ ID NO: 18.
15. The antibody of any one of embodiments 1-9, wherein the antibody comprises a heavy chain variable region comprising the amino acid sequence set forth in SEQ ID NO: 27, and a light chain variable region comprising the amino acid sequence set forth in SEQ ID NO: 28.
16. The antibody of any one of embodiments 1-9, wherein the antibody comprises a heavy chain variable region comprising the amino acid sequence set forth in SEQ ID NO: 37, and a light chain variable region comprising the amino acid sequence set forth in SEQ ID NO: 38.
17. The antibody of any one of embodiments 1-9, wherein the antibody comprises a heavy chain variable region comprising the amino acid sequence set forth in SEQ ID NO: 47, and a light chain variable region comprising the amino acid sequence set forth in SEQ ID NO: 48.
18. The antibody of any one of embodiments 1-17, wherein the antibody comprises a human framework.
19. The antibody of any one of embodiments 1-18, wherein the antibody is a human antibody.
20. The antibody of any one of embodiments 1-19, wherein the antibody comprises a full-length immunoglobulin, a single-chain Fv (scFv) fragment, a Fab fragment, a Fab' fragment, a F(ab')2, an Fv fragment, a disulfide stabilized Fv fragment (dsFv), a (dsFv)2, a Fv-Fc fusion, a scFv-Fc fusion, a scFv-Fv fusion, a diabody, a tribody, a tetrabody or any combination thereof.
21. The antibody of any one of embodiments 1-20**,** wherein the antibody comprises a Fc region.
22. The antibody of embodiment 21, wherein the Fc region comprises a human Fc region.
23. The antibody of embodiment 21 or 22**,** wherein the Fc region comprises a Fc region selected from the group consisting of the Fc regions of IgG, IgA, IgD, IgE and IgM.
24. The antibody of any one of embodiments 21-23, wherein the Fc region comprises a Fc region selected from the group consisting of the Fc regions of IgG1, IgG2, IgG3 and IgG4.
25. The antibody of any one of embodiments 21-24, wherein the Fc region comprises an IgG1 Fc region or an IgG4 Fc region.
26. The antibody of any one of embodiments 21-25, wherein the Fc region comprises a C-terminal lysine.
27. The antibody of any one of embodiments 21-25, wherein the Fc region comprises a deletion of a C-terminal lysine.
28. The antibody of any one of embodiments 1-27, wherein the antibody is comprised in a multispecific antibody, e.g., a bispecific antibody, wherein the multispecific antibody comprises a second antibody moiety that specifically binds to a second antigen.
29. The antibody of embodiment 28, wherein the second antigen is a tumor associated antigen.
30. The antibody of embodiment 29, wherein the tumor associated antigen is selected from the group consisting of Her-2, EGFR, PDL1, MSLN, c-Met, B Cell Maturation Antigen (BCMA), carbonic anhydrase IX (CA1X), carcinoembryonic antigen (CEA), CD5, CD7, CD10, CD19, CD20, CD22, CD30, CD33, CD34, CD38, CD41, CD44, CD49f, CD56, CD74, CD123, CD133, CD138, CD276 (B7H3), epithelial glycoprotein (EGP2), trophoblast cell-surface antigen 2 (TROP-2), epithelial glycoprotein- 40 (EGP-40), epithelial cell adhesion molecule (EpCAM), receptor tyrosine-protein kinases erb-B2,3,4, folate-binding protein (FBP), fetal acetylcholine receptor (AChR), folate receptor-a, Ganglioside G2 (GD2), Ganglioside G3 (GD3), human telomerase reverse transcriptase (hTERT), kinase insert domain receptor (KDR), Lewis A (CA 1.9.9), Lewis Y (LeY), Glypican-3 (GPC3), L1 cell adhesion molecule (L1CAM), Mucin 16 (Muc-16), Mucin 1 (Muc-1), NG2D ligands, oncofetal antigen (h5T4), prostate stem cell antigen (PSCA), prostate-specific membrane antigen (PSMA), tumor- associated glycoprotein 72 (TAG-72), Claudin18.2 (CLDN18.2), vascular endothelial growth factor R2 (VEGF- R2), Wilms tumor protein (WT-1), type 1 tyrosine-protein kinase transmembrane receptor (ROR1), PVR, PVRL2, and any combination thereof.
31. An immunoconjugate comprising the antibody of any one of embodiments 1-30, linked to a therapeutic agent or a label.
32. The immunoconjugate of embodiment 31, wherein the therapeutic agent is a cytotoxin or a radioactive isotope.
33. The immunoconjugate of embodiment 32, wherein the cytotoxin is selected from the group consisting of amanitin, auristatin, calicheamicin, camptothecin, cryptophycin, daunomycin, dolastatin, doxorubicin, duocarmycin, epothilone, esperamicin, geldanamycin, maytansinoid, methotrexate, monomethyl auristatin E ("MMAE"), monomethyl auristatin F ("MMAF"), pyrrolobenzodiazepine, SN-38, rhizoxin, SG2285, tubulysin, vindesine, toxoid, or any combination thereof.
34. The immunoconjugate of embodiment 32 or 33, wherein the antibody and the cytotoxin are linked via a linker.
35. The immunoconjugate of embodiment 34, wherein the linker comprises a maleimidocaproyl group, one or more natural or unnatural amino acids, and a self-immolative group.
36. The immunoconjugate of embodiment 31, wherein the label is selected from the group consisting of a radioisotope, a fluorescent dye and an enzyme.
37. A chimeric antigen receptor (CAR) comprising an extracellular antigen-binding domain that comprises an antibody of any one of embodiments 1-30.
38. The CAR of embodiment 37, wherein the antibody is a scFv.
39. An immunoresponsive cell comprising a CAR of embodiment 37 or 38.
40. The immunoresponsive cell of embodiment 39, wherein the immunoresponsive cell is selected from the group consisting of a T cell, a Natural Killer (NK) cell, a cytotoxic T lymphocyte (CTL), a regulatory T cell, a Natural Killer T (NKT) cell and a myeloid cell.
41. The immunoresponsive cell of embodiment 39 or 40, wherein the immunoresponsive cell is a T cell.
42. A pharmaceutical composition comprising a) the antibody of any one of embodiments 1-30, the immunoconjugate of any one of embodiments 31-36, or the immunoresponsive cell of any one of embodiments 39-41, and b) a pharmaceutically acceptable carrier.
43. A nucleic acid encoding the antibody of any one of embodiments 1-30.
44. A vector comprising the nucleic acid of embodiment 43.
45. A host cell comprising the nucleic acid of embodiment 43 or the vector of embodiment 44.
46. A method for preparing an antibody of any one of embodiments 1-30 comprising expressing the antibody in the host cell of embodiment 45 and isolating the antibody from the host cell.
47. A method of reducing tumor burden in a subject, the method comprising administering to the subject an effective amount of the antibody of any one of embodiments 1-30, the immunoconjugate of any one of embodiments 31-36, the immunoresponsive cell of any one of embodiments 39-41, or the pharmaceutical composition of embodiment 42.
48. The method of embodiment 47, wherein the method reduces the number of tumor cells.
49. The method of embodiment 47 or 48, wherein the method reduces tumor size.
50. The method of any one of embodiments 47-49, wherein the method eradicates the tumor in the subject.
51. The method of any one of embodiments 47-50, wherein the tumor is selected from the group consisting of mesothelioma, lung cancer, pancreatic cancer, ovarian cancer, breast cancer, colon cancer, pleural tumor, glioblastoma, esophageal cancer, gastric cancer, synovial sarcoma, thymic carcinoma, endometrial carcinoma, stomach cancer, cholangiocarcinoma, head and neck cancer, blood cancer, prostate cancer, cervical cancer, melanoma, squamous cell carcinoma, liver cancer and a combination thereof.
52. A method of treating and/or preventing a neoplasm, the method comprising administering to the subject an effective amount of the antibody of any one of embodiments 1-30, the immunoconjugate of any one of embodiments 31-36, the immunoresponsive cell of any one of embodiments 39-41, or the pharmaceutical composition of embodiment 42.
53. A method of lengthening survival of a subject having a neoplasm, the method comprising administering to the subject an effective amount of the antibody of any one of embodiments 1-30, the immunoconjugate of any one of embodiments 31-36, the immunoresponsive cell of any one of embodiments 39-41, or the pharmaceutical composition of embodiment 42.
54. The method of embodiment 52 or 53, wherein the neoplasm is selected from the group consisting of mesothelioma, lung cancer, pancreatic cancer, ovarian cancer, breast cancer, colon cancer, pleural tumor, glioblastoma, esophageal cancer, gastric cancer, synovial sarcoma, thymic carcinoma, endometrial carcinoma, stomach cancer, cholangiocarcinoma, head and neck cancer, blood cancer, prostate cancer, cervical cancer, melanoma, squamous cell carcinoma, liver cancer and a combination thereof.
55. An antibody of any one of embodiments 1-30 for use as a medicament.
56. An antibody of any one of embodiments 1-30 for use in treating cancer.
57. A pharmaceutical composition of embodiment 42 for use as a medicament.
58. A pharmaceutical composition of embodiment 42 for use in treating cancer.
59. The antibody of embodiment 56 or the pharmaceutical composition of embodiment 58, wherein the cancer is selected from the group consisting of mesothelioma, lung cancer, pancreatic cancer, ovarian cancer, breast cancer, colon cancer, pleural tumor, glioblastoma, esophageal cancer, gastric cancer, synovial sarcoma, thymic carcinoma, endometrial carcinoma, stomach cancer, cholangiocarcinoma, head and neck cancer, blood cancer, prostate cancer, cervical cancer, melanoma, squamous cell carcinoma, liver cancer and a combination thereof.
60. A kit comprising the antibody of any one of embodiments 1-30, the immunoconjugate of any one of embodiments 31-36, the pharmaceutical composition of embodiment 42, the nucleic acid of embodiment 43, the vector of embodiment 44, or the immunoresponsive cell of any one of embodiments 39-41.
61. The kit of embodiment 60, further comprising a written instruction for treating and/or preventing a neoplasm.
62. A use of the antibody of any one of embodiments 1-30, the immunoconjugate of any one of embodiments 31-36, the immunoresponsive cell of any one of embodiments 39-41, or the pharmaceutical composition of embodiment 42 in the manufacture of a medicament for reducing tumor burden in a subject, treating and/or preventing a neoplasm, or lengthening survival of a subject having a neoplasm.
63. The use of embodiment 62, wherein the tumor or neoplasm is selected from the group consisting of mesothelioma, lung cancer, pancreatic cancer, ovarian cancer, breast cancer, colon cancer, pleural tumor, glioblastoma, esophageal cancer, gastric cancer, synovial sarcoma, thymic carcinoma, endometrial carcinoma, stomach cancer, cholangiocarcinoma, head and neck cancer, blood cancer, prostate cancer, cervical cancer, melanoma, squamous cell carcinoma, liver cancer and a combination thereof.

### SEQUENCE TABLE

| **SEQ ID NO** | **NAME** | **AMINO ACID SEQUENCE** |
|---|---|---|
| 1. | 34C7D11 VH CDR1 | **GYAMS** |
| 2. | 34C7D11 VH CDR2 | **TISDVGSDTYYPDNLEG** |
| 3. | 34C7D11 VH CDR3 | **DDWDAYGMDF** |
| 4. | 34C7D11 VL CDR1 | **KSSQSLLHSDGETYLN** |
| 5. | 34C7D11 VL CDR2 | **MMSKLDS** |
| 6. | 34C7D11 VL CDR3 | **WQGTHFPQT** |
| 7. | 34C7D11 VH | EVQLVESGGGLVKPGGSLKLSCAASGFTFSGYAMSWVRQ TPEKRLEWVATISDVGSDTYYPDNLEGRFTISRDDAKNN LFLQMSHLRSADTAIYYCVRDDWDAYGMDFWGQGTVT VTP |
| 8. | 34C7D11 VL | DVVLTQTPLTLSVTIGQPASISCKSSQSLLHSDGETYLNW LLQRPGQSPKRLIYMMSKLDSGVPDRFSGSGSGTDFTLKV SRVEAEDLGIYYCWQGTHFPQTFGGGTKLEIK |
| 9. | 34C7D11 chimera HC | EVQLVESGGGLVKPGGSLKLSCAASGFTFSGYAMSWVRQ TPEKRLEWVATISDVGSDTYYPDNLEGRFTISRDDAKNN LFLQMSHLRSADTAIYYCVRDDWDAYGMDFWGQGTVT VTPASTKGPSVFPLAPSSKSTSGGTAALGCLVKDYFPEPVT VSWNSGALTSGVHTFPAVLQSSGLYSLSSVVTVPSSSLGT QTYICNVNHKPSNTKVDKKVEPKSCDKTHTCPPCPAPELL GGPSVFLFPPKPKDTLMISRTPEVTCVVVDVSHEDPEVKFN WYVDGVEVHNAKTKPREEQYNSTYRVVSVLTVLHQDWL NGKEYKCKVSNKALPAPIEKTISKAKGQPREPQVYTLPPSR EEMTKNQVSLTCLVKGFYPSDIAVEWESNGQPENNYKTT PPVLDSDGSFFLYSKLTVDKSRWQQGNVFSCSVMHEALH NHYTQKSLSLSPGK |
| 10. | 34C7D11 chimera LC | DVVLTQTPLTLSVTIGQPASISCKSSQSLLHSDGETYLNW LLQRPGQSPKRLIYMMSKLDSGVPDRFSGSGSGTDFTLKV SRVEAEDLGIYYCWQGTHFPQTFGGGTKLEIKRTVAAPS VFIFPPSDEQLKSGTASVVCLLNNFYPREAKVQWKVDNAL QSGNSQESVTEQDSKDSTYSLSSTLTLSKADYEKHKVYAC EVTHQGLSSPVTKSFNRGEC |
| 11. | 43E5C6 VH CDR1 | **SCAMS** |
| 12. | 43E5C6 VH CDR2 | **TISEDGGSTYYLDNLQG** |
| 13. | 43E5C6 VH CDR3 | **DDWDGYALDY** |
| 14. | 43E5C6 VL CDR1 | **ESSQSLLYSDGETYLN** |
| 15. | 43E5C6 VL CDR2 | **LVSKLDS** |
| 16. | 43E5C6 VL CDR3 | **WQGSHFPQT** |
| 17. | 43E5C6 VH | EVQLVESEGGLVKPGGSLKVSCVASGFTLSSCAMSWVRQ TPEKRLEWVATISEDGGSTYYLDNLQGRFTLSRDDAKNT LYLQMSHVKSDDTAIYYCVRDDWDGYALDYWGQGTSV TVTS |
| 18. | 43E5C6 VL | DVVMTQTPLTLSVTIGQPASISCESSQSLLYSDGETYLNW LLQRPGQSPKRLIYLVSKLDSGVPDRFTGSGSGTDFTLRIS RVEAEDLGIYYCWQGSHFPQTFGGGTKLEIK |
| 19. | 43E5C6 chimera HC | EVQLVESEGGLVKPGGSLKVSCVASGFTLSSCAMSWVRQ TPEKRLEWVATISEDGGSTYYLDNLQGRFTLSRDDAKNT LYLQMSHVKSDDTAIYYCVRDDWDGYALDYWGQGTSV TVTSASTKGPSVFPLAPSSKSTSGGTAALGCLVKDYFPEPV TVSWNSGALTSGVHTFPAVLQSSGLYSLSSVVTVPSSSLGT QTYICNVNHKPSNTKVDKKVEPKSCDKTHTCPPCPAPELL GGPSVFLFPPKPKDTLMISRTPEVTCVVVDVSHEDPEVKFN WYVDGVEVHNAKTKPREEQYNSTYRVVSVLTVLHQDWL NGKEYKCKVSNKALPAPIEKTISKAKGQPREPQVYTLPPSR EEMTKNQVSLTCLVKGFYPSDIAVEWESNGQPENNYKTT PPVLDSDGSFFLYSKLTVDKSRWQQGNVFSCSVMHEALH NHYTQKSLSLSPGK |
| 20. | 43E5C6 chimera LC | DVVMTQTPLTLSVTIGQPASISCESSQSLLYSDGETYLNW LLQRPGQSPKRLIYLVSKLDSGVPDRFTGSGSGTDFTLRIS RVEAEDLGIYYCWQGSHFPQTFGGGTKLEIKRTVAAPSV FIFPPSDEQLKSGTASVVCLLNNFYPREAKVQWKVDNALQ SGNSQESVTEQDSKDSTYSLSSTLTLSKADYEKHKVYACE VTHQGLSSPVTKSFNRGEC |
| 21. | 70B8F3D9 VH CDR1 | **SYWMQ** |
| 22. | 70B8F3D9 VH CDR2 | **EIDPLDSYTNYNQKFKG** |
| 23. | 70B8F3D9 VH CDR3 | **WGDRVDY** |
| 24. | 70B8F3D9 VL CDR1 | **KSSQSLLSSNGKTYLN** |
| 25. | 70B8F3D9 VL CDR2 | **LVSTLDS** |
| 26. | 70B8F3D9 VL CDR3 | **LQGTHFPYA** |
| 27. | 70B8F3D9 VH | QVQLQQPGAELVKPGTSVKLSCRSSGYTFTSYWMQWVR QRPGQGLEWIGEIDPLDSYTNYNQKFKGKATLTVDTSSS TAYMQLSVLTSEDSAVYSCAIWGDRVDYWGQGTSVTVS S |
| 28. | 70B8F3D9 VL | DVVMTQTPLSLSVTLGQPASISCKSSQSLLSSNGKTYLNW LLQRPGQSPKRLIYLVSTLDSGVPDRFAGSGSGTDFTLKIS RVEAEDLGVYYC LQGTHFPYA FGGGTKLELK |
| 29. | 70B8F3D9 chimera HC | QVQLQQPGAELVKPGTSVKLSCRSSGYTFTSYWMQWVR QRPGQGLEWIGEIDPLDSYTNYNQKFKGKATLTVDTSSS TAYMQLSVLTSEDSAVYSCAIWGDRVDYWGQGTSVTVS SASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPELLGG PSVFLFPPKPKDTLMISRTPEVTCVVVDVSHEDPEVKFNW YVDGVEVHNAKTKPREEQYNSTYRVVSVLTVLHQDWLN GKEYKCKVSNKALPAPIEKTISKAKGQPREPQVYTLPPSRE EMTKNQVSLTCLVKGFYPSDIAVEWESNGQPENNYKTTPP VLDSDGSFFLYSKLTVDKSRWQQGNVFSCSVMHEALHNH YTQKSLSLSPGK |
| 30. | 70B8F3D9 chimera LC | DVVMTQTPLSLSVTLGQPASISC**KSSQSLLSSNGKTYLN**W LLQRPGQSPKRLIY**LVSTLDS**GVPDRFAGSGSGTDFTLKIS RVEAEDLGVYYC**LQGTHFPYA**FGGGTKLELKRTVAAPS VFIFPPSDEQLKSGTASVVCLLNNFYPREAKVQWKVDNAL QSGNSQESVTEQDSKDSTYSLSSTLTLSKADYEKHKVYAC EVTHQGLSSPVTKSFNRGEC |
| 31. | h34C VH CDR1 | **GYAMS** |
| 32. | h34C VH CDR2 | **TISDVGSDTYYPDNLEG** |
| 33. | h34C VH CDR3 | **DDWDAYGMDF** |
| 34. | h34C VL CDR1 | **KSSQSLLHSDGETYLN** |
| 35. | h34C VL CDR2 | **MMSKLDS** |
| 36. | h34C VL CDR3 | **WQGTHFPQT** |
| 37. | h34C VH | EVQLVESGGGLVKPGGSLRLSCAASGFTFSGYAMSWVRQ APGKGLEWVATISDVGSDTYYPDNLEGRFTISRDNAKNS LYLQMNSLRAEDTAVYYCVRDDWDAYGMDFWGQGTL VTVSS |
| 38. | h34C VL | DVVMTQSPLSLPVTLGQPASISCKSSQSLLHSDGETYLNW LQQRPGQSPRRLIYMMSKLDSGVPDRFSGSGSGTDFTLKI SRVEAEDVGVYYCWQGTHFPQTFGGGTKVEIK |
| 39. | h34C HC | EVQLVESGGGLVKPGGSLRLSCAASGFTFSGYAMSWVRQ APGKGLEWVATISDVGSDTYYPDNLEGRFTISRDNAKNS LYLQMNSLRAEDTAVYYCVRDDWDAYGMDFWGQGTL VTVSSASTKGPSVFPLAPSSKSTSGGTAALGCLVKDYFPEP VTVSWNSGALTSGVHTFPAVLQSSGLYSLSSVVTVPSSSL GTQTYICNVNHKPSNTKVDKKVEPKSCDKTHTCPPCPAPE LLGGPSVFLFPPKPKDTLMISRTPEVTCVVVDVSHEDPEVK FNWYVDGVEVHNAKTKPREEQYNSTYRVVSVLTVLHQD WLNGKEYKCKVSNKALPAPIEKTISKAKGQPREPQVYTLP PSREEMTKNQVSLTCLVKGFYPSDIAVEWESNGQPENNY KTTPPVLDSDGSFFLYSKLTVDKSRWQQGNVFSCSVMHE ALHNHYTQKSLSLSPGK |
| 40. | h34C LC | DVVMTQSPLSLPVTLGQPASISCKSSQSLLHSDGETYLNW LQQRPGQSPRRLIYMMSKLDSGVPDRFSGSGSGTDFTLKI SRVEAEDVGVYYCWQGTHFPQTFGGGTKVEIKRTVAAP SVFIFPPSDEQLKSGTASVVCLLNNFYPREAKVQWKVDNA LQSGNSQESVTEQDSKDSTYSLSSTLTLSKADYEKHKVYA CEVTHQGLSSPVTKSFNRGEC |
| 41. | h70B VH CDR1 | **SYWMQ** |
| 42. | h70B VH CDR2 | **EIDPLDSYTNYNQKFKG** |
| 43. | h70B VH CDR3 | **WGDRVDY** |
| 44. | h70B VL CDR1 | **KSSQSLLSSNGKTYLN** |
| 45. | h70B VL CDR2 | **LVSTLDS** |
| 46. | h70B VL CDR3 | **LQGTHFPYA** |
| 47. | h70B VH | QVQLVQSGAEVKKPGASVKVSCKASGYTFTSYWMQWV RQAPGQGLEWIGEIDPLDSYTNYNQKFKGRATMTRDTSI STAYMELSRLRSDDTAVYSCAIWGDRVDYWGQGTLVTV SS |
| 48. | h70B VL | DVVMTQSPLSLPVTLGQPASISCKSSQSLLSSNGKTYLNW LQQRPGQSPRRLIYLVSTLDSGVPDRFSGSGSGTDFTLKIS RVEAEDVGVYYCLQGTHFPYAFGGGTKVEIK |
| 49. | h70B HC | QVQLVQSGAEVKKPGASVKVSCKASGYTFTSYWMQWV RQAPGQGLEWIGEIDPLDSYTNYNQKFKGRATMTRDTSI STAYMELSRLRSDDTAVYSCAIWGDRVDYWGQGTLVTV SSASTKGPSVFPLAPSSKSTSGGTAALGCLVKDYFPEPVTV SWNSGALTSGVHTFPAVLQSSGLYSLSSVVTVPSSSLGTQT YICNVNHKPSNTKVDKKVEPKSCDKTHTCPPCPAPELLGG PSVFLFPPKPKDTLMISRTPEVTCVVVDVSHEDPEVKFNW YVDGVEVHNAKTKPREEQYNSTYRVVSVLTVLHQDWLN GKEYKCKVSNKALPAPIEKTISKAKGQPREPQVYTLPPSRE EMTKNQVSLTCLVKGFYPSDIAVEWESNGQPENNYKTTPP VLDSDGSFFLYSKLTVDKSRWQQGNVFSCSVMHEALHNH YTQKSLSLSPGK |
| 50. | h70B LC | DVVMTQSPLSLPVTLGQPASISCKSSQSLLSSNGKTYLNW LQQRPGQSPRRLIYLVSTLDSGVPDRFSGSGSGTDFTLKIS RVEAEDVGVYYCLQGTHFPYAFGGGTKVEIKRTVAAPSV FIFPPSDEQLKSGTASVVCLLNNFYPREAKVQWKVDNALQ SGNSQESVTEQDSKDSTYSLSSTLTLSKADYEKHKVYACE VTHQGLSSPVTKSFNRGEC |
| 51. | 34C7D11 VH#1 | EVQLVESGGGLVKPGGSLRLSCAASGFTFSGYAMSWVRQ APGKGLEWVSTISDVGSDTYYPDNLEGRFTISRDNAKNS LYLQMNSLRAEDTAVYYCVRDDWDAYGMDFWGQGTL VTVSS |
| 52. | 34C7D11 VH#2 | EVQLVESGGGLVKPGGSLRLSCAASGFTFSGYAMSWVRQ APGKGLEWVATISDVGSDTYYPDNLEGRFTISRDNAKNS LYLQMNSLRAEDTAVYYCVRDDWDAYGMDFWGQGTL VTVSS |
| 53. | 34C7D11 VH#3 | EVQLVESGGGLVQPGGSLRLSCAASGFTFS**GYAMS**WVRQ APGKGLEWVA**TISDVGSDTYYPDNLEG**RFTISRDNAKNN LYLQMNSLRAEDTAVYYCVR**DDWDAYGMDF**WGQGTL VTVSS |
| 54. | 34C7D11 VL#1 | DVVMTQSPLSLPVTLGQPASISCKSSQSLLHSDGETYLNW LQQRPGQSPRRLIYMMSKLDSGVPDRFSGSGSGTDFTLKI SRVEAEDVGVYYCWQGTHFPQTFGGGTKVEIK |
| 55. | 34C7D11 VL#2 | DIVMTQTPLSLSVTPGQPASISCKSSQSLLHSDGETYLNW LLQKPGQSPQRLIYMMSKLDSGVPDRFSGSGSGTDFTLKI SRVEAEDVGVYYCWQGTHFPQTFGGGTKVEIK |
| 56. | 34C7D11 VL#3 | DVVMTQTPLSLSVTPGQPASISCKSSQSLLHSDGETYLNW LLQKPGQSPQRLIYMMSKLDSGVPDRFSGSGSGTDFTLKI SRVEAEDVGVYYCWQGTHFPQTFGGGTKVEIK |
| 57. | 34C7D11 VL#4 | DVVLTQTPLSLSVTPGQPASISCKSSQSLLHSDGETYLNW LLQKPGQSPKRLIYMMSKLDSGVPDRFSGSGSGTDFTLKI SRVEAEDVGIYYCWQGTHFPQTFGGGTKVEIK |
| 58. | 43E5C6 VH#1 | EVQLVESGGGLVQPGGSLRLSCAASGFTLSSCAMSWVRQ APGKGLEWVSTISEDGGSTYYLDNLQGRFTISRDNSKNT LYLQMNSLRAEDTAVYYCVRDDWDGYALDYWGQGTLV TVSS |
| 59. | 43E5C6 VH#2 | EVQLVESGGGLVQPGGSLRLSCAASGFTLSSCAMSWVRQ APGKGLEWVATISEDGGSTYYLDNLQGRFTLSRDNSKNT LYLQMNSLRAEDTAVYYCVRDDWDGYALDYWGQGTLV TVSS |
| 60. | 43E5C6 VH#3 | EVQLVESGGGLVQPGGSLRLSCAASGFTLSSCAMSWVRQ APGKGLEWVATISEDGGSTYYLDNLQGRFTLSRDDSKNT LYLQMNSLKADDTAVYYCVRDDWDGYALDYWGQGTL VTVSS |
| 61. | 43E5C6 VL#1 | DVVMTQSPLSLPVTLGQPASISCESSQSLLYSDGETYLNW LQQRPGQSPRRLIYLVSKLDSGVPDRFSGSGSGTDFTLKIS RVEAEDVGVYYCWQGSHFPQTFGGGTKVEIK |
| 62. | 43E5C6 VL#2 | DVVMTQSPLSLPVTLGQPASISCESSQSLLYSDGETYLNW LQQRPGQSPKRLIYLVSKLDSGVPDRFSGSGSGTDFTLKIS RVEAEDVGIYYCWQGSHFPQTFGGGTKVEIK |
| 63. | 43E5C6 VL#3 | DVVMTQSPLSLPVTLGQPASISCESSQSLLYSDGETYLNW LLQRPGQSPKRLIYLVSKLDSGVPDRFSGSGSGTDFTLKIS RVEAEDVGIYYCWQGSHFPQTFGGGTKVEIK |
| 64. | 70B8F3D9 VH#1 | QVQLVQSGAEVKKPGASVKVSCKASGYTFTSYWMQWV RQAPGQGLEWIGEIDPLDSYTNYNQKFKGRATMTRDTSI STAYMELSRLRSDDTAVYSCAIWGDRVDYWGQGTLVTV SS |
| 65. | 70B8F3D9 VH#2 | QVQLVQSGAEVKKPGASVKVSCKSSGYTFTSYWMQWVR QAPGQGLEWIGEIDPLDSYTNYNQKFKGKATLTVDTSIST AYMELSRLRSDDTAVYSCAIWGDRVDYWGQGTLVTVSS |
| 66. | 70B8F3D9 VH#3 | QVQLVQSGAEVKKPGASVKVSCKASGYTFTSYWMQWV RQAPGQGLEWIGEIDPLDSYTNYNQKFKGRATMTRDTST STVYMELSSLRSEDTAVYSCAIWGDRVDYWGQGTLVTVS S |
| 67. | 70B8F3D9 VH#4 | QVQLVQSGAEVKKPGASVKVSCKSSGYTFTSYWMQWVR QAPGQGLEWIGEIDPLDSYTNYNQKFKGKATLTVDTSTS TAYMELSSLRSEDTAVYSCAIWGDRVDYWGQGTLVTVSS |
| 68. | 70B8F3D9 VL#1 | DVVMTQSPLSLPVTLGQPASISC**KSSQSLLSSNGKTYLN**W LQQRPGQSPRRLIY**LVSTLDS**GVPDRFSGSGSGTDFTLKIS RVEAEDVGVYYC**LQGTHFPYA**FGGGTKVEIK |
| 69. | 70B8F3D9 VL#2 | DVVMTQSPLSLPVTLGQPASISCKSSQSLLSSNGKTYLNW LQQRPGQSPKRLIYLVSTLDSGVPDRFSGSGSGTDFTLKIS RVEAEDVGVYYCLQGTHFPYAFGGGTKVEIK |
| 70. | hLIV22 HC | QVQLVQSGAEVKKPGASVKVSCKASGLTIEDYYMH WVRQAPGQGLEWMGWIDPENGDTEYGPKFQGRV TMTRDTSINTAYMELSRLRSDDTAVYYCAVHNAHY GTWFAYWGQGTLVTVSSASTKGPSVFPLAPSSKSTS GGTAALGCLVKDYFPEPVTVSWNSGALTSGVHTFPA VLQSSGLYSLSSVVTVPSSSLGTQTYICNVNHKPSNT KVDKKVEPKSCDKTHTCPPCPAPELLGGPSVFLFPPK PKDTLMISRTPEVTCVVVDVSHEDPEVKFNWYVDGV EVHNAKTKPREEQYNSTYRVVSVLTVLHQDWLNGK EYKCKVSNKALPAPIEKTISKAKGQPREPQVYTLPPS REEMTKNQVSLTCLVKGFYPSDIAVEWESNGQPENN YKTTPPVLDSDGSFFLYSKLTVDKSRWQQGNVFSCS VMHEALHNHYTQKSLSLSPGK |
| 71. | hLIV22 LC | DVVMTQSPLSLPVTLGQPASISCRSSQSLLHSSGNTY LEWYQQRPGQSPRPLIYKISTRFSGVPDRFSGSGSGT DFTLKISRVEAEDVGVYYCFQGSHVPYTFGGGTKVE IKRTVAAPSVFIFPPSDEQLKSGTASVVCLLNNFYPRE AKVQWKVDNALQSGNSQESVTEQDSKDSTYSLSSTL TLSKADYEKHKVYACEVTHQGLSSPVTKSFNRGEC |
| 72. | hLIV1-Full length | MARKLSVILILTFALSVTNPLHELKAAAFPQTTEKISPNWE SGINVDLAISTRQYHLQQLFYRYGENNSLSVEGFRKLLQNI GIDKIKRIHIHHDHDHHSDHEHHSDHERHSDHEHHSEHEH HSDHDHHSHHNHAASGKNKRKALCPDHDSDSSGKDPRNS QGKGAHRPEHASGRRNVKDSVSASEVTSTVYNTVSEGTH FLETIETPRPGKLFPKDVSSSTPPSVTSKSRVSRLAGRKTNE SVSEPRKGFMYSRNTNENPQECFNASKLLTSHGMGIQVPL NATEFNYLCPAIINQIDARSCLIHTSEKKAEIPPKTYSLQIA WVGGFIAISIISFLSLLGVILVPLMNRVFFKFLLSFLVALAV GTLSGDAFLHLLPHSHASHHHSHSHEEPAMEMKRGPLFSH LSSQNIEESAYFDSTWKGLTALGGLYFMFLVEHVLTLIKQ FKDKKKKNQKKPENDDDVEIKKQLSKYESQLSTNEEKVD TDDRTEGYLRADSQEPSHFDSQQPAVLEEEEVMIAHAHPQ EVYNEYVPRGCKNKCHSHFHDTLGQSDDLIHHHHDYHHI LHHHHHQNHHPHSHSQRYSREELKDAGVATLAWMVI MGDGLHNFSDGLAIGAAFTEGLSSGLSTSVAVFCHELPHE LGDFAVLLKAGMTVKQAVLYNALSAMLAYLGMATGIFIG HYAENVSMWIFALTAGLFMYVALVDMVPEMLHNDASDH GCSRWGYFFLQNAGMLLGFGIMLLISIFEHKIVFRINF |
| 73. | hLIV1-ECD-His | FPQTTEKISPNWESGINVDLAISTRQYHLQQLFYRYGENNS LSVEGFRKLLQNIGIDKIKRIHIHHDHDHHSDHEHHSDHER HSDHEHHSEHEHHSDHDHHSHHNHAASGKNKRKALCPD HDSDSSGKDPRNSQGKGAHRPEHASGRRNVKDSVSASEV TSTVYNTVSEGTHFLETIETPRPGKLFPKDVSSSTPPSVTSK SRVSRLAGRKTNESVSEPRKGFMYSRNTNENPQECFNASK LLTSHGMGIQVPLNATEFNYLCPAIINQIDARSCLIHTSEKK AEIPPKTYSLQIAWHHHHHH |
| 74. | cyno-LIV1-Full length | MARKLSVILILTFTLSVTNPLHELKSAAAFPQTTEKISPNW ESGINVDLAITTRQYHLQQLFYRYGENNSLSVEGFRKLLQ NIGIDKIKRIHIHHDHDHHSDHEHHSDHEHHSDHEHHSHR NHAASGKNKRKALCPEHDSDSSGKDPRNSQGKGAHRPEH ANGRRNVKDSVSTSEVTSTVYNTVSEGTHFLETIETPKLFP KDVSSSTPPSVTEKSLVSRLAGRKTNESMSEPRKGFMYSR NTNENPQECFNASKLLTSHGMGIQVPLNATEFNYLCPAIIN QIDARSCLIHTSEKKAEIPPKTYSLQIAWVGGFIAISIISFLSL LGVILVPLMNRVFFKFLLSFLVALAVGTLSGDAFLHLLPHS HASHHHSHSHEEPAMEMKRGPLFSHLSSQNIEESAYFDST WKGLTALGGLYFMFLVEHVLTLIKQFKDKKKKNQKKPEN DDDVEIKKQLSKYESQLSTNEEKVDTDDRTEGYLRADSQE PSHFDSQQPAILEEEEVMIAHAHPQEVYNEYVPRGCKNKC HSHFHDTLGQSDDLIHHHHDYHHILHHHHHQNHHPHSHS QRYSREELKDAGIATLAWMVIMGDGLHNFSDGLAIGAAF TEGLSSGLSTSVAVFCHELPHELGDFAVLLKAGMTVKQA VLYNALSAMLAYLGMATGIFIGHYAENVSMWIFALTAGL FMYVALVDMVPEMLHNDASDHGCSRWGYFFLQNAGML LGFGIMLLISIFEHKIVFRINF |
| 75. | murine LIV1-Full length | MATDLSVIMILTFALWVTSPLHELQSTAAFSQTTEKINSN WEPGVNVDLAVTMQRHHLQQLFYRYGENDSLSVEGFRK LLQNIGIDKIKRVHIHHDHEHHADHEHHSDHEHHSDHEHH SDHEHHSDHEHHSDHEHHSHRSHTVAGKNNRKAFCPDLD SDNSGKNPRTSLGKGSRPAEHMNGRRNIKESASSSEVTSA VYNAVSEGTRFVETIETPKPGRRTKDVNPSTPPSITEKSRV GRLSRLARKKSNESVSEPRKSFMYSRNTNDNIQECFNTTK LLTSHGMSIQALLNATEFNYLCPAIINQIDARACLIHTASEK KAEIPPKTYSLQIAWLGGFIAISIISFLSLLGVILVPLMNRVF FKFLLSFLVALAVGTLSGDALLHLLPHSHASHQHSHSHEE PAMEMKRGPLFSHLSAQNIEESSYFDSTWKGLTALGGLYF MFLVEHVLTLIKQFKDKKKKNQKKPENDEDVESKKQLSK YDSQLSSNEEKVDPGERPESYLRADSQEPSPFDSQQPTMLE EEEVMIAHAHPQEVYNEYVPRGCKNKCHSHFHDTLGQSD DLIHHHHDYHHILHHHHHQNHHPHSHSQRYSREELKDAGI ATLAWMVIMGDGLHNFSDGLAIGAAFTEGLSSGLSTSVA VFCHELPHELGDFAVLLKAGMTVKQAVLYNALSAMLAY LGMATGIFIGHYAENVSMWIFALTAGLFMYVALVDMVPE MLHNDASDHGCSRWGYFFLQNAGILLGFGIMLLISIFEHKI VFRINF |
| 76. | rat-LIV1-Full length | MATNLSVIMILTFALWVTNPLHELQSTAAFSQTTEKINSN WESGINVDVAVTMQRHHLQQLFYRYGENDSLSVEGFRKL LQNIGIDKIKRVHIHHDHERHSDHERHSDHERHSHRGHAA AGKNSRKAFCPDLDSDNSGKNPNTSQGKGSRPAEHVNGR RNGKESASSSEVTSAVYNTVSEGTHFLETIETPKPGRRTKD INPSTPPSITEKSRVGRLSRLARRKGNDSVSEPRKSFMYSR TSNDNIQECFNATKLLTSHGMSVQALLNATEFNYLCPAIIN QIDARSCLIHTASEKKAEIPPKTYSLQIAWLGGFIAISIISFLS LLGVILVPLMNRVFFKFLLSFLVALAVGTLSGDALLHLLP HSHASHHHSHSHEEPAMEMKRGPLFSHLSAQNLEESSYFD STWKGLTALGGLYFMFLVEHVLTLIKQFKDKKKKNQKKP ENDEDVESKKQLSKYESQLSTNEEKVDTGERPESYLQADS QEPSPFDSQQPTLLEEEEVMIAHAHPQEVYNEYVPRGCKN KCHSHFHDTLGQSDDLIHHHHDYHHILHHHHHQNHHPHS HSQRYSREELKDAGIATLAWMVIMGDGLHNFSDGLAIGA AFTEGLSSGLSTSVAVFCHELPHELGDFAVLLKAGMTVKQ AVLYNALSAMLAYLGMATGIFIGHYAENVSMWIFALTAG LFMYVALVDMVPEMLHNDASDHGCSRWGYFFLQNAGIL LGFGIMLLISIFEHKIVFRINF |

The following examples are merely illustrative of the presently disclosed subject matter and should not be considered as limitations in any way.

### EXAMPLES

### Example 1. Screening of anti-LIV-1 antibodies

Anti-LIV-1 antibodies were screened as follows. A human LIV-1 full length (SEQ ID No:72) coding sequence and a recombinant protein hLIV-1-ECD-his (SEQ ID No:73, ACRO) were subsequently employed to immunize mice and hybridoma cells were obtained by fusion of spleen cells from the immunized mice with myeloma cells. Positive clones were further screened by ELISA and FACS.

Briefly, a 96-well plate was first coated with the recombinant protein human LIV-1-ECD-His (working volume 30 µL) at 2 µg/ml and incubated overnight at 4°C. Then it was washed with PBS containing 0.05% Tween 20 (PBST) for 3 times to remove the excess protein. Thereafter, the plate was blocked with SuperBlock T20 (ThermoFisher) at room temperature for 1 hour, washed with PBST for 3 times, and then added the diluted culture supernatant of the hybridoma cells. It was left at room temperature for 1 hour, and then washed with PBST for 3 times. 30 µl horseradish peroxidase labeled goat anti-mouse IgG (Cwbio, https://www.cwbio.com/) diluted at 1:2000 with PBS was added, left at room temperature for 1 hour, and washed with PBST for 6 times. TMB was added for color development, and the reaction was stopped with 30 µL ELISA stop solution (www.sangon.com). The absorbance at 450 nm was read in a microplate reader.

The positive hybridoma clones screened by ELISA were further screened by flow cytometry. First, the CHO-S cells expressing the human LIV-1 full-length were washed twice with PBS (2% FBS), and the cell density was adjusted to 5×10⁵ cells/100 µL. The supernatant of the hybridoma cell was added and incubated at 4°C for 30 min. The cells were harvested by centrifugation at 500×g for 5 min and washed twice with PBS (2% FBS). Goat anti-mouse IgG-FITC diluted at 1:100 (Cwbio, https://www.cwbio.com/) was added, incubated at 4°C for 30 min, and the cells were washed twice with PBS (2% FBS). Finally, the cells were suspended in 200 µL PBS and analyzed by flow cytometry.

The three clones exhibiting positive results in both ELISA and FACS (34C7D11, 43E5C6 and 70B8F3D9) were selected for screening light and heavy chain sequences.

### Example 2. Expression of anti-LIV-1 chimeric antibodies

The fragments encoding the variable regions of light and heavy chains were amplified according to the method described in Anke Krebber et al. (Journal of Immunological Methods 201. 1997. 35-55). Briefly, the total RNAs were extracted from the monoclonal hybridoma cell lines 34C7D11, 43E5C6 and 70B8F3D9, and the single-strand cDNA was prepared using the reverse transcription kit (Takara). Using the primes complement with mouse light chain and heavy chain variable regions as described in Anke Krebber *et al.* (supra), the fragments encoding the heavy chain variable regions (SEQ ID Nos: 7, 17, and 27 respectively) and light chain variable regions (SEQ ID Nos: 8, 18, and 28 respectively) were amplified by PCR. Then, the heavy chain variable regions and light chain variable regions of 34C7D11, 43E5C6, and 70B8F3D9 were fused with the constant region of IgG1 heavy chain and the constant region of IgG1 light chain respectively by recombination engineering. Thus, the expression plasmids of the chimeric antibodies 34C7D11, 43E5C6, and 70B8F3D9 were obtained.

The expression plasmids were transfected into ExpiCHO cells for transient expression of the chimeric antibodies of 34C7D11, 43E5C6, and 70B8F3D9. Subsequently, the resulting chimeric antibodies were analyzed by SDS-PAGE and SEC, showing good expression and high purity.

### Example 3. Binding of the anti-LIV-1 chimeric antibodies to LIV-1

The binding ability of the anti-LIV-1 chimeric antibodies to human LIV-1 together with cynomolgus monkey LIV-1, rat LIV-1 and mouse LIV-1 was determined by ELISA.

First, a plate was coated with hLIV-1-ECD-his, cynoLIV-1-ECD-his, murine LIV-1-ECD-his or rat LIV-1-ECD-his (ACRO) at 2 µg/ml (working volume 30 µL) and incubated overnight at 4°C. Then, it was washed with PBS containing 0.05% Tween 20 (PBST) for 3 times to remove the excess protein. Thereafter, the plate was blocked with 60 µL SuperBlock T20 (ThermoFisher) at room temperature for 1 hour, washed with PBST for 3 times, and then added the anti-LIV-1 chimeric antibodies in 5-fold serial dilutions starting from 100 nM. An anti-LIV-1 reference antibody (Reference Ab, heavy chain: SEQ ID No: 70 and light chain: SEQ ID No: 71) synthesized in-house based on the sequence information disclosed in US9228026B2 as hLIV22 was used as a positive control. IgG1 isotype control (Biointron, Cat. No. B117901) was used as a negative control. The plate was left at room temperature for 1 hour, and then washed with PBST for 3 times. 30 µl horseradish peroxidase labeled goat anti-human secondary antibody (Jackson) diluted at 1:5000 with PBS was added, left at room temperature for 1 hour, and washed with PBST for 3 times. TMB was added for color development, and the reaction was stopped with 30 µL ELISA stop solution (www.sangon.com). The absorbance at 450 nm was read in a microplate reader.

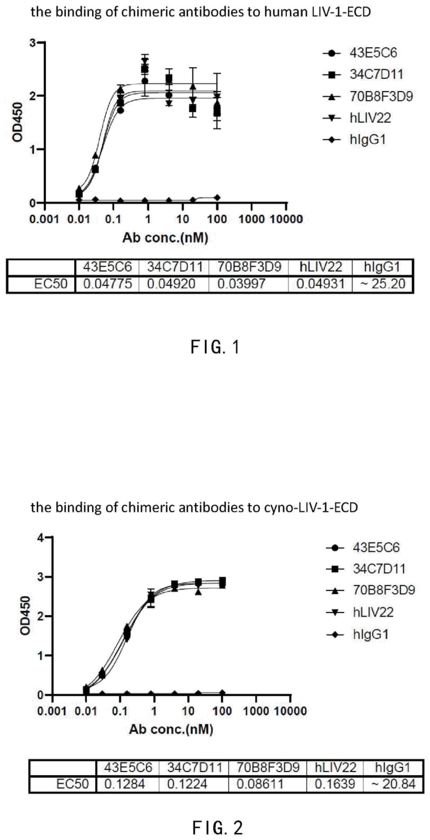

As shown in Fig. 1, the chimeric antibodies 34C7D11 and 43E5C6 exhibit a binding affinity to hLIV-1 comparable to that of Reference Ab and 70B8F3D9 shows a binding affinity to hLIV-1 significantly superior to that of Reference Ab. Similarly, 70B8F3D9 binds to cyno-LIV1-ECD with an affinity nearly 2 times of that of Reference Ab (Fig. 2), while both 34C7D11 and 43E5C6 show a binding affinity to cyno-LIV1-ECD better than that of Reference Ab (Fig. 2).

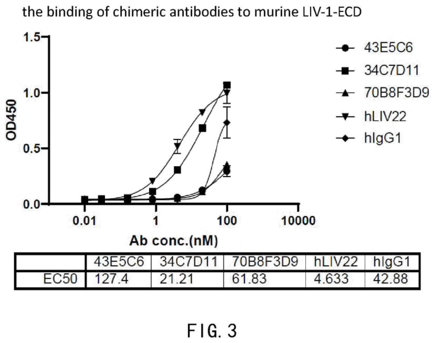

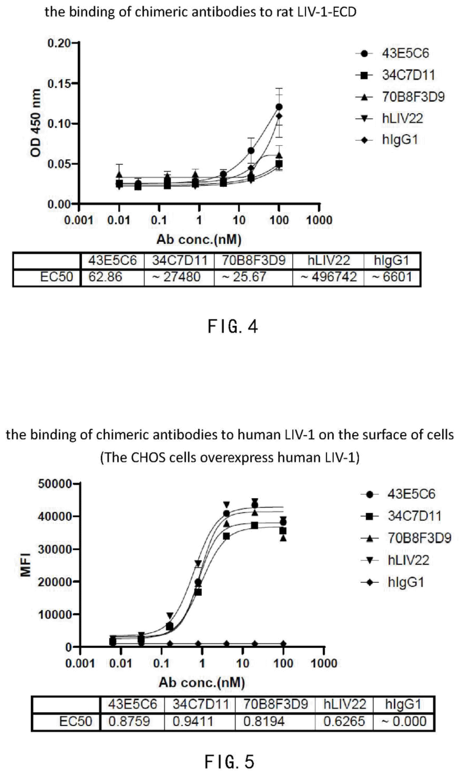

In contrast, all three chimeric antibodies exhibit very weak binding to murine LIV-1 (Fig.3) and even no binding to rat LIV-1 (Fig.4).

The whole cell binding ability of the anti-LIV-1 chimeric antibodies to hLIV-1 and cyno-LIV-1 was further determined by flow cytometry. First, the CHO-S cells expressing human LIV-1, the MCF-7 breast cancer cells expressing LIV-1, and the CHO-S cells expressing cyno-LIV-1 were washed twice with PBS (2% FBS), and the cell density was adjusted to 5×10⁵ cells/100 µL. The three anti-LIV-1 chimeric antibodies in 5-fold serial dilutions starting from 100 nM were added and incubated at 4°C for 60 min. Reference Ab was used as a positive control and IgG1 isotype control (Biointron, Cat. No. B117901) was used as a negative control. The cells were harvested by centrifugation at 500×g for 5 min and washed twice with PBS (2% FBS). Goat anti-human IgG-PE (Invitrogen) diluted at 1:100 was added, incubated at 4°C for 30 min, and the cells were washed twice with PBS (2% FBS). Finally, the cells were suspended in 200 µL PBS and analyzed by flow cytometry.

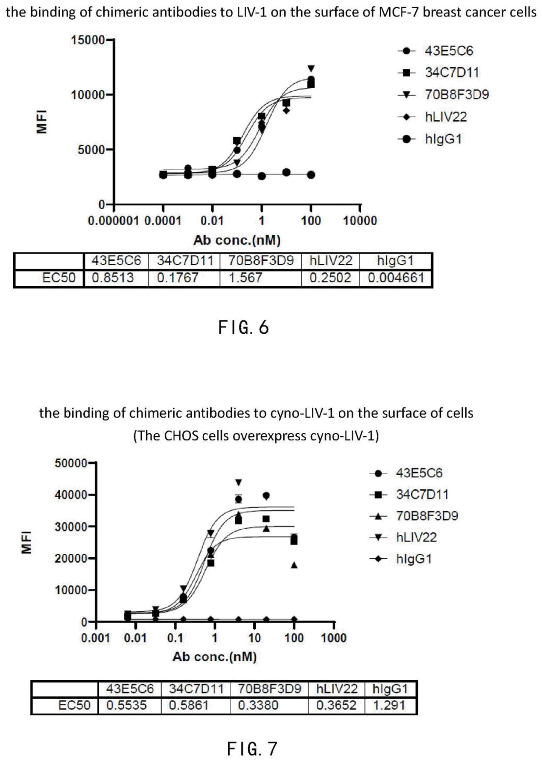

As shown in Figs.5-7, the whole cell binding abilities of chimeric antibodies 34C7D11, 43E5C6, and 70B8F3D9 were comparable to that of Reference Ab.

### Example 4. Kinetic curve determination of the anti-LIV-1 chimeric antibodies

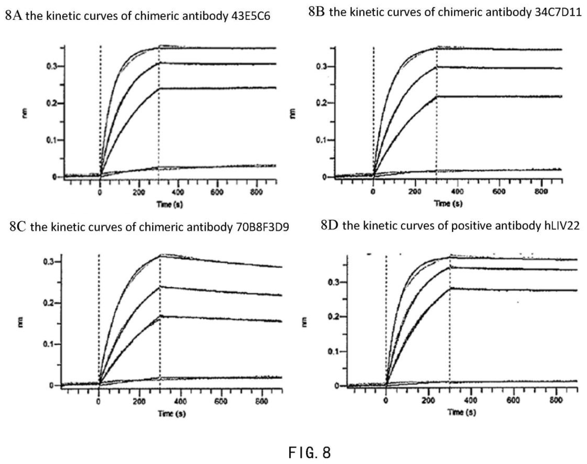

The affinity and kinetic constants of the anti-LIV-1 chimeric antibodies with respect to LIV-1 were measured by Octet. First, hLIV-1-ECD-his protein (ACRO) was fixed on HIS 1K biosensors. Then, the three anti-LIV-1 chimeric antibodies (34C7D11, 43E5C6, and 70B8F3D9) were diluted with HBSPE buffer in 5-fold serial dilutions starting from 100 nM for a total of 4 dilutions and flowed through HIS 1K biosensors to obtain the kinetic curve data. Reference Ab was used as a control. The resulting data were analyzed by software, and the kinetic curves of association and dissociation (FIG. 8) and kinetic constants (Table 1) were obtained by modeling and fitting.

As shown in table 1 and Fig. 8, the binding affinities of the chimeric antibodies 34C7D11 and 43E5C6 are much better than that of Reference Ab. And compared with Reference Ab hLIV22, the chimeric antibody 70B8F3D9 has a comparable association rate but faster disassociation rate.

**Table 1. The kinetic constants of the anti-LIV-1 chimeric antibodies**

| **mAb** | **Ag** | **KD (M)** | **ka (1/Ms)** | **kdis (1/s)** |
|---|---|---|---|---|
| 43E5C6 | hLIV-1-ECD-his | 1.94E-11 | 2.33E+05 | 5.23E-06 |
| 34C7D11 | hLIV-1-ECD-his | 6.51E-11 | 2.69E+05 | 1.51E-05 |
| 70B8F3D9 | hLIV-1-ECD-his | 9.90E-10 | 1.51E+05 | 1.50E-04 |
| Reference Ab hLIV22 | hLIV-1-ECD-his | 1.34E-10 | 2.67E+05 | 3.57E-05 |

### Example 5. Epitope binning of the anti-LIV-1 chimeric antibodies

Epitope binning was performed via Octet to analyze the epitopes on human LIV-1 bound by the three chimeric antibodies. Reference Ab (hLIV22) was used as a positive control and IgG1 isotype control (Biointron, Cat. No. B117901) was used as a negative control. First, the hLIV-1-ECD-his (ACRO) was fixed on HIS 1K biosensors, and the antibodies at saturated concentration (20 nM) were bound with HIS 1K biosensors, and then the biosensors are bound with the antibodies, respectively. The corresponding curves were obtained. The resulting data were analyzed via a software, and epitope-related data were obtained.

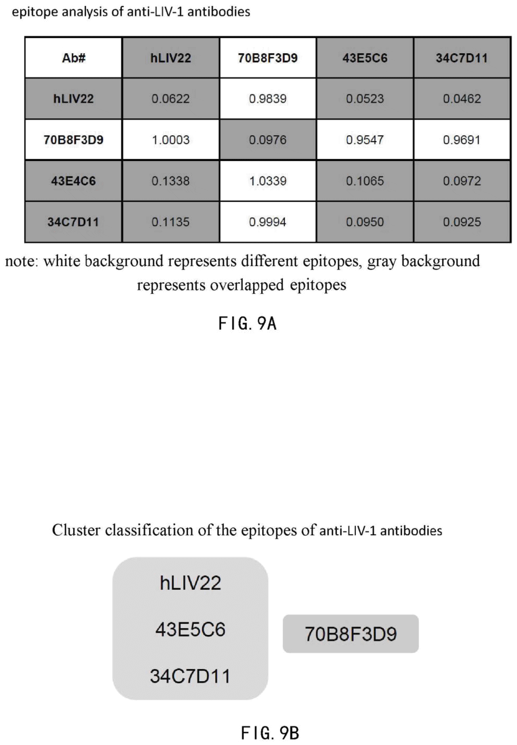

As shown in Fig. 9, the chimeric antibodies 34C7D11 and 43E5C6 bind to an epitope overlapping with that of the anti-LIV-1 Reference Ab (hLIV22), while the chimeric antibody 70B8F3D9 bind to an epitope different from that of the anti-LIV-1 Reference Ab.

### Example 6. ADCC effects of the anti-LIV-1 chimeric antibodies

The antibody-dependent cell-mediated cytotoxicity (ADCC) of the anti-LIV-1 chimeric antibodies was evaluated using a reporter cell line NFAT-CD16a Jurkat. First, the CHOS-hLIV-1 target cells were adjusted to a cell density of 2×10⁵ cells/mL, and 50 µL cells per well were added to a 96-well plate (Corning) for overnight incubation. On the second day, the anti-LIV-1 chimeric antibodies were diluted in 4-fold serial dilutions for a total of 10 dilutions, with a maximum concentration of 30 µg/mL. 25 µL anti-LIV-1 chimeric antibodies per well were added to the 96-well plate containing target cells. Subsequently, the effector cells with cell density adjusted were added at 25 µL/well to the 96-well plate containing the antibodies and target cells in a ratio of effector cells to target cells of 10:1 or 5:1, respectively. The 96-well plate was incubated in an incubator containing 5% CO₂ at 37 °C for 4-6h, and the chromogenic substrate CellTiter-Glo (promega) was added for detection.

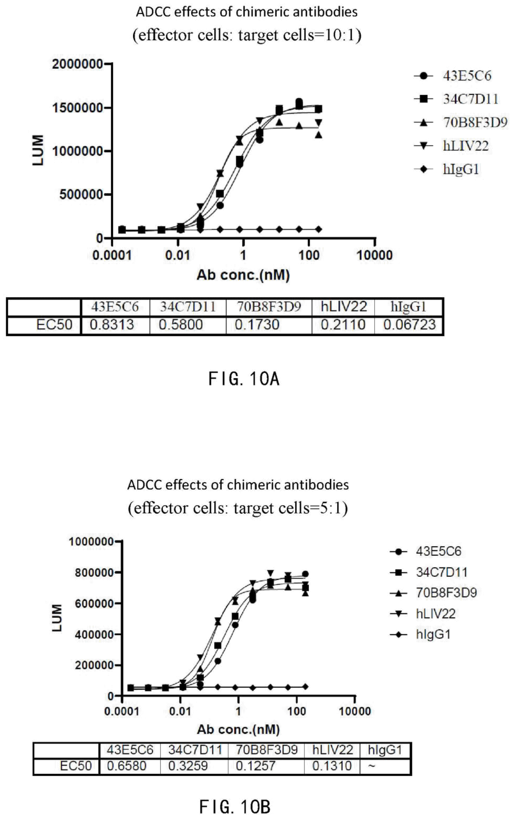

As shown in Fig. 10, the ADCC effects of chimeric antibody 70B8F3D9 was significantly better than that of Reference Ab in an effector-to-target ratio of 10:1 and comparable to that of Reference Ab in an effector-to-target ratio of 5:1.

### Example 7. Internalization of the anti-LIV-1 chimeric antibodies

A pHrodo pH-sensitive probe dye (Invitrogen) was conjugated to the anti-LIV-1 chimeric antibodies by amine reactivity to assess the internalization of the anti-LIV-1 chimeric antibodies into CHOK1-hLIV1 cells. First, the pHrodo dye was reacted with the antibodies in dark at room temperature for 5min, and then added into a 96-well plate containing 1×10⁴ cells/well. The plate was incubated in an incubator containing 5% CO₂ at 37°C for 16h, followed by flow cytometry analysis.

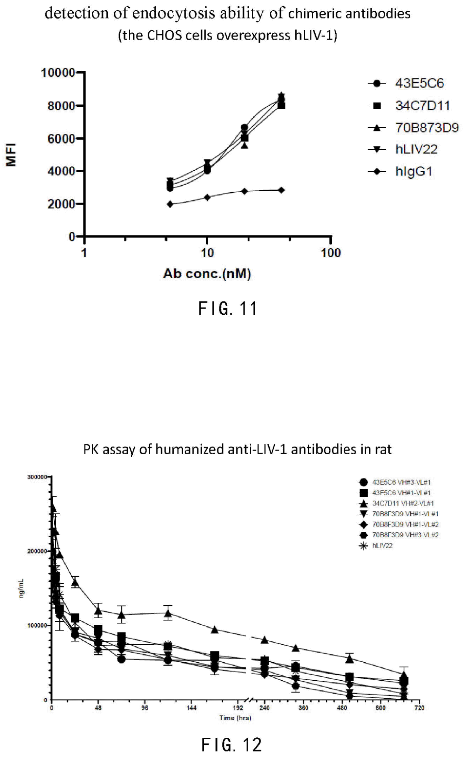

As shown in Fig. 11, the internalization of all the anti-LIV-1 chimeric antibodies is comparable to that of the reference antibody.

### Example 8. Humanization of the anti-LIV-1 antibodies

Three clones 34C7D11, 43E5C6, and 70B8F3D9 were selected for humanization of their framework. Briefly, MOE software was performed using the sequences of the three clones to search database of human germline genes. Ideal germline sequences were selected, and mutations of framework sequences were made to change the framework sequences from mouse to human. For 34C7D11 clone, human germlines IGHV3-21*01 (for heavy chain), IGHV3-07*01 (for heavy chain), IGKV2-30*02 (for light chain) and IGKV2-29*02 (for light chain) were used, the key amino acids thereof were back mutated, and three heavy chains (SEQ ID Nos: 51-53) and four light chains (SEQ ID Nos: 54-57) of humanized 34C7D11 were made. For 43E5C6 clone, human germlines IGHV3-23*04 and IGKV2-30*01 were used for the heavy and light chains respectively, the key amino acids thereof were back mutated, and three heavy chains (SEQ ID Nos: 58-60) and three light chains (SEQ ID Nos: 61-63) of humanized 43E5C6 were made. And for 70B8F3D9 clone, human germlines IGHV1-02*02 (for heavy chain), IGHV1-46*03 (for heavy chain), IGKV2-30*02 (for light chain) and IGKV2-30*01 (for light chain) were used, the key amino acids thereof were back mutated, and four heavy chains (SEQ ID Nos: 64-67) and two light chains (SEQ ID Nos: 68-69) of humanized 70B8F3D9 were made.

Each humanized heavy chain was combined with the corresponding humanized light chain into a humanized anti-LIV-1 antibody. The resulting humanized antibodies were expressed, purified, and evaluated using the binding assay as in Example 3, the kinetic curve assay as in Example 4, the ADCC assay as in Example 6 and the internalization assay as in Example 7. Finally, six humanized anti-LIV-1 antibodies (34C7D11 VH#2-VL#1; 43E5C6 VH#3-VL#1; 43E5C6 VH#1-VL#2; 70B8F3D9 VH#1-VL#1; 70B8F3D9 VH#1-VL#2; and 70B8F3D9 VH#3-VL#2) were selected and the assay results thereof are shown in table 2.

As shown in table 2, all the humanized anti-hLIV-1 antibodies exhibit similar affinity and function to the chimeric antibodies and are significantly superior to that of Reference Ab.

**Table 2. Characterization of the humanized anti-hLIV-1 antibodies**

| **Ab** | **Whole cell binding** | | | | | | **Protein binding** | | **Kinetic** | | | **ADCC** | **Internalization** |
|---|---|---|---|---|---|---|---|---|---|---|---|---|---|
| | **Human LIV1** | | **Cyno LIV1** | | **MCF-7** | | **hLIV1-ECD** | **cynoLIV1-ECD** | **hLIVI-ECD** | | | | |
| | EC50 | Max MFI | EC50 | Max MFI | EC50 | Max MFI | EC50 | EC50 | KD (M) | ka (1/Ms) | kdis (1/s) | | |
| 43E5C6 | 1.259 | 48186.7 | 0.5535 | 35077 | 0.9139 | 10484 | 0.12 | 0.1331 | 5.72E-10 | 2.19E+05 | 1.25E-04 | +++ | +++ |
| 43E5C6 VH#3-VL#1 | 0.661 | 42514 | 0.3476 | 32183 | 0.5168 | 9788 | 0.08876 | 0.08694 | 1.56E-09 | 7.54E+04 | 1.18E-04 | +++ | +++ |
| 43E5C6 VH#1-VL#2 | 1.192 | 47221 | 0.8434 | 32294 | 0.3356 | 7912 | 0.1163 | 0.09798 | 1.57E-09 | 6.22E+04 | 9.74E-05 | +++ | +++ |
| 34C7D11 | 0.6739 | 40972 | 0.5861 | 30043 | 0.4552 | 8095 | 0.1208 | 0.1299 | 6.41E-10 | 1.65E+05 | 1.05E-04 | +++ | +++ |
| 34C7D11 VH#2-VL#1 | 0.9069 | 40334 | 0.7427 | 26182 | 0.4271 | 7823 | 0.1004 | 0.1021 | 9.00E-10 | 8.52E+04 | 7.66E-05 | +++ | +++ |
| 70B8F3D9 | 0.4239 | 31502 | 0.338 | 26830 | 65.74 | 22215 | 0.1011 | 0.09537 | 1.30E-09 | 1.31E+05 | 1.70E-04 | +++ | +++ |
| 70B8F3D9 VH#1-VL#1 | 0.4615 | 30021 | 0.3347 | 25611 | 10.75 | 15300 | 0.0948 | 0.0968 | 2.58E-09 | 1.08E+05 | 2.79E-04 | +++ | +++ |
| 70B8F3D9 VH#1-VL#2 | 0.5247 | 30609 | 0.3298 | 25156 | 1.296 | 8130 | 0.09329 | 0.09167 | 2.10E-09 | 1.18E+05 | 2.47E-04 | +++ | +++ |
| 70B8F3D9 VH#3-VL#2 | 0.6405 | 31743 | 0.345 | 23770 | 1.201 | 7514 | 0.1074 | 0.1012 | 2.10E-09 | 1.18E+05 | 2.47E-04 | +++ | +++ |
| hLIV22 | 0.4710 | 41232.6 | 0.3652 | 36157 | 0.3062 | 7728 | 0.1304 | 0.1513 | 1.26E-09 | 1.65E+05 | 2.07E-04 | +++ | +++ |
| hIgG1 | / | / | / | / | / | 2238 | 183.4 | ~ 289.4 | / | / | / | / | / |

| | | | | | | | | | | | | | |
|---|---|---|---|---|---|---|---|---|---|---|---|---|---|
| Note: + represents a weak internalization or ADCC; ++ represents a moderate internalization or ADCC; +++ represents a strong internalization or ADCC (similar to the positive control Reference Ab); / represents no accurate result. | | | | | | | | | | | | | |

### Example 9. A pharmacokinetics (PK) assay of the humanized anti-LIV-1 antibodies in rat

The six humanized anti-LIV-1 antibodies of example 8 were prepared in PBS, and intravenously injected into female SD rats (3 SD rats in each group) at a dose of 5mg/kg. At 1, 4, 8 hours and 1, 2, 3, 5, 7, 10, 14, 21, 28 days post-dose, blood samples were collected from the rats to determine the level of the antibodies in the blood via ELISA.

As shown in Table 3 and Fig. 12, after the administration of the humanized anti-LIV-1 antibodies to SD rats, they exhibit good PK characteristics, even much better than Reference Ab except 70B8F3D9 VH#3-VL#2.

**Table 3. Pharmacokinetic parameters of the humanized anti-LIV-1 antibody molecules in SD rat**

| **Ab#** | **T1/2(h)** | **Cmax(ng/mL)** | **AUC0-t (mg·h/mL)** | **MRT(h)** |
|---|---|---|---|---|
| 43E5C6 VH#3-VL#1 | 330.24 | 199432.87 | 40196342.00 | 266.36 |
| 43E5C6 VH#1-VL#2 | 391.72 | 226083.50 | 34493057.33 | 547.76 |
| 34C7D11 VH#2-VL#1 | 326.50 | 259634.23 | 52907381.67 | 479.92 |
| 70B8F3D9 VH#1-VL#1 | 155.5 | 175702.36 | 22443670.67 | 259.5 |
| 70B8F3D9 VH#1-VL#2 | 378.91 | 173790.09 | 24146733.67 | 492.98 |
| 70B8F3D9 VH#3-VL#2 | 71.27 | 157064.04 | 20006341.67 | 155.27 |
| hLIV22 | 243.18 | 223509.01 | 32375128.33 | 436.28 |

### Example 10. Preparation of the anti-LIV-1 antibody conjugates

Two representative humanized anti-LIV-1 antibodies 34C7D11 VH#2-VL#1 (aka. h34C) and 70B8F3D9 VH#1-VL#1 (aka. h70B) were used for bioconjugation. Reference Ab was used as a positive control and IgG1 isotype control was used as a negative control.

Briefly, 2 mg anti-LIV-1 antibody in 25 mM PB buffer with a pH of 6.5-8.0 was first reduced by adding 5 to 10 equivalents of TCEP-HCl solution (10 mM, 25 mM PB buffer, Sigma Aldrich, Cat. No.: 75259-1G) and then incubated at 20-37°C for 0.5-16 hours. Thereafter, an organic solvent (e.g. DMSO, DMF or DMAc) was added in a volume ratio of 5-15% v/v and then added 6-20 equivalents of a linker-payload reagent (e.g. MC-VC-PABC-MMAE (see below for its structure), MedChem Express (Shanghai), Cat. No.: HY-15575, CAS RN: 646502-53-6) dissolved in the organic solvent (e.g. DMSO, DMF or DMAc). Subsequently, the reaction mixture was incubated at 20-37 °C for 0.5-4 hours for the conjugation of the antibody to the link-payload reagent. The resulting conjugate was then dialyzed overnight in 40 mM PB, pH 7.0 to remove the uncoupled reagents and the excess organic solvent. Finally, the purified conjugate was characterized in terms of physicochemical properties and cytotoxicity profile. SEC analysis demonstrates a very high monomer percentage (near to 100%) of the resulting conjugates. The concentration and drug-to-antibody ratio of the conjugates were determined using A280nm, A366nm and the ratio thereof. The DAR values of the conjugates comprising MC-VC-PABC-MMAE were determined to be about 4.

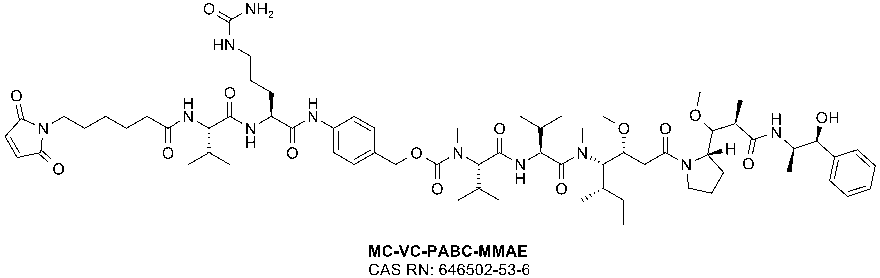

### Example 11. In vitro cell killing of the humanized anti-LIV-1 antibody conjugates

The cell killing of the anti-LIV-1 antibody conjugates were evaluated in BT474 cell line without LIV-1 expression, MCF7-hLIV-1 cell line moderately expressing LIV-1 and HEK293-hLIV-1 highly expressing LIV-1. First, 1×10⁵ cells/well were spread in a 96-well transparent flat plate at 50 µL/well and the 3-fold serial dilutions (the maximum antibody concentration was 100 µg/mL) of conjugates were added at 50 µL/well. The 96-well plate was incubated in an incubator containing 5% CO₂ at 37 °C. 3-4 days later, cell viability was detected using CCK-8 (www.sangon.com) or cell-Titer (Promega).

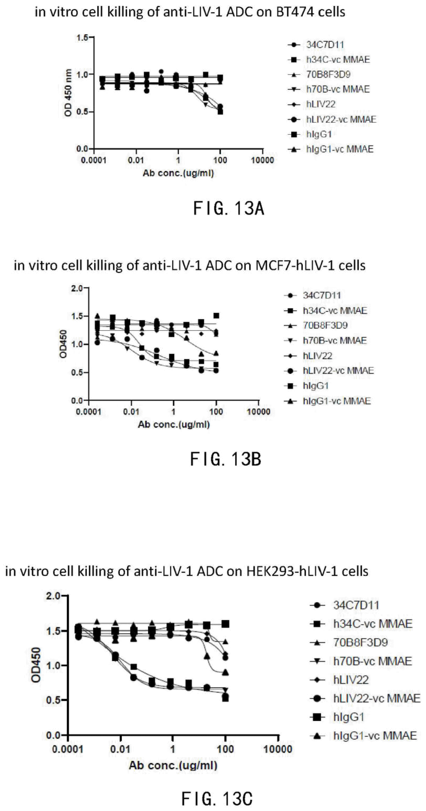

As shown in Fig. 13A, there is no nonspecific killing in BT474 cell line, which is a cell line without LIV-1 expression, except at high doses. Meantime, as shown in Figs.13B-C, for MCF7-hLIV-1 and HEK293-hLIV-1 both with LIV-1 expression, the cell killing abilities of the anti-hLIV-1 conjugates are significantly superior to that of the corresponding antibodies and comparable to the reference conjugate hLIV22-vc MMAE.

### Example 12. In vivo anti-tumor efficacy of the humanized anti-LIV-1 antibody conjugates in mice

6-8 weeks aged BALB/c female mice were subcutaneously inoculated with 1×10⁷ MCF-7 cells overexpressing human LIV-1 (MCF7-hLIV-1). When the tumor size reached an average of about 150 mm³, the mice were randomly grouped (8 mice each group). The mice were administered intravenously with the humanized anti-LIV-1 antibody conjugates once a week for about 2 to 3 weeks. Tumor growth was monitored by caliper measurement until day 17 after the start of treatment.

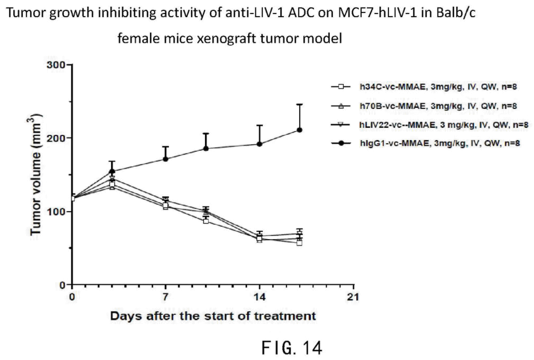

As shown in Fig. 14 and Table 4, the anti-tumor efficacies of the humanized anti-hLIV-1 antibody conjugates are significantly better than that of the reference conjugate hLIV22-vc MMAE.

**Table 4. The tumor growth inhibition by the humanized anti-LIV-1 antibody conjugates in MCF7-hLIV-1 inoculated mice.**

| Group | n^{a} | Treatment | Dose (mg/kg) | Dosing Route | Dosing SchedµLe | TGI(%) |
|---|---|---|---|---|---|---|
| 1 | 8 | h34C-vc-MMAE | 3 | IV | QW x 3W | 72.99 |
| 2 | 8 | h70B-vc-MMAE | 3 | IV | QW x 3W | 70.25 |
| 3 | 8 | hLIV22-vc-MMAE | 3 | IV | QW x 3W | 67.08 |
| 4 | 8 | hIgG1-vc-MMAE | 3 | IV | QW x 3W | / |

In addition to the various embodiments depicted and claimed, the disclosed subject matter is also directed to other embodiments having other combinations of the features disclosed and claimed herein. As such, the particular features presented herein can be combined with each other in other manners within the scope of the disclosed subject matter such that the disclosed subject matter includes any suitable combination of the features disclosed herein. The foregoing description of specific embodiments of the disclosed subject matter has been presented for purposes of illustration and description. It is not intended to be exhaustive or to limit the disclosed subject matter to those embodiments disclosed.

It will be apparent to those skilled in the art that various modifications and variations can be made in the compositions and methods of the disclosed subject matter without departing from the spirit or scope of the disclosed subject matter. Thus, it is intended that the disclosed subject matter include modifications and variations that are within the scope of the appended claims and their equivalents.

Various publications, patents and patent applications are cited herein, the contents of which are hereby incorporated by reference in their entireties.

## Claims

1. An antibody that binds to LIV-1 comprising:
a) a heavy chain variable region comprising:
(1) a heavy chain variable region CDR-H1 comprising an amino acid sequence of any one of SEQ ID NOs: 1, 11, 21, 31, and 41, or a variant thereof comprising up to about 3 amino acid substitutions;
(2) a heavy chain variable region CDR-H2 comprising an amino acid sequence of any one of SEQ ID NOs: 2, 12, 22, 32, and 42, or a variant thereof comprising up to about 3 amino acid substitutions; and
(3) a heavy chain variable region CDR-H3 comprising an amino acid sequence of any one of SEQ ID NOs: 3, 13, 23, 33, and 43, or a variant thereof comprising up to about 3 amino acid substitutions; and
b) a light chain variable region comprising:
(1) a light chain variable region CDR-L1 comprising an amino acid sequence of any one of SEQ ID NOs: 4, 14, 24, 34, and 44, or a variant thereof comprising up to about 3 amino acid substitutions;
(2) a light chain variable region CDR-L2 comprising an amino acid sequence of any one of SEQ ID NOs: 5, 15, 25, 35, and 45, or a variant thereof comprising up to about 3 amino acid substitutions; and
(3) a light chain variable region CDR-L3 comprising an amino acid sequence of any one of SEQ ID NOs: 6, 16, 26, 36, and 46, or a variant thereof comprising up to about 3 amino acid substitutions.

2. The antibody of claim 1, wherein the antibody binds to LIV-1 with a KD of about 1x10⁻⁹ M or less.

3. The antibody of claim 1 or 2, wherein the antibody binds to LIV-1 with a KD of about 5x10⁻¹⁰ M or less.

4. The antibody of any one of claims 1-3, wherein antibody binds to LIV-1 with a KD of between about 1x10⁻¹¹ M and about 1x10⁻¹⁰ M.

5. The antibody of any one of claims 1-4, wherein the antibody binds to LIV-1 with a KD of between about 1x10⁻¹¹ M and about 5x10⁻¹¹ M.

6. The antibody of any one of claims 1-5, wherein the antibody cross-competes with a reference anti-LIV-1 antibody comprising:
a) a heavy chain variable domain (VH) sequence comprising (1) a CDR-H1 comprising the amino acid sequence set forth in SEQ ID NO: 1, (2) a CDR- H2 comprising the amino acid sequence set forth in SEQ ID NO: 2, and (3) a CDR-H3 comprising the amino acid sequence set forth in SEQ ID NO: 3; and a light chain variable domain (VL) sequence comprising (1) a CDR-L1 comprising the amino acid sequence set forth in SEQ ID NO: 4, (2) a CDR-L2 comprising the amino acid sequence set forth in SEQ ID NO: 5, and (3) a CDR-L3 comprising the amino acid sequence set forth in SEQ ID NO: 6;
b) a heavy chain variable domain (VH) sequence comprising (1) a CDR-H1 comprising the amino acid sequence set forth in SEQ ID NO: 11, (2) a CDR- H2 comprising the amino acid sequence set forth in SEQ ID NO: 12, and (3) a CDR-H3 comprising the amino acid sequence set forth in SEQ ID NO: 13; and a light chain variable domain (VL) sequence comprising (1) a CDR-L1 comprising the amino acid sequence set forth in SEQ ID NO: 14, (2) a CDR-L2 comprising the amino acid sequence set forth in SEQ ID NO: 15, and (3) a CDR-L3 comprising the amino acid sequence set forth in SEQ ID NO: 16;
c) a heavy chain variable domain (VH) sequence comprising (1) a CDR-H1 comprising the amino acid sequence set forth in SEQ ID NO: 21, (2) a CDR- H2 comprising the amino acid sequence set forth in SEQ ID NO: 22, and (3) a CDR-H3 comprising the amino acid sequence set forth in SEQ ID NO: 23; and a light chain variable domain (VL) sequence comprising (1) a CDR-L1 comprising the amino acid sequence set forth in SEQ ID NO: 24, (2) a CDR-L2 comprising the amino acid sequence set forth in SEQ ID NO: 25, and (3) a CDR-L3 comprising the amino acid sequence set forth in SEQ ID NO: 26;
d) a heavy chain variable domain (VH) sequence comprising (1) a CDR-H1 comprising the amino acid sequence set forth in SEQ ID NO: 31, (2) a CDR- H2 comprising the amino acid sequence set forth in SEQ ID NO: 32, and (3) a CDR-H3 comprising the amino acid sequence set forth in SEQ ID NO: 33; and a light chain variable domain (VL) sequence comprising (1) a CDR-L1 comprising the amino acid sequence set forth in SEQ ID NO: 34, (2) a CDR-L2 comprising the amino acid sequence set forth in SEQ ID NO: 35, and (3) a CDR-L3 comprising the amino acid sequence set forth in SEQ ID NO: 36; or
e) a heavy chain variable domain (VH) sequence comprising (1) a CDR-H1 comprising the amino acid sequence set forth in SEQ ID NO: 41, (2) a CDR- H2 comprising the amino acid sequence set forth in SEQ ID NO: 42, and (3) a CDR-H3 comprising the amino acid sequence set forth in SEQ ID NO: 43; and a light chain variable domain (VL) sequence comprising (1) a CDR-L1 comprising the amino acid sequence set forth in SEQ ID NO: 44, (2) a CDR-L2 comprising the amino acid sequence set forth in SEQ ID NO: 45, and (3) a CDR-L3 comprising the amino acid sequence set forth in SEQ ID NO: 46.

7. The antibody of any one of claims 1-6, wherein the antibody comprises:
a) a heavy chain variable region that comprises a CDR-H1 domain, a CDR-H2 domain and a CDR-H3 domain, wherein the CDR-H1 domain, the CDR-H2 domain and the CDR-H3 domain respectively comprise a CDR-H1 domain, a CDR-H2 domain and a CDR-H3 domain comprised in a reference heavy chain variable region comprising the amino acid sequence selected from the group consisting of SEQ ID NOs: 7, 17, 27, 37, and 47; and
b) a light chain variable region that comprises a CDR-L1 domain, a CDR-L2 domain and a CDR-L3 domain, wherein the CDR-L1 domain, the CDR-L2 domain and the CDR-L3 domain respectively comprise a CDR-L1 domain, a CDR-L2 domain and a CDR-L3 domain comprised in a reference light chain variable region comprising the amino acid sequence selected from the group consisting of SEQ ID NOs: 8, 18, 28, 38, and 48.

8. The antibody of any one of claims 1-7, wherein the antibody comprises a heavy chain variable domain (VH) sequence comprising (1) a CDR-H1 comprising the amino acid sequence set forth in SEQ ID NO: 1, (2) a CDR- H2 comprising the amino acid sequence set forth in SEQ ID NO: 2, and (3) a CDR-H3 comprising the amino acid sequence set forth in SEQ ID NO: 3; and a light chain variable domain (VL) sequence comprising (1) a CDR-L1 comprising the amino acid sequence set forth in SEQ ID NO: 4, (2) a CDR-L2 comprising the amino acid sequence set forth in SEQ ID NO: 5, and (3) a CDR-L3 comprising the amino acid sequence set forth in SEQ ID NO: 6.

9. The antibody of any one of claims 1-7, wherein the antibody comprises a heavy chain variable domain (VH) sequence comprising (1) a CDR-H1 comprising the amino acid sequence set forth in SEQ ID NO: 11, (2) a CDR- H2 comprising the amino acid sequence set forth in SEQ ID NO: 12, and (3) a CDR-H3 comprising the amino acid sequence set forth in SEQ ID NO: 13; and a light chain variable domain (VL) sequence comprising (1) a CDR-L1 comprising the amino acid sequence set forth in SEQ ID NO: 14, (2) a CDR-L2 comprising the amino acid sequence set forth in SEQ ID NO: 15, and (3) a CDR-L3 comprising the amino acid sequence set forth in SEQ ID NO: 16.

10. The antibody of any one of claims 1-7, wherein the antibody comprises a heavy chain variable domain (VH) sequence comprising (1) a CDR-H1 comprising the amino acid sequence set forth in SEQ ID NO: 21, (2) a CDR- H2 comprising the amino acid sequence set forth in SEQ ID NO: 22, and (3) a CDR-H3 comprising the amino acid sequence set forth in SEQ ID NO: 23; and a light chain variable domain (VL) sequence comprising (1) a CDR-L1 comprising the amino acid sequence set forth in SEQ ID NO: 24, (2) a CDR-L2 comprising the amino acid sequence set forth in SEQ ID NO: 25, and (3) a CDR-L3 comprising the amino acid sequence set forth in SEQ ID NO: 26.

11. The antibody of any one of claims 1-7, wherein the antibody comprises a heavy chain variable domain (VH) sequence comprising (1) a CDR-H1 comprising the amino acid sequence set forth in SEQ ID NO: 31, (2) a CDR- H2 comprising the amino acid sequence set forth in SEQ ID NO: 32, and (3) a CDR-H3 comprising the amino acid sequence set forth in SEQ ID NO: 33; and a light chain variable domain (VL) sequence comprising (1) a CDR-L1 comprising the amino acid sequence set forth in SEQ ID NO: 34, (2) a CDR-L2 comprising the amino acid sequence set forth in SEQ ID NO: 35, and (3) a CDR-L3 comprising the amino acid sequence set forth in SEQ ID NO: 36.

12. The antibody of any one of claims 1-7, wherein the antibody comprises a heavy chain variable domain (VH) sequence comprising (1) a CDR-H1 comprising the amino acid sequence set forth in SEQ ID NO: 41, (2) a CDR- H2 comprising the amino acid sequence set forth in SEQ ID NO: 42, and (3) a CDR-H3 comprising the amino acid sequence set forth in SEQ ID NO: 43; and a light chain variable domain (VL) sequence comprising (1) a CDR-L1 comprising the amino acid sequence set forth in SEQ ID NO: 44, (2) a CDR-L2 comprising the amino acid sequence set forth in SEQ ID NO: 45, and (3) a CDR-L3 comprising the amino acid sequence set forth in SEQ ID NO: 46.

13. The antibody of any one of claims 1-9, wherein the antibody comprises a heavy chain variable region comprising the amino acid sequence set forth in SEQ ID NO: 7, and a light chain variable region comprising the amino acid sequence set forth in SEQ ID NO: 8.

14. The antibody of any one of claims 1-9, wherein the antibody comprises a heavy chain variable region comprising the amino acid sequence set forth in SEQ ID NO: 17, and a light chain variable region comprising the amino acid sequence set forth in SEQ ID NO: 18.

15. The antibody of any one of claims 1-9, wherein the antibody comprises a heavy chain variable region comprising the amino acid sequence set forth in SEQ ID NO: 27, and a light chain variable region comprising the amino acid sequence set forth in SEQ ID NO: 28.

16. The antibody of any one of claims 1-9, wherein the antibody comprises a heavy chain variable region comprising the amino acid sequence set forth in SEQ ID NO: 37, and a light chain variable region comprising the amino acid sequence set forth in SEQ ID NO: 38.

17. The antibody of any one of claims 1-9, wherein the antibody comprises a heavy chain variable region comprising the amino acid sequence set forth in SEQ ID NO: 47, and a light chain variable region comprising the amino acid sequence set forth in SEQ ID NO: 48.

18. The antibody of any one of claims 1-17, wherein the antibody comprises a human framework.

19. The antibody of any one of claims 1-18, wherein the antibody is a human antibody.

20. The antibody of any one of claims 1-19, wherein the antibody comprises a full-length immunoglobulin, a single-chain Fv (scFv) fragment, a Fab fragment, a Fab' fragment, a F(ab')2, an Fv fragment, a disulfide stabilized Fv fragment (dsFv), a (dsFv)2, a Fv-Fc fusion, a scFv-Fc fusion, a scFv-Fv fusion, a diabody, a tribody, a tetrabody or any combination thereof.

21. The antibody of any one of claims 1-20**,** wherein the antibody comprises a Fc region.

22. The antibody of claim 21, wherein the Fc region comprises a human Fc region.

23. The antibody of claim 21 or 22**,** wherein the Fc region comprises a Fc region selected from the group consisting of the Fc regions of IgG, IgA, IgD, IgE and IgM.

24. The antibody of any one of claims 21-23, wherein the Fc region comprises a Fc region selected from the group consisting of the Fc regions of IgG1, IgG2, IgG3 and IgG4.

25. The antibody of any one of claims 21-24, wherein the Fc region comprises an IgG1 Fc region or an IgG4 Fc region, preferably an IgG1 Fc region.

26. The antibody of any one of claims 21-25, wherein the Fc region comprises a C-terminal lysine.

27. The antibody of any one of claims 21-25, wherein the Fc region comprises a deletion of a C-terminal lysine.

28. The antibody of any one of claims 1-27, wherein the antibody is comprised in a multispecific antibody, e.g., a bispecific antibody, wherein the multispecific antibody comprises a second antibody moiety that specifically binds to a second antigen.

29. The antibody of claim 28, wherein the second antigen is a tumor associated antigen.

30. The antibody of claim 29, wherein the tumor associated antigen is selected from the group consisting of Her-2, EGFR, PDL1, MSLN, c-Met, B Cell Maturation Antigen (BCMA), carbonic anhydrase IX (CA1X), carcinoembryonic antigen (CEA), CD5, CD7, CD10, CD19, CD20, CD22, CD30, CD33, CD34, CD38, CD41, CD44, CD49f, CD56, CD74, CD123, CD133, CD138, CD276 (B7H3), epithelial glycoprotein (EGP2), trophoblast cell-surface antigen 2 (TROP-2), epithelial glycoprotein- 40 (EGP-40), epithelial cell adhesion molecule (EpCAM), receptor tyrosine-protein kinases erb-B2,3,4, folate-binding protein (FBP), fetal acetylcholine receptor (AChR), folate receptor-a, Ganglioside G2 (GD2), Ganglioside G3 (GD3), human telomerase reverse transcriptase (hTERT), kinase insert domain receptor (KDR), Lewis A (CA 1.9.9), Lewis Y (LeY), Glypican-3 (GPC3), L1 cell adhesion molecule (L1CAM), Mucin 16 (Muc-16), Mucin 1 (Muc-1), NG2D ligands, oncofetal antigen (h5T4), prostate stem cell antigen (PSCA), prostate-specific membrane antigen (PSMA), tumor- associated glycoprotein 72 (TAG-72), Claudin18.2 (CLDN18.2), vascular endothelial growth factor R2 (VEGF- R2), Wilms tumor protein (WT-1), type 1 tyrosine-protein kinase transmembrane receptor (ROR1), PVR, PVRL2, and any combination thereof.

31. An immunoconjugate comprising the antibody of any one of claims 1-30, linked to a therapeutic agent or a label.

32. The immunoconjugate of claim 31, wherein the therapeutic agent is a cytotoxin or a radioactive isotope.

33. The immunoconjugate of claim 32, wherein the cytotoxin is selected from the group consisting of amanitin, auristatin, calicheamicin, camptothecin, cryptophycin, daunomycin, dolastatin, doxorubicin, duocarmycin, epothilone, esperamicin, geldanamycin, maytansinoid, methotrexate, monomethyl auristatin E ("MMAE"), monomethyl auristatin F ("MMAF"), pyrrolobenzodiazepine, SN-38, rhizoxin, SG2285, tubulysin, vindesine, toxoid, or any combination thereof.

34. The immunoconjugate of claim 32 or 33, wherein the antibody and the cytotoxin are linked via a linker.

35. The immunoconjugate of claim 34, wherein the linker comprises a maleimidocaproyl group, one or more natural or unnatural amino acids, and a self-immolative group.

36. The immunoconjugate of claim 31, wherein the label is selected from the group consisting of a radioisotope, a fluorescent dye and an enzyme.

37. A chimeric antigen receptor (CAR) comprising an extracellular antigen-binding domain that comprises an antibody of any one of claims 1-30.

38. The CAR of claim 37, wherein the antibody is a scFv.

39. An immunoresponsive cell comprising a CAR of claim 37 or 38.

40. The immunoresponsive cell of claim 39, wherein the immunoresponsive cell is selected from the group consisting of a T cell, a Natural Killer (NK) cell, a cytotoxic T lymphocyte (CTL), a regulatory T cell, a Natural Killer T (NKT) cell and a myeloid cell.

41. The immunoresponsive cell of claim 39 or 40, wherein the immunoresponsive cell is a T cell.

42. A pharmaceutical composition comprising a) the antibody of any one of claims 1-30, the immunoconjugate of any one of claims 31-36, or the immunoresponsive cell of any one of claims 39-41, and b) a pharmaceutically acceptable carrier.

43. A nucleic acid encoding the antibody of any one of claims 1-30.

44. A vector comprising the nucleic acid of claim 43.

45. A host cell comprising the nucleic acid of claim 43 or the vector of claim 44.

46. A method for preparing an antibody of any one of claims 1-30 comprising expressing the antibody in the host cell of claim 45 and isolating the antibody from the host cell.

47. A method of reducing tumor burden in a subject, the method comprising administering to the subject an effective amount of the antibody of any one of claims 1-30, the immunoconjugate of any one of claims 31-36, the immunoresponsive cell of any one of claims 39-41, or the pharmaceutical composition of claim 42.

48. The method of claim 47, wherein the method reduces the number of tumor cells.

49. The method of claim 47 or 48, wherein the method reduces tumor size.

50. The method of any one of claims 47-49, wherein the method eradicates the tumor in the subject.

51. The method of any one of claims 47-50, wherein the tumor is selected from the group consisting of mesothelioma, lung cancer, pancreatic cancer, ovarian cancer, breast cancer, colon cancer, pleural tumor, glioblastoma, esophageal cancer, gastric cancer, synovial sarcoma, thymic carcinoma, endometrial carcinoma, stomach cancer, cholangiocarcinoma, head and neck cancer, blood cancer, prostate cancer, cervical cancer, melanoma, squamous cell carcinoma, liver cancer and a combination thereof.

52. A method of treating and/or preventing a neoplasm, the method comprising administering to the subject an effective amount of the antibody of any one of claims 1-30, the immunoconjugate of any one of claims 31-36, the immunoresponsive cell of any one of claims 39-41, or the pharmaceutical composition of claim 42.

53. A method of lengthening survival of a subject having a neoplasm, the method comprising administering to the subject an effective amount of the antibody of any one of claims 1-30, the immunoconjugate of any one of claims 31-36, the immunoresponsive cell of any one of claims 39-41, or the pharmaceutical composition of claim 42.

54. The method of claim 52 or 53, wherein the neoplasm is selected from the group consisting of mesothelioma, lung cancer, pancreatic cancer, ovarian cancer, breast cancer, colon cancer, pleural tumor, glioblastoma, esophageal cancer, gastric cancer, synovial sarcoma, thymic carcinoma, endometrial carcinoma, stomach cancer, cholangiocarcinoma, head and neck cancer, blood cancer, prostate cancer, cervical cancer, melanoma, squamous cell carcinoma, liver cancer and a combination thereof.

55. A use of the antibody of any one of claims 1-30, the immunoconjugate of any one of claims 31-36, the immunoresponsive cell of any one of claims 39-41, or the pharmaceutical composition of claim 42 in the manufacture of a medicament for reducing tumor burden in a subject, treating and/or preventing a neoplasm, or lengthening survival of a subject having a neoplasm.

56. The use of claim 55, wherein the tumor or neoplasm is selected from the group consisting of mesothelioma, lung cancer, pancreatic cancer, ovarian cancer, breast cancer, colon cancer, pleural tumor, glioblastoma, esophageal cancer, gastric cancer, synovial sarcoma, thymic carcinoma, endometrial carcinoma, stomach cancer, cholangiocarcinoma, head and neck cancer, blood cancer, prostate cancer, cervical cancer, melanoma, squamous cell carcinoma, liver cancer and a combination thereof.

57. An antibody of any one of claims 1-30 for use as a medicament.

58. An antibody of any one of claims 1-30 for use in treating cancer.

59. A pharmaceutical composition of claim 42 for use as a medicament.

60. A pharmaceutical composition of claim 42 for use in treating cancer.

61. The antibody of claim 58 or the pharmaceutical composition of claim 60, wherein the cancer is selected from the group consisting of mesothelioma, lung cancer, pancreatic cancer, ovarian cancer, breast cancer, colon cancer, pleural tumor, glioblastoma, esophageal cancer, gastric cancer, synovial sarcoma, thymic carcinoma, endometrial carcinoma, stomach cancer, cholangiocarcinoma, head and neck cancer, blood cancer, prostate cancer, cervical cancer, melanoma, squamous cell carcinoma, liver cancer and a combination thereof.

62. A kit comprising the antibody of any one of claims 1-30, the immunoconjugate of any one of claims 31-36, the immunoresponsive cell of any one of claims 39-41, the pharmaceutical composition of claim 42, the nucleic acid of claim 43, or the vector of claim 44.

63. The kit of claim 62, further comprising a written instruction for treating and/or preventing a neoplasm.
